(19) Europäisches Patentamt
European Patent Office
Office européen des brevets

(11) EP 0 753 070 B1

(12) # FASCICULE DE BREVET EUROPEEN

(45) Date de publication et mention de la délivrance du brevet:
**25.09.2002 Bulletin 2002/39**

(21) Numéro de dépôt: **95918049.8**

(22) Date de dépôt: **24.04.1995**

(51) Int Cl.[7]: **C12N 15/87**, A61K 47/48, A61K 48/00, C12Q 1/68

(86) Numéro de dépôt international:
**PCT/FR95/00535**

(87) Numéro de publication internationale:
**WO 95/030020 (09.11.1995 Gazette 1995/48)**

(54) **NOUVEAUX COMPLEXES D'ACIDE NUCLEIQUE ET DE POLYMERE, LEUR PROCEDE DE PREPARATION ET LEUR UTILISATION POUR LA TRANSFECTION DE CELLULES**

NEUE NUKLEINSAURE-POLYMER KOMPLEXE, VERFAHREN ZUR DEREN HERSTELLUNG UND VERWENDUNG ZUR TRANSFEKTION VON ZELLEN

NOVEL NUCLEIC ACID/POLYMER COMPLEXES, METHOD FOR PREPARING SAME AND USE THEREOF FOR CELL TRANSFECTION

(84) Etats contractants désignés:
**AT BE CH DE DK ES FR GB GR IE IT LI LU MC NL PT SE**

(30) Priorité: **28.04.1994 FR 9405174**

(43) Date de publication de la demande:
**15.01.1997 Bulletin 1997/03**

(73) Titulaire: **I.D.M. IMMUNO-DESIGNED MOLECULES**
**75011 Paris (FR)**

(72) Inventeurs:
- **MIDOUX, Patrick**
  **F-45100 Orléans (FR)**
- **ERBACHER, Patrick**
  **F-45000 Orléans (FR)**
- **ROCHE-DEGREMONT, Annie-Claude**
  **F-45640 Sandillon (FR)**
- **MONSIGNY, Michel**
  **F-45590 Saint-Cyr-en-Val (FR)**

(74) Mandataire: **Grosset-Fournier, Chantal Catherine**
**Grosset-Fournier & Demachy s.a.r.l.**
**20, rue de Maubeuge**
**75009 Paris (FR)**

(56) Documents cités:
**WO-A-92/13570**

- **TETRAHEDRON 49(32), 6991-7000, 6 Août 1993 NEGRE, E. ET AL. 'Synthesis of an allopurinol riboside-mannosylated poly-L-lysine conjugate'**
- **NUCLEIC ACIDS RES. 21(4), 871-8, 25 Février 1993 MIDOUX, P. ET AL. 'Specific gene transfer mediated by lactosylated poly-L-lysine into hepatoma cells' cité dans la demande**
- **BIOCHEMICAL AND BIOPHYSICAL RESEARCH COMMUNICATIONS, vol. 167, no. 3, 30 Mars 1990 DULUTH, MINNESOTA US, pages 1044-1049, MIDOUX, P. ET AL. 'Drug targeting: anti-HSV-1 activity of mannosylated polymer-bound 9-(2-phosphonylmethoxyethyl)adenine'**
- **CURRENT OPINION IN CELL BIOLOGY, vol. 4, no. 2, Avril 1992 pages 257-266, VARKI, A. 'Selectins and other mammalian sialic acid-binding lectins' cité dans la demande**
- **ADVANCED DRUG DELIVERY REVIEWS, vol. 14, 1994 pages 1-24, MONSIGNY, M. ET AL. 'Glycoconjugates as carriers for specific delivery of therapeutic drugs and genes'**
- **BIOCONJUGATE CHEMISTRY, vol. 6, Juillet 1995 - Août 1995 WASHINGTON US, pages 401-410, ERBACHER, P. ET AL. 'Glycosylated polylysine/DNA complexes: gene transfer efficiency in relation with the size and the sugar substitution level of glycosylated polylysines and with the plasmid size'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 753 070 B1

## Description

L'introduction d'un gène étranger dans une cellule est d'un grand intérêt pour la thérapie génique. Tandis que dans les expériences *in vitro*, des méthodes générales utilisant la précipitation par le phosphate de calcium, le dextran DEAE, ou les lipides cationiques sont appropriées, des méthodes plus sélectives sont nécessaires pour transférer de façon spécifique un gène dans une population de cellules données, dans le but de développer une thérapie génique. Parmi ces méthodes sélectives, le transfert de gène peut être obtenu en utilisant, soit un matériel viral modifié, allant d'un virus de vaccine à un rétrovirus, soit des liposomes ciblés, soit des complexes de gènes et de macromolécules ciblés. Les complexes ADN/vecteurs tels que asialoorosomucoïde, insuline ou transferrine liés à la polylysine ont été déjà proposés comme vecteurs guides de plasmides permettant la transfection de cellules selon un procédé d'endocytose induit par les récepteurs correspondants: le récepteur spécifique des galactosides (lectine) pour l'asialoorosomucoïde, le récepteur de l'insuline et le récepteur de la transferrine.

Il a été établi que de nombreuses cellules animales possèdent des lectines membranaires [Monsigny M., Roche A.C., Kieda C., Midoux P., Obrenovitch A. Characterization and biological implications of membrane lectins in tumor, lymphoid and myeloid cells. Biochimie, 1988: **70**: 1633-49; Varki A. Selectin and other mammalian sialic acid binding lectins. Curr. Op. Cell. Biol., 1992, **4**: 257-66] qui reconnaissent spécifiquement des osides de structures diverses. En particulier, la lectine membranaire des cellules du parenchyme hépatique qui reconnaît des structures glucidiques comportant un résidu galactose en position terminale, c'est-à-dire un galactose ayant toutes ses fonctions alcooliques libres, ce qui est le cas de glycoprotéines sériques désialysées [Ashwel G., Harford J. Carbohydrate-specific receptors of the liver. Ann. Rev. Biochem., 1982, **51**: 531-54].

La spécificité de ces lectines dépend du type de cellules et par conséquent, les lectines membranaires sont de bons candidats pour le transfert de gène par des complexes glycoconjugués/ADN comme porteurs spécifiques. Les glycoconjugués solubles portant des résidus de sucre définis ont été utilisés pour introduire efficacement des médicaments, y compris des drogues cytotoxiques, des toxines, des immunomodulateurs, des drogues antivirales [Monsigny, M., Roche, A.C., Kieda, C., Midoux, P. and Obrenovitch, A. (1988) Biochimie **70**: 1633-1649 2; Roche, A. C., Midoux, P., Pimpaneau, V., Nègre, E., Mayer, R. and Monsigny, M. (1990) Res. Virol. **141**: 243-249] et des oligonucléotides [Bonfils E., Depierreux C., Midoux P., Thuong N.T., Monsigny M., Roche A.C. Drug targeting: synthesis and endocytosis of oligonucleotide-neoglycoprotein conjugales. Nucleic Acids Res., 1992, **20**: 4621-9; Bonfils E., Mendès C., Roche A.C., Monsigny M., Midoux P. Uptake by macrophages of a biotinylated oligo-$\alpha$-deoxythymidylate by using mannosylated streptavidin. Bioconjuguate Chem., 1992, **3**: 277-84].

Le transport des plasmides par des macromolécules susceptibles d'être reconnues spécifiquement par des composés de la membrane plasmique des cellules cibles relève d'une démarche imitant le mécanisme d'entrée du matériel génétique viral dans la cellule. Dans tous les cas décrits jusqu'à présent, le complexe plasmide-transporteur macromoléculaire est reconnu spécifiquement par un récepteur membranaire qui entraîne le complexe dans des vésicules d'endocytose, dans des endosomes, et probablement dans d'autres compartiments intracellulaires plus profonds, éloignés de la membrane plasmique.

Cependant, le passage transmembranaire de l'ADN plasmidique est une étape critique vis à vis de la libération dudit ADN dans le cytosol et/ou le noyau, où le gène sera exprimé.

L'invention a pour objet de nouveaux complexes stables d'acide nucléique et de polymère substitué.

L'invention a également pour objet de nouveaux complexes d'acide nucléique et de polymère substitués susceptibles, en se dissociant, de relarguer l'acide nucléique, afin de permettre une bonne expression de l'acide nucléique dans les cellules.

L'invention a pour objet de nouveaux complexes d'acide nucléique et de polymère substitué ne présentant pas de signaux de reconnaissance et susceptibles de transfecter plusieurs types de cellules.

L'invention a pour objet de nouveaux complexes d'acide nucléique et de polymère substitué présentant des signaux de reconnaissance reconnus par des récepteurs membranaires, conférant un caractère sélectif de la transfection vis à vis de différents types cellulaires.

L'invention a pour objet un procédé de transfection spécifique *in vitro* ou *in vivo.*

L'invention a également pour objet de nouveaux conjugués de polylysine susceptibles d'être complexés à un acide nucléique en vue de la transfection sélective d'une cellule.

L'invention a également pour objet de nouvelles compositions pharmaceutiques contenant, à titre de substance active, un complexe d'ADN et de polymère substitué, notamment de polylysine substituée.

L'invention a également pour objet de nouveaux complexes d'acide nucléique et de polymère substitué possédant une haute solubilité dans le sérum physiologique et divers milieux de cultures, susceptibles d'être administrés *in vivo* à des doses très élevées.

Dans une de ses définitions les plus générales, l'invention concerne un complexe entre au moins un acide nucléique qui est chargé négativement et au moins un conjugué polymérique chargé positivement, la liaison entre l'acide nucléique et le conjugué polymérique étant de nature électrostatique, le conjugué polymérique contenant des

motifs monomères portant des fonctions $NH_3^+$ libres des susdits motifs et étant tel que:

- les fonctions $NH_3^+$ libres des susdits motifs sont substituées dans un rapport d'au moins 10%, avantageusement de 45% à 70%, notamment de 60%, ce rapport étant déterminé par méthode colorimétrique (Fields R. (1971) The measurement of amino groups on proteins and peptides. Biochem. J., 124: 581-590) ou avantageusement de 35% à 70%, notamment de 40%, ce rapport étant déterminé par résonance magnétique nucléaire (RMN), par des résidus non chargés entraînant une diminution des charges positives par rapport au même conjugué polymérique non substitué, facilitant le relargage de l'acide nucléique par la dissociation du complexe,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les fonctions $NH_3^+$ libres des susdits motifs et/ou les groupes hydroxyles des susdits résidus pouvant être également substituées par une molécule qui constitue un signal de reconnaissance reconnu par un récepteur membranaire cellulaire, sous réserve que le conjugué polymérique, après substitution par les susdits résidus et par les susdits signaux de reconnaissance, contienne au moins 30% de fonctions $NH_3^+$ libres.

L'originalité de l'invention repose sur l'utilisation d'un polymère substitué par une quantité suffisante de résidus permettant,

i) de former des complexes stables avec un acide nucléique, ARN et ADN, notamment l'ADN, par interactions électrostatiques avec les charges négatives de l'acide nucléique, notamment de l'ADN et le reste des charges positives du polymère substitué par les susdits résidus, et
ii) de faciliter la dissociation du complexe et le relargage de l'acide nucléique afin de permettre une bonne expression du gène dans les cellules.

En effet, le susdit polymère substitué permet une condensation de l'ADN qui reste très forte par suite d'un phénomène coopératif entre les charges positives et négatives du polymère et de l'ADN. Le polymère substitué par exemple à 58% avec un susdit résidu possède moins de charges positives, ce qui réduit la coopérativité des interactions et facilite la dissociation entre l'ADN et le polymère.
La dissociation du complexe peut être mesurée dans les conditions décrites à propos de la figure 6.
Pris isolement, le conjugué polymérique contient des monomères portant des fonctions $NH_2$ libres, susceptibles dans les conditions de pH appropriées (pH < 10), de devenir $NH_3^+$.
Par ailleurs, la présence d'un signal de reconnaissance membranaire cellulaire n'est pas obligatoire.
L'expression selon laquelle "les résidus substituant $NH_3^+$ ne correspondent à aucun signal de reconnaissance membranaire cellulaire" signifie qu'ils ne correspondent à aucun signal dans la mesure des connaissances actuelles de la littérature.
Par signal de reconnaissance reconnu par un récepteur membranaire cellulaire, on désigne généralement une molécule ou un complexe moléculaire capable de reconnaître sélectivement un ligand (affinité signal-récepteur ≥ $10^3$l/mole).
Le nombre de signaux de reconnaissance qui substituent les $NH_3^+$ libres des susdits motifs et/ou les groupes hydroxyles des susdits résidus varie de 0 à 40%.
Etant donné que le nombre de $NH_3^+$ libres sur le conjugué polymérique doit être d'au moins 30%, lorsque les $NH_3^+$ des susdits motifs sont substitués par 10% de résidus non chargés notamment gluconoyle, les signaux de reconnaissance peuvent être jusqu'à 40% sur les 90% des $NH_3^+$ non engagés avec des résidus non chargés et/ou sur les groupes hydroxyles des susdits résidus. Lorsque les $NH_3^+$ des susdits motifs sont substitués par 45% de résidus non chargés, les signaux de reconnaissance peuvent être sur 25 des 55% des $NH_3^+$ non engagés avec les résidus non chargés et/ou sur les hydroxyles des susdits résidus. En revanche, lorsque le nombre de $NH_3^+$ engagés dans des liaisons avec les résidus augmente jusqu'à 70%, afin que le conjugué polymérique garde au moins 30% de $NH_3^+$ libres, les signaux de reconnaissance ne peuvent plus être que sur les hydroxyles des susdits résidus.
Le taux de substitution des fonctions $NH_3^+$ libres des susdits motifs par des résidus non chargés peut être déterminé selon 2 méthodes:

1°) une méthode colorimétrique après réaction de l'acide 2,4,6 trinitobenzène sulfonique (TNBS) (Fields R. (1971) The measurement of amino groups on proteins and peptides. Biochem. J., 124: 581-590) avec les groupes ε-amino des résidus lysine libres de la polylysine gluconoylée par rapport à la polylysine non substituée;
le nombre moyen de résidus gluconoyle fixés par molécule de polylysine est obtenu par différence avec le

nombre moyen de résidus lysine par molécule de polylysine;
2°) une méthode physique en utilisant la résonance magnétique nucléaire (RNM); les spectres RMN sont réalisés à 300 MHz avec 4 mg de polylysine gluconoylée lyophilisée dans $D_2O$ et repris dans 0,5 ml de $D_2O$ (Figures 9 et 10);

le nombre moyen x de résidus gluconoyle fixés par molécule de polylysine est déterminée à partir de la relation:

$$x = 3/2 \,.\, (h_{GlcA}/h_{Lys}).\ DP$$

où $h_{Lys}$ est la valeur de l'intégration à 1,7 ppm correspondant aux 6 protons des carbones 3, 4 et 5 (Figure 1) d'un résidu lysine de la polylysine, $h_{GlcA}$ est la valeur de l'intégration à 3,8 ppm correspondant à 4 protons d'un groupe gluconoyle (Figures 9 et 10) et DP est le degré de polymérisation de la polylysine; la formule ci-dessus est à adapter en fonction de la nature du résidu.

Il faut noter que la détermination du nombre de résidus non chargés sur le conjugué polymérique dépend de la méthode utilisée. Ce nombre est surestimé lorsque l'on utilise le dosage colorimétrique qui permet de calculer par différence un nombre relatif de résidus non chargés sur le conjugué polymérique. En revanche, la RMN permet de calculer directement le nombre de résidus non chargés indépendamment de la concentration en conjugué polymérique.

Dans ce qui précède ou dans ce qui suit, sauf indications contraires, le taux de substitution des fonctions $NH_3^+$ libres est donné dans le cadre de la méthode colorimétrique.

L'invention concerne notamment un complexe entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres des susdits motifs et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives par rapport au même conjugué polymérique non substitué, facilitant le relargage de l'acide nucléique par la dissociation du complexe,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les fonctions $NH_3^+$ libres des susdits motifs des susdits résidus pouvant être également substituées par au moins une molécule qui constitue un signal de reconnaissance reconnu par un récepteur membranaire cellulaire, sous réserve que le conjugué polymérique contienne au moins 30% de fonctions $NH_3^+$ libres.

L'invention concerne notamment un complexe entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres des susdits motifs et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives par rapport au même conjugué polymérique non substitué, facilitant le relargage de l'acide nucléique par la dissociation du complexe,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les groupes hydroxyles des susdits résidus pouvant être substituées par au moins une molécule qui constitue un signal de reconnaissance reconnu par un récepteur membranaire cellulaire, sous réserve que le conjugué polymérique contienne au moins 30% de fonctions $NH_3^+$ libres.

Selon un mode de réalisation avantageux, l'invention concerne un complexe entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de

motifs monomères portant des fonctions $NH_3^+$ libres, notamment des résidus de lysine et étant tel que:

- les fonctions $NH_3^+$ libres des susdits motifs sont substituées dans un rapport d'au moins 10%, avantageusement de 45% à 70%, notamment d'environ 60%, par des résidus non chargés entraînant une diminution des charges positives par rapport au même conjugué polymérique non substitué, facilitant ainsi la dissociation du complexe et le relargage de l'acide nucléique,
- les susdits résidus possédant en outre les propriétés suivantes:

  → ils comportent au moins un groupe hydroxyle,
  → ils ne correspondent à aucun signal de reconnaissance de cellules,

- 0 à 40% du nombre des fonctions $NH_3^+$ libres des susdits motifs étant également substituées par une molécule qui constitue un signal de reconnaissance reconnu par un récepteur membranaire cellulaire, ce signal de reconnaissance ayant un poids moléculaire inférieur à 5000, sous réserve que le conjugué polymérique contienne au moins 30% de fonctions $NH_3^+$ libres,

et lorsqu'il est présent, ce signal de reconnaissance pouvant l'être à raison d'une molécule pour environ 10 000 motifs du conjugué polymérique ou d'environ 60 molécules pour environ 10 000 motifs du conjugué polymérique.

L'invention concerne plus particulièrement un complexe entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres, notamment des résidus de lysine et étant tel que:

- les fonctions $NH_3^+$ libres des susdits motifs sont substituées dans un rapport d'au moins 10%, avantageusement de 45% à 70%, notamment d'environ 60%, par des résidus non chargés entraînant une diminution des charges positives par rapport au même conjugué polymérique non substitué, facilitant ainsi la dissociation du complexe et le relargage de l'acide nucléique,
- les susdits résidus possédant en outre les propriétés suivantes:

  → ils comportent au moins un groupe hydroxyle,
  → ils ne correspondent à aucun signal de reconnaissance membranaire cellulaire,

- les fonctions $NH_3^+$ libres des susdits motifs pouvant être également substituées par une molécule qui constitue un signal de reconnaissance membranaire cellulaire, ce signal de reconnaissance ayant un poids moléculaire inférieur à 5000,

et lorsqu'il est présent, ce signal de reconnaissance pouvant l'être à raison d'une molécule pour environ 10 000 motifs du conjugué polymérique ou d'environ 60 molécules pour environ 10 000 motifs du conjugué polymérique.

Dans ces conditions, compte tenu du faible nombre de signaux de reconnaissance, au moins 30% des $NH_3^+$ du conjugué polymérique sont libres.

Lorsqu'ils sont présents, les signaux de reconnaissance ont pour but de conférer à la transfection la sélectivité de la transfection vis à vis de différents types cellulaires et de rendre efficace la transfection *in vivo*.

Les signaux de reconnaissance ont également pour effet, compte tenu de leur charge généralement neutre, d'entraîner une diminution des charges positives du conjugué polymérique.

Les signaux de reconnaissance sont des molécules de petite masse moléculaire (< 5000 daltons).

Le nombre de molécules de signal de reconnaissance fixé sur le polymère modifié peut être,

- pour une molécule signal de très haute affinité vis à vis de son récepteur, de 0,5 à 5, avantageusement 1 molécule pour environ 10 000 motifs monomères de polymère substitué soit 1 molécule pour environ 50 molécules de polymère substitué;
- pour une molécule signal de moyenne affinité vis à vis de son récepteur, d'environ 10 à environ 100, avantageusement 60 molécules pour environ 10 000 motifs monomères de polymère substitué.

Une molécule signal de très haute affinité correspond à une valeur de Ka d'au moins $10^6$ l/mole.

Une molécule signal de moyenne affinité correspond à une valeur de Ka d'au moins $10^4$ l/mole.

Selon un mode de réalisation avantageux, dans les complexes de l'invention, le polymère contient un groupement polymérique de formule (I) suivante:

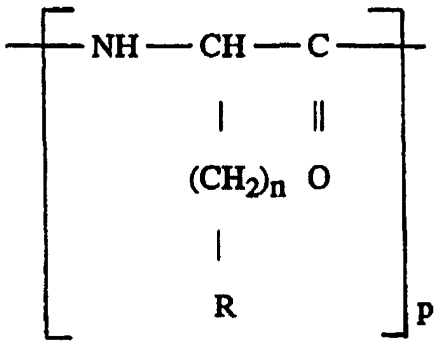

(I)

dans laquelle:

- . p est un nombre entier variant de 2 à 500 de préférence de 150 à 200,
- . n est un nombre entier variant de 1 à 5 et vaut de préférence 4,
- . ce groupement polymérique contenant un nombre de p radicaux R parmi lesquels:

  - * 10% à 70% du nombre des radicaux R représentent un groupe NH-CO-$(CHOH)_m$-$R_1$, notamment un reste dihydroxypropionoyle, érythrononoyle, thréonoyle, ribonoyle, arabinoyle, xylonoyle, lyxonoyle, gluconoyle, galactonoyle, mannonoyle, glycoheptonoyle, glycooctonoyle,

- . m est un nombre entier de 2 à 15, de préférence de 2 à 7,
- . $R_1$ représente H ou un radical alcoyle de 1 à 15 atomes de carbone, notamment $CH_3$,

  - * le reste des radicaux, c'est-à-dire 30% à 90% du nombre des radicaux R représentent $NH_3^+$,
  - * R pouvant en outre être constitué de 0 à 25% par une molécule qui constitue un signal de reconnaissance, sous réserve que le conjugué polymérique contienne au moins 30% de fonctions $NH_3^+$ libres, et notamment à raison de 0,5 à 5, avantageusement de 1 molécule pour environ 10 000 motifs,

ou à raison de 10 à 100, avantageusement de 60 molécules pour environ 10 000 motifs.

Selon un mode de réalisation avantageux, l'invention concerne un complexe comme définit précédemment, dans lequel le polymère comprend un groupement polymérique de formule (II):

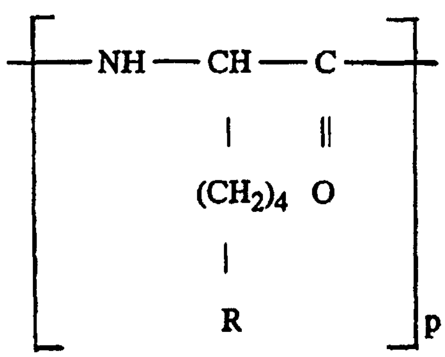

(II)

dans laquelle:

- . p a les significations indiquées ci-dessus,

  - * 10% à 70% du nombre des résidus R représentent un groupe NH-CO-$(CHOH)_m$-$R_1$, m et $R_1$ ayant la signification indiquée ci-dessus,
  - * le reste des radicaux, c'est-à-dire 30% à 90% du nombre des radicaux R représentent $NH_3^+$, et de 0 à 25% des radicaux R pouvant être subtitués par une molécule qui constitue un signal de reconnaissance reconnu par un récepteur membranaire cellulaire, sous réserve que le conjugué polymérique contienne au moins 30% de fonctions $NH_3^+$ libres.

Selon un autre mode de réalisation, dans les complexes de l'invention, le polymère comprend un groupement polymérique de formule (II):

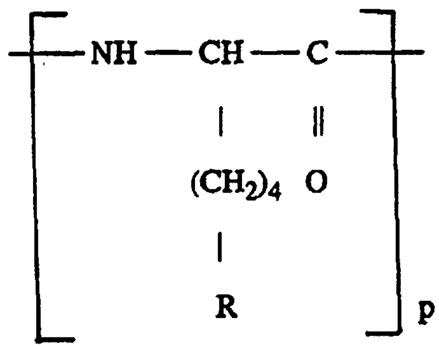

(II)

dans laquelle:

. p a les significations indiquées ci-dessus,

* 10% à 70% du nombre des résidus R représentent un groupe $NH-CO-(ROH)_m-R_1$, m et $R_1$ ayant la signification indiquée ci-dessus,
* le reste des radicaux, c'est-à-dire 30% à 90% du nombre des radicaux R représentent $NH_3^+$.

Dans cette classe de complexes de l'invention, le polymère est de la polylysine.
Comme le montrent les exemples, les cellules HepG2 (hepatocarcinome humain) sont efficacement transfectées par de la polylysine substituée par 58 ± 12% (110 ± 22 résidus) gluconoyles (environ 300 fois plus qu'avec le plasmide seul). Les polylysines substituées par peu ou trop de résidus gluconoyles sont pas ou peu efficaces pour obtenir une bonne transfection.
La polylysine substituée par 58 ± 12% de gluconoyles a permis de transfecter différentes cellules (humaines et murines, adhérantes ou en suspension) avec une grande efficacité, modulée selon le type cellulaire et le promoteur utilisé.
Selon un autre mode de réalisation avantageux, dans les complexes de l'invention, le polymère comprend un groupement polymérique de formule (II):

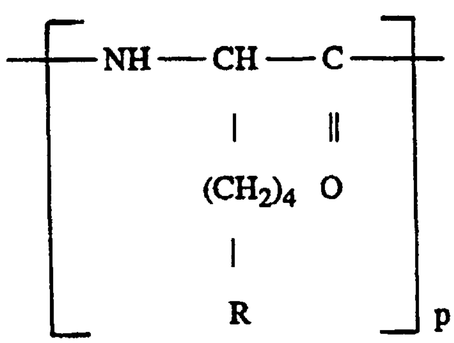

(II)

dans laquelle:
p a la signification indiquée ci-dessus,

. ce groupement polymérique contenant un nombre p radicaux R parmi lesquels:

* 10% à 70% du nombre des radicaux R représentent un groupe $NH-CO-(CHOH)_m-R_1$, m et $R_1$ ayant les significations indiquées ci-dessus,
* le reste des radicaux, c'est-à-dire 30% à 90% des radicaux R représentent d'une part $NH_3^+$ et d'autre part une molécule qui constitue un signal de reconnaissance à raison de 0,5 à 5, avantageusement de 1 molécule pour

10 000 motifs.

Selon un autre mode de réalisation, dans les complexes de l'invention, le polymère comprend un groupement polymérique de formule (II):

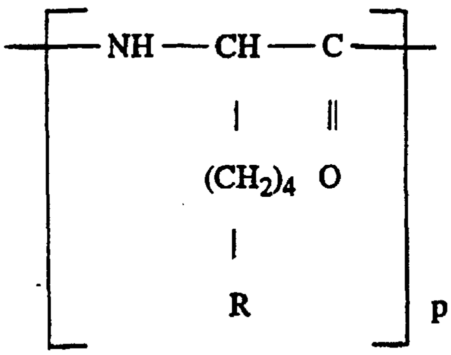

(II)

dans laquelle:

- p a la signification indiquée ci-dessus,
- ce groupement polymérique contenant un nombre p radicaux R parmi lesquels:

  - * 10% à 70% du nombre des radicaux R représentent un groupe NH-CO-$(CHOH)_m$-$R_1$, m et $R_1$ ayant les significations indiquées ci-dessus,
  - * le reste des radicaux, c'est-à-dire 30% à 90% de ces radicaux R représentent d'une part $NH_3^+$ et d'autre part une molécule qui constitue un signal de reconnaissance à raison de 10 à 100, avantageusement de 60 molécules pour environ 10 000 motifs.

Dans les complexes de l'invention, le signal de reconnaissance peut être choisi parmi:

A) - des osides simples ou complexes reconnus par des lectines membranaires, et choisis parmi:

**a. Asialo-oligoside de type triantennaire lactosamine: récepteur d'asialoglycoprotéine**

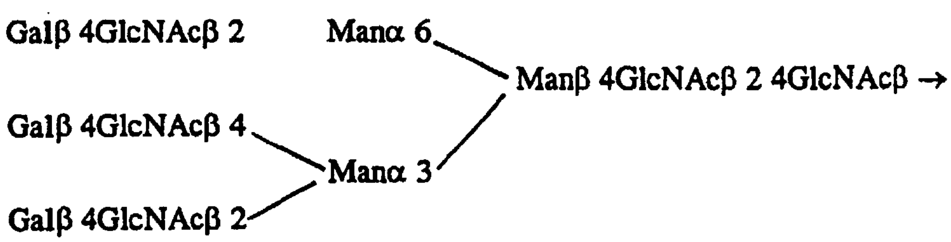


**b. Asialo oligoside de type lactosamine tetraantennaire: récepteur d'asialoglycoprotéine**

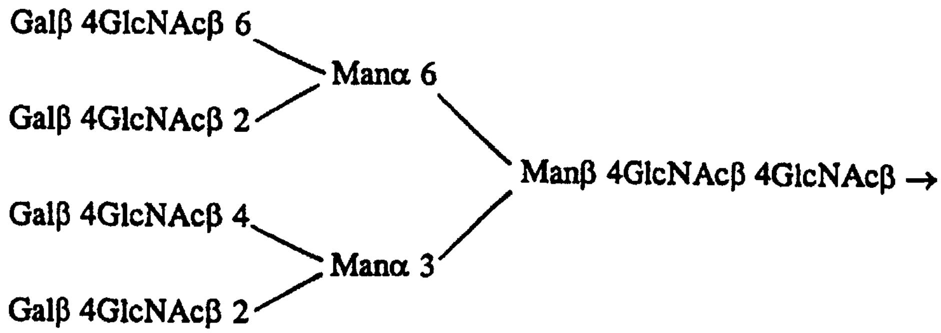

c. Lewis x: LECAM 2/3

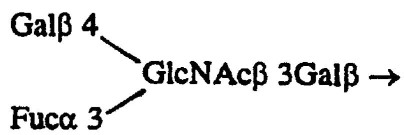

d. Lewis x sialyl: LECAM 3/2

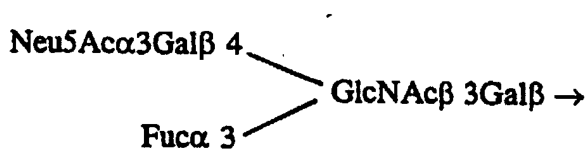

e. Dérivé de Lewis x sulfaté (HNK1): LECAM 1

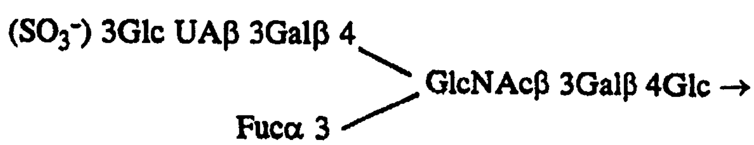

f. Oligomannoside: récepteur du mannose

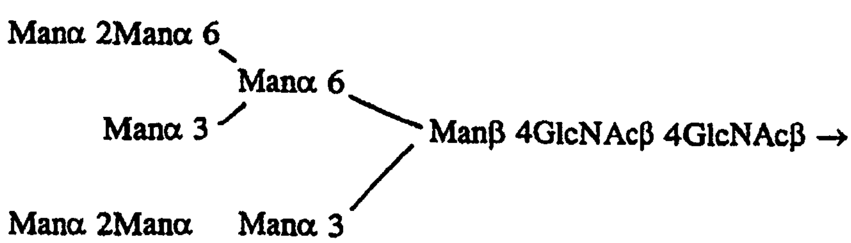

g. Oligomannoside phosphorylé: récepteur de mannose 6 phosphate

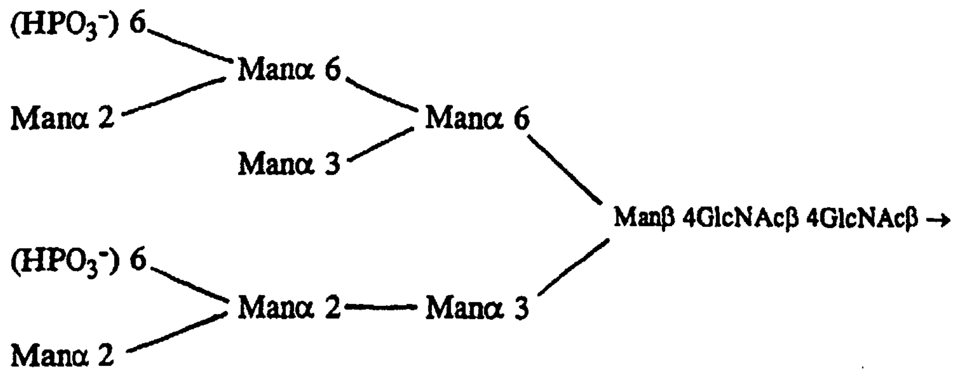

**h. Oligosaccharide de type lactosamine sulfaté: récepteur de GalNAc 4 sulfaté**

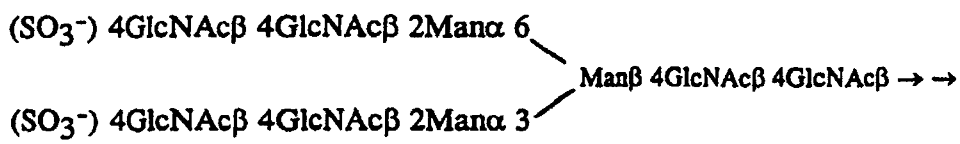

B) des peptides

a) peptides anti-inflammatoires ou certains de leurs fragments reconnus par des récepteurs de la paroi vasculaire, tels que par exemple:

- polypeptide vasodilatateur intestinal (VIP)
  HSDAVFTDNYTRLRKQMAVKKYLNSILN-$NH_2$
- polypeptide atrial natriurétique (ANP)
  SLRRSSCFGGRMDRIGAQSGLGCNSFRY
- lipocortine
  HDMNKVLDL
- bradykinine
  RPPGFSPFR;

b) peptides ligands des intégrines, tels que les peptides contenant la séquence RGD tel que le récepteur de la fibronectine;
c) facteurs chimiotactiques, tels que les formyl peptides et antagonistes:
FMLP, (N-formyl-Met-Leu-Phé);
d) hormones peptidiques tels que

$\alpha$-MSH: Ac-SYSMEHFRWGKPV-$NH_2$

C) Métabolites naturels tels que:

- la biotine,
- le tétrahydrofolate,
- l'acide folique,
- la carnitine.

Dans les complexes de l'invention, l'acide nucléique peut être choisi parmi:

a) des gènes marqueurs, tels que

- gènes contenant la luciférase,
- gènes contenant la $\beta$-galactosidase,

- gènes contenant la chloramphénicol acétyl transférase,
- gènes conférant la résistance à un antibiotique, tel que l'hygromycine ou la néomycine;

b) des gènes à visée thérapeutique, tels que

- récepteurs des lipoprotéines de faible densité, déficient dans les cas d'hypercholestérolémie (foie),
- facteurs de coagulation: facteurs VIII et IX,
- phénylalanine-hydroxylase (phénylcétonurie),
- adénosine désaminase (immunodéficience ADA),
- enzymes lysosomiques, tels que la β-glucosidase dans le cas de la maladie de Gaucher,
- dystrophine et minidistriphine (myophatie),
- tyrosine hydroxylase (Parkinson),
- facteurs de croissance des neurones (Alzheimer),
- CFTR cystic-fibrosis transmembrane conductance regulator (mucoviscidose),
- alpha1-antitrypsine,
- cytokines (interleukines, TNF: facteur de nécrose des tumeurs),
- thymidine kinase du virus Herpes simplex,
- NO synthase,
- récepteurs de l'angiotensine II,
- génes suppresseurs de tumeurs, tels que le gène de la protéine p53,
- protéines du MHC, système majeur d'histocompatibilité, en particulier HLA-B7,
- cytosine désaminase,
- gènes codant pour des ARN sens et antisens,
- gènes codant pour des ribozymes,

c) des gènes à visée vaccinale

- gènes codant pour des antigènes viraux (vaccination), par exemple: gène codant pour la nucléoprotéine du virus de la grippe.

Une classe avantageuse de complexes selon l'invention, comprend ceux dans lesquels:

- le polymère, notamment la polylysine présente un degré de polymérisation d'environ 100 à environ 500, de préférence 190,
- les fonctions $NH_3$+ libres des motifs lysine étant substituées à 60% par des groupements gluconoyle et éventuellement par une molécule constituant un signal de reconnaissance pour 10 000 résidus de lysine lorsque ladite molécule signal possède une affinité d'au moins $10^6$ l $mole^{-1}$ vis à vis du récepteur de la cellule que le complexe doit cibler ou éventuellement par 60 molécules de signal de reconnaissance pour 10 000 résidus de lysine lorsque ladite molécule signal possède une affinité inférieure d'au moins $10^4$ l $mole^{-1}$ vis à vis du susdit récepteur,
- l'acide nucléique présente un poids moléculaire d'environ $6.10^5$ à environ $25.10^6$, et
- le rapport entre le nombre moyen de paires de base de l'acide nucléique par molécule de motif de monomère, notamment la lysine est d'environ 0,9 à environ 1,1, de préférence d'environ 0,95 à environ 1,05.

L'invention concerne également un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres des susdits motifs et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives par rapport au même conjugué polymérique non substitué, facilitant le relargage de l'acide nucléique par la dissociation du complexe,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les fonctions $NH_3^+$ libres des susdits motifs et/ou les groupes hydroxyles des susdits résidus pouvant être également substituées par au moins une molécule qui constitue un signal de reconnaissance reconnu par un récepteur

membranaire cellulaire, sous réserve que le conjugué polymérique contienne au moins 30% de fonctions $NH_3^+$ libres.

Ce conjugué polymérique est un composant intermédiaire des complexes décrits précédemment.

Selon un mode de réalisation avantageux, l'invention concerne un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres des susdits motifs et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives par rapport au même conjugué polymérique non substitué, facilitant le relargage de l'acide nucléique par la dissociation du complexe,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les fonctions $NH_3^+$ libres des susdits motifs des susdits résidus pouvant être également substituées par au moins une molécule qui constitue un signal de reconnaissance reconnu par un récepteur membranaire cellulaire, sous réserve que le conjugué polymérique contienne au moins 30% de fonctions $NH_3^+$ libres.

Selon un mode de réalisation avantageux, l'invention concerne un complexe entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres des susdits motifs et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives, par rapport au même conjugué polymérique non substitué, facilitant le relargage de l'acide nucléique par la dissociation du complexe,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les groupes hydroxyles des susdits résidus pouvant être également substituées par au moins une molécule qui constitue un signal de reconnaissance reconnu par un récepteur membranaire cellulaire, sous réserve que le conjugué polymérique contienne au moins 30% de fonctions $NH_3^+$ libres.

L'invention concerne également un conjugué polymérique chargé positivement, contenant des motifs portant des fonctions $NH_3^+$ libres, notamment des résidus de lysine et étant tel que:

- les fonctions $NH_3^+$ libres des susdits motifs sont substituées dans un rapport d'au moins 10%, avantageusement de 45% à 70% par des résidus non chargés entraînant une diminution des charges positives par rapport au même conjugué polymérique non substitué,
- les susdits résidus possédant en outre les propriétés suivantes:

  → ils comportent au moins un groupe hydroxyle,
  → ils ne correspondent à aucun signal de reconnaissance de cellules,

- les fonctions $NH_3^+$ libres des susdits motifs pouvant être également substituées par une molécule qui constitue un signal de reconnaissance membranaire cellulaire, ce signal de reconnaissance ayant un poids moléculaire inférieur à 5000,

et lorsqu'il est présent, ce signal de reconnaissance peut l'être à raison d'une molécule pour environ 10 000 motifs du conjugué polymérique ou d'environ 60 molécules pour environ 10 000 motifs du conjugué polymérique.

Une classe avantageuse de conjugués polymériques de l'invention contient un groupement polymérique de formule suivante:

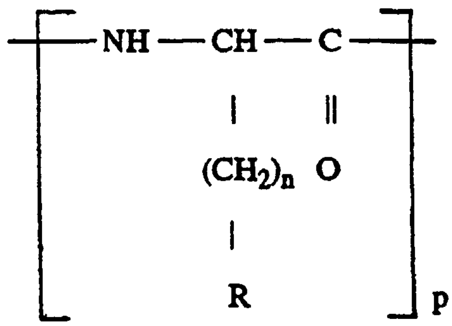

(I)

dans laquelle:

. p est un nombre entier variant de 2 à 500 de préférence de 150 à 200 n est un nombre entier variant de 1 à 5 et vaut de préférence 4,
. ce groupement polymérique contenant un nombre de p radicaux R parmi lesquels:

  * 10% à 70% du nombre des radicaux R représentent un groupe $NH-CO-(CHOH)_m-R_1$, notamment un reste dihydroxypropionoyle, érythrononoyle, thréonoyle, ribonoyle, arabinoyle, xylonoyle, lyxonoyle, gluconolye, galactonoyle, mannonoyle, glycoheptonoyle, glycooctonoyle, m est un nombre entier de 2 à 15, de préférence de 2 à 7,

. $R_1$ représente H ou un radical alcoyle de 1 à 15 atomes de carbone, notamment $CH_3$,

  * le reste des radicaux, c'est-à-dire 30% à 90% du nombre des radicaux R représente $NH_3^+$,
  * R pouvant en outre être constitué de 0 à 25% par une molécule qui constitue un signal de reconnaissance, sous réserve que le conjugué polymérique contienne au moins 30% de fonctions $NH_3^+$ libres, et notamment à raison de 0,5 à 5, avantageusement de 1 molécule pour environ 10 000 motifs,

ou à raison de 10 à 100, avantageusement de 60 molécules pour environ 10 000 motifs.

Une autre classe avantageuse de conjugués polymériques selon l'invention contient un groupement polymérique de formule (II):

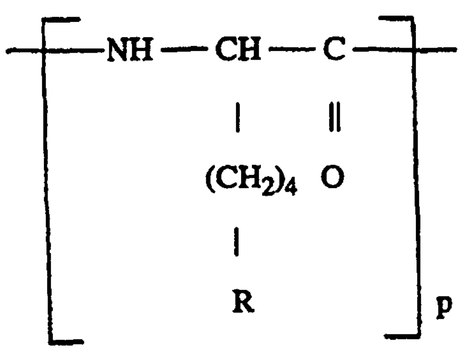

(II)

dans laquelle p a les significations indiquées ci-dessus,

* 10% à 70% du nombre des résidus R représentent un groupe $NH-CO-(CHOH)_m-R_1$, m et $R_1$ ayant la signification indiquée à la ci-dessus,
* le reste des radicaux, c'est-à-dire 30% à 90% du nombre des radicaux R représentent $NH_3^+$.

Une autre classe avantageuse de conjugués polymériques selon l'invention contient un groupement polymérique de formule (II):

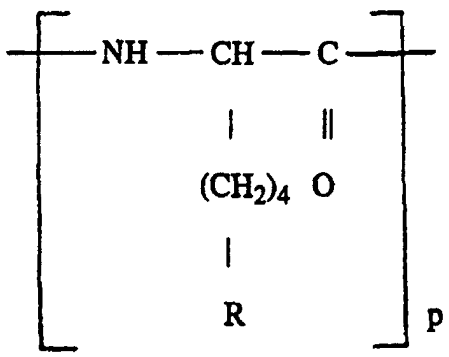

(II)

dans laquelle:

. p a la signification indiquée ci-dessus,
. ce groupement polymérique contenant un nombre p radicaux R parmi lesquels:

* 10% à 70% du nombre des radicaux R représentent un groupe NH-CO-$(CHOH)_m$-$R_1$, m et $R_1$ ayant les significations indiquées ci-dessus,
* le reste des radicaux, c'est-à-dire 30% à 90% des radicaux R représentent d'une part $NH_3^+$ et d'autre part une molécule qui constitue un signal de reconnaissance à raison de 0,5 à 5, avantageusement de 1 molécule pour 10 000 motifs.

Une autre classe avantageuse de conjugués polymériques selon l'invention contient un groupement polymériquede formule (II):

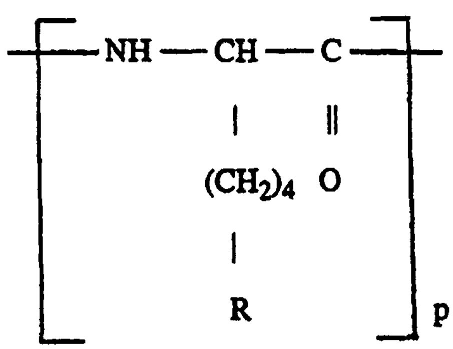

(II)

dans laquelle:

. p a la signification indiquée ci-dessus,
. ce groupement polymérique contenant un nombre p radicaux R parmi lesquels:

* 10% à 70% du nombre des radicaux R représentent un groupe NH-CO-$(CHOH)_m$-$R_1$, m et $R_1$ ayant les significations indiquées à la revendication 2,
* le reste des radicaux, c'est-à-dire 30% à 90% de ces radicaux R représentent d'une part $NH_3^+$ et d'autre part une molécule qui constitue un signal de reconnaissance à raison de 10 à 100, avantageusement de 60 molécules pour environ 10 000 motifs.

Dans les conjugués polymériques de l'invention, le signal de reconnaissance membranaire cellulaire peut être choisi parmi ceux explicités à propos des complexes décrits ci-dessus.
L'invention vise également un procédé de préparation de complexes décrits ci-dessus.
De façon générale, un polymère comportant des amines primaires (fonctions $NH_3^+$ libres) est partiellement substitué par action d'un acide organique hydroxylé (l'acide gluconoïque en particulier), en milieu organique.
Par exemple, un sel de polylysine (notamment sous forme de p-toluène sulfonate) est dissous dans un solvant

organique, (notamment le diméthylsulfoxide) en présence d'une base (notamment la diisopropyléthylamine) et traité par un acide organique hydroxylé activé (notamment la gluconolactone).

Les signaux de reconnaissance sont fixés sur le polymère, soit avant soit après l'introduction des acides organiques hydroxylés.

Les signaux de reconnaissance peuvent être liés aux groupements aminés du polymère ou aux hydroxyles des acides organiques hydroxylés; ces substitutions suivent l'un quelconques des protocoles connus de l'homme de l'art.

A titre d'exemple de fixation des signaux de reconnaissance sur la polylysine gluconoylée, on indique ci-après la fixation d'osides ou d'oligosides.

1) Fixation d'osides.

Des osides simples sont sous la forme de dérivés phénylisothiocyanates qui peuvent réagir dans le DMSO en présence de diisopropyléthylamine avec les fonctions ε-amine des résidus lysines libres de la polylysine gluconoylée comme précédemment décrit dans: Midoux et al., 1993, Nucleic Acids Res., 21: 871-878.

2) Fixation d'oligosides.

Des oligosides complexes tels que les asialo-oligosides de types triantennaire ou tétraantennaire ou Lewis x sont obtenus sous forme de glycopeptides selon la méthode décrite par Nadia Normand Sdiqui, 1995, Synthèse de glycoconjugués spécifiques de lectines membranaires et leur utilisation pour cibler oligonucléotides et gènes. (Thèse universitaire du 5 janvier 1995, Université d'Orléans).

Les glycopeptides sous forme phénylisothiocyanates sont fixés sur les fonctions ε-amine des résidus lysines libres de la polylysine gluconoylée comme précédemment décrit dans: Midoux et al., 1993, Nucleic Acids Res., 21: 871-878.

Le complexe acide nucléique/conjugué polymérique est obtenu en mélangeant une solution de l'acide nucléique concerné et une solution du conjugué polymérique. De préférence, lesdites solutions sont préparées à partir du sérum physiologique ou d'un tampon ou d'un milieu cytocompatible.

L'invention concerne également l'utilisation d'un complexe ou d'un conjugué selon l'invention, pour la transfection *in vitro, ex vivo* ou *in vivo* de cellules à l'aide d'un gène, notamment ceux définis précédemment.

L'invention vise également l'utilisation d'un complexe ou d'un conjugué selon l'invention, pour transfecter des cellules qui peuvent être choisies parmi:

- des cellules souches hématopoiétiques;
- cellules du foie;
- cellules des muscles squelettiques;
- cellules de la peau:
  - . fibroblastes,
  - . kératinocytes,
  - . cellules dendritiques,
  - . mélanocytes.
- cellules des parois vasculaires;
  - . endothéliales;
  - . musculaires lisses;
- cellules épithéliales des voies aériennes;
- cellules du système nerveux central;
- cellules cancéreuses;
- cellules du système immunitaire, telles que lymphocytes, macrophages, cellules NK etc...

Une méthode de transfection *in vitro, ex vivo* ou *in vivo* de l'invention comprend la mise en présence un complexe de l'invention, dans un milieu contenant des cellules à transfecter, dans des conditions telles qu'il y a:

- passage du complexe à partir du milieu dans le cytoplasme des cellules,
- relargage de l'acide nucléique impliqué dans le susdit complexe dans le cytosol des cellules,
- transcription et expression de l'acide nucléique dans les cellules transfectées.

L'acide nucléique est délivré dans le cytosol et/ou dans le noyau de la cellule où il doit s'exprimer.

L'invention concerne également des compositions pharmaceutiques, comprenant à titre de substance active, l'un au moins des complexes ou l'un au moins des conjugués selon l'invention, en association avec un véhicule pharmaceutiquement acceptable.

Les complexes ou conjugués de l'invention peuvent également être partie à une trousse ou un kit, comprenant par exemple:

- un conjugué polymérique selon l'invention, par exemple, la polylysine, substituée par un résidu entraînant une diminution des charges des groupes $NH_3^+$ libres, ce conjugué polymérique étant apte à comporter éventuellement un signal de reconnaissance, lequel est préalablement fixé ou non sur le conjugué polymérique, ledit signal de reconnaissance étant fonction de la cellule à cibler,
- éventuellement un plasmide contenant au moins un gène à transférer, et le système de régulation du susdit gène,
- des réactifs permettant la fixation éventuelle du signal de reconnaissance sur le susdit conjugué polymérique,
- des réactifs permettant la formation d'un complexe selon l'invention entre le conjugué polymérique et le gène à transférer,
- des réactifs permettant la transfection de la cellule par le susdit complexe.

Concernant le signal de reconnaissance, il faut souligner qu'il n'est pas nécessairement présent sur le conjugué polymérique. Il peut en effet être à part dans la trousse et être greffé sur le conjugué polymérique avant l'utilisation. Par ailleurs, le signal de reconnaissance peut être absent de la trousse et l'utilisateur peut, en fonction des cellules à cibler, utiliser le signal de reconnaissance de son choix pour le greffer sur le conjugué polymérique de la trousse.

L'invention concerne également l'utilisation d'un complexe ou d'un conjugué selon l'invention, pour la préparation d'un médicament destiné, par exemple, au traitement d'une déficience métabolique congénitale ou acquise, ou au traitement de tumeurs, ou pour la préparation d'un vaccin, par exemple vaccin contre la grippe.

Les conjugués polymériques et les complexes de l'invention peuvent être utilisés pour transfecter *ex vivo* toute cellule apte à présenter un antigène, par exemple, des précurseurs de macrophages, des macrophages, des cellules B ou des cellules dendritiques.

Lorsqu'on souhaite transfecter des macrophages, ceux-ci peuvent être préparés selon la méthode décrite par M. Chokri et coll. dans Anticancer Research 12, 2257-2260, 1992.

Les complexes et conjugués polymériques de l'invention peuvent être utilisés pour transfecter des macrophages en dehors de l'organisme, lorsqu'ils sont en milieu de culture, avant ou après séparation par élutriation.

On peut utiliser une méthode analogue à celle utilisée pour la transfection des cellules HepG2, mais en utilisant un oligosaccharide ciblant, par exemple le récepteur du mannose; (pour la transfection des cellules HepG2, on peut se reporter aux exemples ci-après ou à l'article de C. Sureau, J. L. Romet-Lemonne, J. Mullins et M. Essex: "Production of hepatitis B virus by a differentiated human hepatoma cell line after transfection with cloned circular HBV DNA. Cell, **47,** p. 37-47, 1986, ou à l'article de Midoux et al., intitulé: "Specific gene transfer mediated by lactosylated poly-L-lysine into hepatoma cells", Nucleic Acids Research, 1993, vol. 21, N° 4, pages 871-878).

Les macrophages transfectés "*ex vivo*" sont réinjectés au patient après vérification de l'efficacité de la transfection selon des méthodes classiques d'immunomarquage.

L'acide nucléique dans le complexe de l'invention peut être:

- soit un gène à visée thérapeutique pour pallier une déficience métabolique congénitale ou acquise, (par exemple, facteurs de coagulation, tel que le facteur VIII ou le facteur IX),
- soit un gène à visée vaccinale (par exemple, un gène codant pour une protéine exprimée à la surface d'une tumeur, ou un gène de protéine virale, bactérienne ou parasitaire).

Dans le cas d'une vaccination par réinjection de macrophages transfectés, la protéine antigénique est exprimée et est en partie présente à la surface du macrophage permettant une présentation antigénique MHC type 1 dépendante.

L'acide nucléique dans le complexe de l'invention peut être également un gène conférant aux macrophages des propriétés nouvelles soit directement, soit par expression de cytokines,

. cytokines ayant un effet directement sur le macrophage, lui conférant des propriétés physiologiques nouvelles;
  par exemple: - transfection du gène du $\gamma$ interféron; dans ce cas le macrophage est auto activé en permanence, ce qui augmente ses propriétés cytotoxiques;

  - transfection du gène du TNF$\alpha$ modifié ou non; dans ce cas il y a augmentation des capacités anti tumorales des macrophages;

- cytokines ayant un effet sur les populations cellulaires au voisinage des macrophages transfectés;
  par exemple: - transfection du gène de l'IL2 pour stimuler les cellules T cytotoxiques au voisinage de la tumeur colonisée par les macrophages.

**Description des figures**

Figure 1:

Elle représente un fragment de polylysine gluconoylée.

Figure 1 bis:

Elle représente un fragment de polylysine dans laquelle certaines des fonctions $NH_3^+$ de la polylysine sont substituées telles que R = $NH_3^+$ ou NNHCO$(CHOH)_m$ $R_1$, $R_1$ ayant les significations indiquées ci-dessus.

Figure 1 ter:

Elle représente l'analyse par électrophorèse sur un gel d'agarose à 0,6% du plasmide pSV2Luc complexé avec des quantités variables de polylysine gluconoylée.

Les complexes ADN/polylysine gluconoylée sont préparés en ajoutant goutte à goutte sous mélange constant, des quantités variables (de 0 à 8 μg) de polylysine gluconoylée dans 60 μl de DMEM, à 2 μg (0,6 pmol) de plasmide pSV2Luc dans 140 μl de DMEM. Après 30 minutes à 20°C, 20 μl de chaque échantillon est analysé par électrophorèse à travers un gel d'agarose à 0,6% contenant du bromure d'éthidium pour visualiser l'ADN dans un tampon Tris borate EDTA (Tris 95 mM, acide borique 89 mM et EDTA 2,5 mM), pH 8,6. pLK-GlcA/ADN = 0 (a), 15 (b), 30 (c), 60 (d), 90 (e), 120 (f), 150 (g), 180 (h), 210 (i) et 240 (j).

Figure 2:

Elle représente le transfert de gène dans les cellules HepG2 en utilisant des polylysines gluconoylées (GlcA-pLK).

Les valeurs des gluconoyles sont déterminées par dosage colorimétrique.

Les complexes ADN/polymère formés entre le plasmide pSV2Luc et la polylysine substituée par différentes quantités de résidus gluconoyles (de 15 à 70%) ont été déterminés par électrophorèse en gel d'agarose. La polylysine substituée par plus de 140 résidus gluconoyles ne peut former un complexe avec le plasmide.

Les cellules HepG2 ont été incubées à 37°C pendant 4 heures en présence de 100 μM de chloroquine avec 1.5 nM de plasmide complexé avec chacun des conjugués. Le milieu a été éliminé et les cellules ont été incubées en absence de chloroquine et de plasmide. L'expression du gène de la luciférase a été déterminée 48 heures plus tard en mesurant l'activité de la luciférase dans les lysats cellulaires. Les valeurs relatives de la lumière émise (RLU) ont été exprimées en mg de protéine ce qui correspond à 1,2 million de cellules HepG2, en fonction du rapport molaire GlcA/pLK et du degré de substitution de polylysine (%).

Figure 2 bis:

Elle représente le transfert de gène dans les cellules HepG2 en utilisant des polylysines gluconoylées dans les conditions identiques à celles décrites pour la figure 2.

La différence avec la figure 2 est que les valeurs des gluconoyles sont calculées à partir des résultats RMN.

Figure 2 ter:

Elle concerne la formation des complexes ADN/polylysine gluconoylée.

Elle concerne notamment l'étude de la quantité de polylysine gluconoylée complexée par molécule d'ADN (plasmide de 5 kb) en fonction du rapport molaire polylysine gluconoylée/ADN (P/ADN). Les complexes ADN/ polylysine gluconoylée sont formés dans 1 ml de DMEM entre le plasmide pSV2Luc (3 pmole) et de la polylysine gluconoylée marquée avec la fluorescéine et contenant 70 résidus gluconoyle. Les solutions sont centrifugées à grande vitesse. La quantité de polylysine gluconoylée complexée par molécule d'ADN (colonnes blanches) est la quantité totale de polylysine gluconoylée (déterminée en mesurant l'absorbance à 495 nm des solutions avant centrifugation (colonne hachurée) moins la quantité de polylysine gluconoylée libre (déterminée en mesurant l'absorbance à 495 nm des surnageants après centrifugation: colonne noire).

Figure 2 quater:

Elle concerne la formation des complexes ADN/polylysine gluconoylée en fonction du nombre de résidus gluconoyles fixés sur la polylysine.

Les complexes ADN/polylysine gluconoylée sont formés dans 0,2 ml de DMEM entre le plasmide pSV2Luc (0,6 pmole) et des polylysines gluconoylées contenant jusqu'à 110 résidus gluconoyles. Le rapport $NH_3^+$/nucléotide représente le nombre de charges positives par polylysine gluconoylée multiplié par le nombre de polylysine gluconoylée par ADN divisé par le nombre de charges négatives portées par l'ADN dans les complexes formés avec le plus petit rapport molaire polylysine gluconoylée/ADN induisant un retard complet de l'ADN en électrophorèse en gel d'agarose. En insert: on a représenté la variation de la quantité de polylysine gluconoylée par molécule d'ADN dans les complexes formés avec le plus petit rapport polylysine gluconoylée/ADN induisant un retard complet de l'ADN en électrophorèse en gel d'agarose. P/ADN est la quantité de polylysine gluconoylée par molécule d'ADN; GlcA/pLK ets le nombre moyen de résidus gluconoyles par molécule de polylysine.

Figure 3:

Elle représente le transfert de gène dans les cellules HepG2 en utilisant des polylysines gluconoylées (GlcA-pLK)

Les complexes ADN/polymère formés entre le plasmide pSV2Luc et la polylysine substituée par différentes quantités de résidus gluconoyles (de 15 à 70%) ont été déterminés par électrophorèse en gel d'agarose. La polylysine substituée par plus de 140 résidus gluconoyles ne peut former un complexe avec le plasmide. Les cellules HepG2 ont été incubées à 37°C pendant 4 heures en présence de 100 μM de chloroquine avec 1.5 nM de plasmide complexé avec chacun des conjugués. Le milieu a été éliminé et les cellules ont été incubées en absence de chloroquine et de plasmide. L'expression du gène de la luciférase a été déterminée 48 heures plus tard en mesurant l'activité de la luciférase dans des lysats cellulaires. Les valeurs relatives de la lumière émise (RLU) ont été exprimées par rapport à celle obtenue dans la même expérience lors de la transfection des cellules HepG2 avec le conjugué polylysine lactosylé ($Lact_{60}$pLK). En ordonnés, on a représenté les valeurs RLU/RLU de $Lact_{60}$pLK, en fonction du rapport molaire GlcA/pLK d'une part, et de degré de substitution de polylysine (%).

Figure 3 bis:

Elle concerne l'influence du nombre de résidus lactose.

L'activité optimale d'un conjugué polymérique (polymère substitué par des lactoses) apparaît lorsque 30% des groupes $NH_3^+$ sont substitués par du lactose.

Figure 4:

Elle concerne le transfert de gène dans différentes cellules en utilisant la polylysine gluconoylée (GlcA-pLK)

Un complexe ADN/polymère a été formé entre le plasmide pSV2Luc et la polylysine substituée par 120 résidus gluconoyles. Les cellules ont été incubées à 37°C pendant 4 heures en présence de 100 μM de chloroquine avec 1.5 nM de plasmide complexé avec la polylysine gluconoylée.

Le milieu a été éliminé et les cellules ont été incubées en absence de chloroquine et de plasmide. L'expression du gène de la luciférase a été déterminée 48 heures plus tard en mesurant l'activité de la luciférase dans les lysats cellulaires. Les valeurs relatives de la lumière émise (RLU) ont été exprimées par mg de protéine. MacroH = macrophages humains dérivés de monocytes; RBE4 = cellules endothéliales de cerveau de rat; HEL = cellules leucémiques de la lignée érythroïde.

Figure 5:

Elle représente le transfert de gène dans différentes cellules en utilisant la polylysine gluconoylée (GlcA-pLK)

Un complexe ADN/polymère a été formé entre le plasmide CMVLuc et la polylysine substituée par 120 résidus gluconoyles. Les cellules ont été incubées à 37°C pendant 4 heures en présence de 100 μM de chloroquine avec 1.5 nM de plasmide complexé avec la polylysine gluconoylée. Le milieu a été éliminé et les cellules ont été incubées en absence de chloroquine et de plasmide. L'expression du gène de la luciférase a été déterminée 48 heures plus tard en mesurant l'activité de la luciférase dans les lysats cellulaires. Les valeurs relatives de la lumière émise (RLU) ont été exprimées par mg de protéine. 3LL = cellules (souris) du carcinome pulmonaire de Lewis; MacroH = macrophages humains dérivés de monocytes; RBE4 = cellules endothéliales de cerveau de rat; HepG2 = hépatocarcinome humain; HEL = cellules leucémiques de la lignée érythroïde; K562 = cellules leucémiques de la lignée érythroïde.

Figure 6:

Elle concerne la mesure de la dissociation des complexes formés entre le plasmide pSV2Luc et la polylysine (degré de polymérisation = 190) substituée par des lactoses.
Des complexes ont été formés entre le plasmide pSV2Luc avec soit la polylysine (pLK), soit la polylysine substituée par 60 résidus de lactose ($Lact_{60}$pLK), soit la polylysine substituée par 80 résidus de lactose ($Lact_{80}$pLK). Les complexes ont été formés dans une solution de NaCl 0.15 M. la concentration de NaCl a été ensuite augmentée. Les solutions de complexes ADN/polymère à différentes concentrations en NaCl ont été filtrées au travers d'une membrane de nitrocellulose 0.45 µm. dans cette expérience, l'ADN non complexé à la polylysine passe au travers du filtre alors que l'ADN complexé est retenu sur le filtre. La quantité d'ADN dissociée de la polylysine a été déterminée en mesurant la quantité d'ADN présent dans les filtrats en utilisant le DAPI (4',6-diamino-2-phenylindole), (λem = 450 nm; λexc = 360 nm) (Sigma)) comme sonde fluorescente. On a porté en ordonnés le pourcentage d'ADN lié/ADN libre, en fonction de la concentration en NaCl (M). La courbe comportant des ○ correspond à pLK, celle comportant des ● correspond à pLK,-$Lact_{60}$ et celle des ▽ correspond à pLK,-$Lact_{80}$.

Figure 7:

Elle concerne la mesure de la solubilité des complexes.
Des complexes ADN/polymère ont été formés dans une solution de NaCl 0.15 M entre le plasmide pSV2Luc avec soit la polylysine (pLK), la polylysine gluconoylée ($GlcA_{120}$pLK) ou la polylysine substituée par 60 résidus de lactose ($Lact_{60}$pLK). Après 30 minutes à 20°C, l'absorbance à 610 nm des solutions a été mesurée.

Figure 7 bis:

Elle concerne la mesure de la solubilité des complexes.
Des complexes ADN/polymère ont été formés dans une solution de NaCl 0,15 M entre le plasmide pSV2Luc avec soit la polylysine substituée par 30 résidus de lactose (pLK, -$Lact_{30}$) (courbe déterminée par les carrés vides), ou la polylysine substituée par 30 résidus lactose et substituée par 50 résidus de gluconoyle (pLK, -$Lact_{30}$, $GlcA_{50}$) (courbe déterminée par les triangles noirs). Après 30 minutes à 20°C, l'absorbance à 610 nm des solutions a été mesurée.

Figure 8:

Elle concerne le transfert de gène dans une lignée myéloïde.
Les cellules myéloïdes HEL sont transfectées par un complexe réalisé entre un plasmide contenant le gène de la luciférase (PUT650) et la polylysine gluconoylée et biotinylée. Ce complexe est ensuite associé au facteur de croissance des cellules souches (Stem Cell Factor, SCF) par l'intermédiaire de la streptavidine. Les valeurs des histogrammes RLU sont exprimées en unité relative de luminescence par mg de protéines extraites. Les transfections sont réalisées avec (A) le plasmide seul, (B) le plasmide complexé à la polylysine gluconoylée et biotinylée, (C) le complexe (plasmide/polylysine gluconoylée et biotinylée) associé à la streptavidine et (D) le complexe (plasmide/polylysine gluconoylée et biotinylée) associé à la streptavidine et au facteur de croissance des cellules souches.

Figure 9:

Elle concerne le spectre RMN à 300 MHz dans $D_2O$ de la polylysine sous forme p-toluène sulfonate.

1,28 à 1,88 ppm: 6 H des carbones 3, 4 et 5 de la lysine
2,41 ppm: groupe $CH_3$ du p-toluène sulfonate
2,84 à 2,95 ppm: 2 H du carbone 6
4,3 ppm: 1 H du carbone 2
7,38 et 7,7 ppm: protons aromatiques du p-toluène sulfonate

Figure 10:

Elle concerne le spectre RMN à 300 MHz dans $D_2O$ de la polylysine substituée par 73 résidus gluconoyle.

1,28 à 1,88 ppm: 6 H des carbones 3, 4 et 5 de la lysine
2,41 ppm: groupe $CH_3$ du p-toluène sulfonate
2,75 ppm: trace de DMSO
2,97 ppm: 2 H du carbone 6 d'un résidu lysine non gluconoylé

3,26 ppm: 2 H du carbone 6 d'un résidu lysine gluconoylé

3,68 à 3,88 ppm: 4 H d'un résidu gluconoyle (voir spectre n° 1,856B dans: The Aldrich Library of $^{13}C$ and $^{1}H$ FTNMR Spectra EdI C.J. Pouchert and J. Behnke, Vol. 1)

4,3 ppm: 1 H du carbone 8 d'un résidu gluconoyle et 1H du carbone 2 d'un résidu lysine

Tableau 1.

| Transfection des cellules HepG2 par la polylysine lactosylée et gluconoylée. | | | | |
|---|---|---|---|---|
| RLU x $10^{-3}$/mg de protéine | 5.2 | 19 | 671 | 650 |
| Lact/pLK | 0 | 30 | 30 | 60 |
| GleA/pLK | 0 | 0 | 30 | 0 |

Les cellules HepG2 ont été incubées à 37°C en présence de 100 μM de chloroquine et 1.5 nM de plasmide complexé avec chacun des conjugués. Après 4 heures, le milieu a été éliminé et les cellules ont été incubées en absence de chloroquine et de plasmide. L'expression du gène de la luciférase a été déterminée 48 heures plus tard en mesurant l'activité de la luciférase dans les lysats cellulaires. Les valeurs relatives de la lumière émise (RLU) ont été exprimées par mg de protéine ce qui correspond à 1,2 million de cellules HepG2. Lact/pLK est le nombre de molécules de lactose par molécule de polylysine et GlcA/pLK est le nombre de molécules de gluconoyle par molécule de polylysine.

Tableau II.

| Transfection des cellules HepG2 par la polylysine gluconoylée et biotinylée. | |
|---|---|
| | RLU x $10^{-3}$/mg de protéine |
| ADN/GlcA, Bio-pLK/Strep/Bio-LactBSA | 966 |
| ADN/GlcA, Bio-pLK/Strep/Bio-BSA | 248 |
| ADN/GlcA, Bio-pLK/Strep | 237 |
| ADN/GlcA, Bio-pLK | 67 |
| ADN | 0.2 |

Les cellules HepG2 ont été incubées à 37°C en présence de 100 μM de chloroquine avec 1.5 nM de plasmide libre ou complexé avec chacun des conjugués. Après 4 heures, le milieu a été éliminé et les cellules ont été incubées en absence de chloroquine et de plasmide. L'expression du gène de la luciférase a été déterminée 48 heures plutard en mesurant l'activité de la luciférase dans les lysats cellulaires. Les valeurs relatives de la lumière émise (RLU) ont été exprimées par mg de protéine ce qui correspond à 1,2 million de cellules HepG2. GlcA,Bio-pLK = polylysine substituée par 60 gluconoyles et 2.5 biotines; Strep = streptavidine; Bio-LactBSA = sérum albumine lactosylée et biotinylée; Bio-BSA = sérum albumine biotinylée.

Composants chimiques

La luciférine, la chloroquine, le Triton X 100 et l'acide bicinchoninique proviennent de Sigma (St. Louis, MO USA); la L-glutamine, le diméthylsulfoxide ($Me_2SO$), l'ATP, le glycérol et $MgCl_2$ proviennent de Merck (Darmstadt, Allemagne); le dithiothréitol et le D-gluconolactone proviennent de Serva (Heidelberg, Allemagne); la diisopropyléthylamine, l'acide p-toluène sulfonique, l'EDTA proviennent de Aldrich (Strasbourg, France); Dowex 2 x 8, (0,3-0,9 mm de diamètre) provient de Bio-Rad (Richmond, CA); le 4-isothiocyanatophényl-β-D-lactoside, le 4-isothiocyanatophényl-β-D-galactopyranoside sont préparés comme décrit précédemment (Monsigny, M., Roche, A.C. et Midoux, P. (1984), Uptake of neoglycoproteins via membrane lectin(s) of L 1210 cells evidenced by quantitative flow cytofluorometryand drug targeting. Biol., Cell., **51**, 187-196); la poly-L-lysine, HBr (30 000-50 000) (poids moléculaire moyen = 40 000, degré de polymérisation = 190) provient de Bachem Feinchemikalien (Bubendorf, Suisse). La poly-L-lysine, HBr (1 g dans 200 ml de $H_2O$) est passée sur une colonne échangeuse d'anions (Dowex 2 x 8, sous forme $OH^-$, 0,3-0,9 mm de diamètre, 35 x 2.5 cm) de façon à enlever le bromure (Derrien, D., Midoux, P., Petit, C., Negre, E., Mayer, R., Monsigny, M. et Roche, A.C. (1989), Muramyl dipeptide bound to poly-L-lysine substituted with mannose and gluconoyl residues as macrophage activators. Glycoconjugate, J., **6,** 241-255) qui est très cytotoxique pour les cellules (Weiss, S.J., Test, S.T., Eckmann, C.M., Roos, D. et Regiani, S. (1986), Brominating oxidants generated by human eosinophils., Science, **234,** 200-202). La solution effluente est neutralisée avec 10% d'acide p-toluène sulfonique dans l'eau (un composé non cytotoxique) et lyophilisée. La sérum albumine bovine lactosylée (Lact-BSA, comportant un nombre moyen de 39 résidus de lactose) est préparée comme décrit précédemment (Roche, A.C., Barzilay, M., Midoux, P.,

Junqua, S., Sharon, N. and Monsigny, M., (1983), Sugar-specific endocytosis of glycoproteins by lewis lung carcinoma cells. J., Cell. Biochem., **22,** 131-140; Monsigny, M., Roche, A.C. et Midoux, P. (1984), Uptake of neoglycoproteins via membrane lectin(s) of L 1210 cells evidenced by quantitative flow cytofluorometry and drug targeting. Biol., Cell., **51,** 187-196).

Préparation de la polylysine gluconoylée

La poly-L-lysine sous forme hydromide, pLK,HBr 30 000 - 50 000 (masse moléculaire moyenne = 40 000; degré de polymérisation moyen = 190) provient de Bachem Feinchemikalien (Bubendorf, Suisse). La polylysine, HBr (1 g dans 200 ml $H_2O$) est passée sur une colonne échangeuse d'anions (Dowex 2 x 8, forme $OH^-$; 35 x 2,5 cm) dans le but d'enlever le brome qui est toxique pour les cellules. La solution de polylysine est neutralisée avec une solution d'acide p-toluène sulfonique à 10% dans l'eau puis lyophilisée.

La polylysine est partiellement substituée avec des résidus gluconoyles (GlcA) comme suit: la polylysine sous forme p-toluène sulfonate (50 mg; 0.86 µmoles) dissoute dans 3 ml de DMSO (diméthylsufoxyde) en présence de diisopropyléthylamine (37 µl; 205 µmoles) et 1% d'eau, est mise à réagir pendant 24 h à 20°C avec des quantités variables de D-gluconolactone allant de 11 mg (61µmol) à 35 mg (194 µmol). La polylysine gluconoylée est précipitée en ajoutant 10 volumes d'isopropanol. Après centrifugation (1800 g x 15 min), le culot est lavé avec de l'isopropanol et récupéré après une nouvelle centrifugation. Le culot est repris dans l'eau bidistillée et la solution est lyophilisée. Le nombre moyen de résidus gluconoyle fixé par molécule de la polylysine ***est*** déterminé en mesurant le nombre moyen de résidus $\varepsilon$-$NH_2$ lysine libre restant sur la polylysine en utilisant le dosage colorimétrique à l'acide 2,4,6-trinitrobenzene sulfonique TNBS (Fields, R. (1971) The measurement of amino groups in proteins and peptides. Biochem., J., **124**, 581-590). Le poids moléculaire moyen est de 58 000.

Préparation de conjugués de polylysine lactosylée et gluconoylée.

Les polylysines substituées soit avec 30 résidus de lactose ($Lact_{30}$pLK) soit avec 60 résidus de lactose ($Lact_{60}$pLK) ont été préparées comme précédemment décrit (Midoux, P., Mendes, C., Legrand, A., Raimond, J., Mayer, R., Monsigny, M., & Roche, A.C. (1993), Specific gene transfer mediated by lactosylated poly-L-lysine into hepatoma cells. Nucleic Acids, Res., **21,** 871-878). La polylysine lactosylée contenant 30 résidus de lactose (50 mg: 0.745 µmol) est mise à réagir pendant 24h à 20°C avec la D-gluconolactone (7,6 mg; 43 µmol) en présence de diisopropyléthylamine (24 µl; 200 µmol) et 1% de $H_2O$. Le polymère $Lact_{30}$,-GlcA-pLK est précipité et purifié comme décrit précédemment. Le nombre moyen de résidus gluconoyles liés par molécule de conjugué est déterminé en mesurant les groupes $\alpha$-amino de la lysine restant sur la polylysine en utilisant la méthode colorimétrique TNBS (Fields, R. (1971) The measurement of amino groups in proteins and peptides, Biochem., J., **124**, 581-590) est trouvé égal à 30.

Biotinylation de la polylysine et de la polylysine gluconoylée.

La polylysine gluconoylée (contenant 60 résidus gluconoyles) est substituée par de la biotine: le polymère (20 mg; 0.33 µmol) dissous dans 0.5 ml de tampon carbonate/bicarbonate de sodium 0.1 M, pH 9.0, contenant NaCl 0.3 M, est mis à réagir pendant 20 h à 20°C avec 0.93 mg (1.7 µmol) de sulfosuccinimidyl-6-(biotinamido)hexanoate (NHS-LC-biotin, Pierce). Le polymère est précipité en ajoutant 10 volumes d'isopropanol. Après centrifugation (1800 g x 15 min), le culot est lavé avec de l'isopropanol et récupéré après une nouvelle centrifugation. Le culot est repris dans l'eau bidistillée et la solution est lyophilisée. Le nombre moyen de résidu biotine fixé par molécule de polymère, déterminé en utilisant le dosage colorimétrique adapté de Green (Green, N. M. (1965), A spectrophotomatic assay for avidin and biotin based on binding dyes by avidin. Biochem., J., **94**, 23c-24c) en utilisant l'acide 2-(4'-hydroxyazobenzene)benzoïque (HABA) et la streptavidine, est trouvé égal à 2.5.

Préparation de néoglycoprotéines biotinylées

La BSA et la BSA lactosylée (Lact-BSA) (0.23 µmole) dissoutes dans 5 ml de tampon de carbonate sodium 0.1 M, pH 9.0, contenant NaCl 0.3 M sont mises à réagir pendant 20 h à 20°C, avec NHS-LC-biotine (0,65 mg; 1,2 µmol). Les conjugués sont purifiés par filtration sur gel de Trisacryl GF05 (taille de colonne 20 x 2 cm) (Sepracor, Villeneuve la Garenne, France) dans $H_2O$ contenant 5% de n-butanol, puis lyophilisé. Les nombres moyens de résidus de biotine liés par molécule de protéine sont déterminés en utilisant une méthode colorimétrique avec HABA, adaptée de Green (Green, N. M. (1965), A spectrophotomatic assay for avidin and biotin based on binding dyes by avidin. Biochem., J., **94**, 23c-24c) et sont de 1 pour BSA et 2 pour Lact-BSA. Les masses moléculaires moyennes de BSA et Lact-BSA sont 68 000 et 87 200 respectivement.

Cellules et cultures de cellules

Les lignées cellulaires HepG2 (hépatocarcinome humain, ATCC 8065 HB) qui possèdent une lectine membranaire reconnaissant les glycoprotéines se terminant par des résidus β-D-galactose (Schwartz, A.L., Fridovich, S. E., Knowles, B.B. & Lodish, H.F. (1981) Characterization of the asialoglycoprotein receptor in a continuous hepatoma line. J., Bio., Chem., **256**, 8878-8881), les cellules HEL (ATCC TIB 180) et K-562 (ATCC CCL 243) sont cultivées respectivement en milieu DMEM (GIBCO, Reufrewshire, U.K.) et en milieu RPMI 1640 plus 10% de sérum bovin foetal (GIBCO) désactivé par la chaleur, plus 2 mM de L-glutamine (Merck), plus des antibiotiques (100 unités/ml de pénicilline et 100 µg/ml de streptomycine) (Eurobio., Paris, France). Les macrophages humains dérivant de monocytes du sang sont préparés comme décrit dans Roche et al., 1985 (Roche, A.C., Midoux, P., Bouchard, P. and Monsigny, M. (1985) Membrane lectins on human monocytes: maturation-dependent modulation of 6-phosphomannose and mannose receptors. FEBS Letters, **193**, 63-68). Les cellules 3LL sont cultivées comme décrit dans Roche et al., 1983 (Roche, A. C., Barzilay, M., Midoux, P., Junqua, S., Sharon, N. and Monsigny, M. (1983) Sugar-specific endocytosis of glycoproteins by lewis lung carcinoma cells. J., Cell., Biochem., **22,** 131-140). Les cellules RBE4, données par P.O. Couraud (Hôpital Cochin, Paris) sont cultivées sur collagène dans un milieu *a*-MEM et $HamF_{10}$ (50/50, volume; volume) plus 10% de sérum bovin foetal désactivé par la chaleur, plus 2 mM de L-glutamine, plus des antibiotiques (100 unités/ml de pénicilline et 100 µg/ml de streptomycine) en présence de TGFβ.

Les plasmides

Le plasmide pSV2Luc (5.0 kb) est fourni par le Dr. A.B. Brasier (Massachusetts General Hospital, Boston) (Brasier, A.R., Tate, J.E. & Habener, J.F. (1989) Optimized use of the firefly luciferase assay as a reporter gene in mammalian cell lines. Biotechniques, **7**, 1116-1123). Le plasmide CMVLuc est fourni par le Dr. A. Dautry-Varsat (Institut Pasteur, Paris).

Formation de complexes plasmide/conjugué de polylysine optimaux.

Seuls les complexes pour lesquels aucune migration de l'ADN se produit en électrophorèse sur un gel d'agarose, et ainsi appelés complexes d'ADN/polymère optimaux sont utilisés pour transfecter les cellules. Les rapports molaires entre le polymère et l'ADN nécessaires pour former des complexes plasmide pSV2Luc/polymère optimaux sont déterminés par électrophorèse sur un gel d'agarose à 0.6%: les complexes sont préparés en ajoutant goutte à goutte sous mélange constant, des quantités variables de conjugués de polylysine dans 60 µl de DMEM, à 2µg (0.6pmol) de plasmide pSV2Luc dans 140 µl de DMEM. Après incubation pendant 30 minutes à 20°C, 20 µl de chaque échantillon est analysé par électrophorèse à travers un gel d'agarose à 0.6% contenant du bromure d'éthidium pour visualiser l'ADN dans un tampon Tris borate EDTA (Tris 95 mM, acide borique 89 mM et EDTA 2.5 mM), pH 8.6.

Préparation des complexes ADN/vecteurs.

*Complexes plasmide pSV2Luc/conjugué de polylysine.*

Des complexes ADN/polymère optimaux sont préparés en ajoutant goutte à goutte sous agitation constante la polylysine ou un conjugué de poly-L-lysine ($Lact_{60}$pLK, $GlcA_x$-pLK, $30 \leq x \leq 130$, avec $Lact_{30}$pLK, ou $Lact_{30}$-$GlcA_{30}$-pLK) dans 0.6 ml de DMEM à 20 µg (6 pmol) de plasmide pSV2Luc dans 1.4 ml de DMEM. La solution est maintenue pendant 30 minutes à 20°C.

*Complexes de plasmide pSV2Luc/pLK-streptavidine-néoglycoprotéine.*

Les complexes de plasmide pSV2Luc/polylysine biotinylée optimaux, sont formés en ajoutant goutte à goutte sous mélange constant 10 µg (172 pmol) de polylysine gluconoylée et biotinylée (contenant 60 résidus gluconoyles) dans 290 µl de DMEM à 10 µg (3 pmol) de plasmide pSV2Luc dans 0.7 ml de DMEM (rapport molaire entre le polymère et l'ADN proche de 57:1). La solution est maintenue pendant 30 minutes à 20°C. Ensuite les néoglycoprotéines biotinylées (Lact-BSA et BSA) (377 pmol) dans 0.5 ml de DMEM sont ajoutées avec agitation constante à 1 ml de complexe plasmide pSV2Luc/polylysine biotinylée, et puis la streptavidine (27.5 µg; 490 pmol) dans 0.5 ml de DMEM est ajoutée sous agitation (rapport molaire entre la néoglycoprotéine et l'ADN voisin de 125:1) et la solution est maintenue pendant 30 minutes à 20°C.

Transfert de gène

5 x $10^5$ cellules HepG2 par puits sont déposées au jour 0 sur des plaques de culture de tissus de 12 puits, respectivement. Au jour 1, après avoir retiré le milieu, la solution (2 ml) contenant le complexe plasmide/conjugué de polylysine supplémenté avec 1% de sérum bovin foetal inactivé par la chaleur, et porté à une valeur de concentration de 100 µM dans la chloroquine (Luthman, H & Magnusson, G. (1983) High efficiency polyoma DNA transfection of chloroquine treated cells. Nucleic Acids Res., **11**, 1295-1308), est ajoutée dans le puits. Après 4 heures d'incubation à 37°C, le surnageant est retiré et 2 ml de milieu DMEM frais complet sont ajoutés et les cellules sont ensuite incubées pendant 48 h à 37°C.

Test à la luciférase

L'expression génétique de la luciférase est mesurée par luminescence selon la méthode décrite par De Wet et al., 1987, (De Wet, J.R., Wood, K.V., De Luca, M., Helinski, D.R. & Subramani, S. (1987) Firefly luciferase gene: structure and expression in mammalian cells. Mol., Cell., Biol., **7,** 725-737). Le milieu de culture est retiré, et les cellules sont récoltées après incubation à 37°C dans du PBS contenant 0.2 mg/ml de EDTA et 2.5 µg/ml de trypsine (GIBCO) et lavées 3 fois avec du PBS. Le tampon d'homogénéisation (200 µl; 8 mM $MgCl_2$, 1 mM de dithiothréitol, 1 mM d'EDTA, 1% de Triton X 100 et 15% de glycérol, 25 mM de tampon Tris-phosphate pH 7.8), est ajouté au culot. La suspension est agitée par un vortex et conservée pendant 10 min à 20°C. La solution est envoyée en bas du tube par centrifugation (5 min 800 g). L'ATP (95 µl d'une solution 2 mM dans le tampon d'homogénéisation sans Triton X 100) est ajouté à 60 µl du surnageant et la luminescence est enregistrée pendant 4 secondes en utilisant un luminomètre (Lumat LB 9501, Berthold, Wildbach, Allemagne) sur addition automatique de 150 µl de luciférine 167 µM dans l'eau; les mesures sont faites en triple.

Dosage de protéine

Un dosage de protéine est effectué pour chaque échantillon en utilisant la méthode colorimétrique d'acide bicinchoninique (BCA) (Smith, P.K., Krohn, R.I., Hermanson, G.T., Mallia, A.K., Gartner, F.H., Provenzano, M.D., Fujimoto, E.K., Goeke, N.M., Olson, B.J. & Klenk D.C. (1985) Measurement of protein using bicinchoninic acid. Anal., Biochem., **150**, 76-85), adaptée par Hill et Straka (1988) (Hill, H.D. & Straka, J.G. (1988) Protein détermination using bicinchoninic acid in the presence of sulfhydryl reagents. Anal., Biochem., **170**, 203-208) pour se débarrasser de la présence de DTT dans le tampon d'homogénéisation. La mesure de l'expression de luciférase est exprimée comme unité de lumière relative (RLU) par mg de BSA (albumine libre d'acide gras purifiée et cristallisée, A7511, Sigma), 1 mg BSA correspondant à 1,2 x $10^6$ cellules HepG2.

**Résultats**

Polylysine gluconoylée

La poly-L-lysine (masse moléculaire moyenne de la forme salifiée 40.000; degré de polymérisation moyen 190) a été partiellement (de 15 à 70%) acylée sur les fonctions $\varepsilon$-$NH_2$ de la lysine en utilisant la D-gluconolactone, un agent également hydrosolubilisant. Le but est de réduire le nombre de charges positives sur la polylysine et par conséquent de réduire les interactions électrostatiques entre le polymère et un plasmide.

En présence de polylysine, l'ADN se condense fortement par un mécanisme coopératif entre les charges positives de la polylysine et les charges négatives de l'ADN.

La formation des complexes entre un plasmide de 5 kb tel que le plasmide pSV2Luc contenant le gène de la luciférase avec des polylysines substituées par des quantités croissantes de résidus gluconoyles est analysée par électrophorèse en gel d'aragose et les complexes ADN/polymère optimaux correspondent à ceux pour lesquels l'ADN ne migre pas en électrophorèse par suite de la totale condensation de l'ADN, sont ainsi aisément déterminés (Figure 1 ter). Au-delà de 80% de substitution, la polylysine gluconoylée ne forme plus de complexe avec l'ADN.

La quantité de polylysine gluconoylée complexée par molécule d'ADN en fonction du rapport (P/ADN) entre la polylysine gluconoylée et l'ADN a été déterminé (Figure 2 ter). Lorsque celui-ci est compris entre 100 et 200, la quantité de polylysine gluconoylée libre est pratiquement nulle, en d'autre terme, toute la polylysine est complexée avec l'ADN. Par conséquent, aucune purification de ces complexes n'est nécessaire et une toxicité induite par de la polylysine libre sera écartée. En outre, les complexes réalisés avec ces rapports sont des complexes qui permettent une très bonne efficacité de transfection.

Comme le montrent les résultats des figures 2 et 2 bis, l'expression de la luciférase par les cellules HepG2 (hépatocarcinome humain) augmente avec la quantité de résidus gluconoyles fixée sur la polylysine et atteint un maximum (environ 300 fois plus qu'avec le plasmide seul) (ligne de base) lorsque la polylysine est substituée par 45 à 70% (88 à 132 résidus) gluconoyles lorsque le dosage est colorimétrique (figure 2) ou 35 à 70% (70 à 132 résidus) gluconoyles lorsque le dosage est effectué par RMN (figure 2 bis). Les polylysines substituées par peu ou trop de résidus gluconoyles sont pas ou peu efficaces. A titre de comparaison, l'expression de la luciférase obtenue dans ces mêmes cellules et dans les mêmes conditions en utilisant la polylysine lactosylée dans les conditions optimales, permet de conclure que la transfection obtenue avec la polylysine gluconoylée (substituée à 58%) est comparable à celle obtenue avec la polylysine lactosylée dans les conditions optimales.

Dans les complexes ADN/polymères pour lesquels l'ADN ne migre pas en électrophorèse par suite de la totale condensation de l'ADN, le rapport molaire entre la quantité de polylysine gluconoylée et l'ADN augmente et le rapport entre le nombre de charges positives et le nombre de charges négatives augmente linéairement lorsque le nombre de résidus gluconoyles fixés sur la polylysine augmente (Figure 2 quater). Les complexes présentent un excès de charges négatives lorsque la polylysine est faiblement substituée et sont globalement neutres lorsque la polylysine est substituée à moitié. Dans les conditions maximales de transfection (35 à 45% de substitution), les complexes sont proches de la neutralité. Ceci indique que le caractère polycationique de la polylysine n'est pas en cause dans l'efficacité de la transfection. Le caractère neutre ou anionique des complexes ADN/polylysine gluconoylée limitant les interactions non spécifiques avec les cellules est un atout supplémentaire pour leur utilisation *in vivo*.

La polylysine substituée par 45 à 70% (88 à 132 résidus) de gluconoyles permet de préparer des complexes ADN/polymère fortement concentrés, jusqu'à 100 µg/ml d'ADN, alors qu'avec la polylysine lactosylée, il est seulement possible de préparer des complexes 10 fois moins concentrés. Dans la figure 7, est montrée, de façon comparative, la solubilité en fonction de la concentration en ADN, des complexes ADN/polylysine ADN/polylysine gluconoylée et ADN/polylysine lactosylée. Cette étude est réalisée en mesurant la turbidité (mesure de l'absorbance à 610 nm) des différentes solutions. Les complexes ADN/polylysine et ADN/polylysine gluconoylée restent solubles jusqu'à plus de 100 µg/ml, alors qu'avec la polylysine lactosylée, les complexes précipitent à partir de 20 µg/ml. En présence de polymère, l'ADN se condense fortement par suite d'un phénomène coopératif entre les charges positives et négatives des deux polymères. Dans le cas de la polylysine gluconoylée, environ 60% des charges positives étant substituées, la coopérativité des interactions électrostatiques entre l'ADN et le polymère est diminuée, ce qui facilite une dissociation des complexes ADN/polymère et en particulier un relargage de l'ADN dans la cellule et permet une bonne expression du gène. Une étude de la modification de la coopérativité des interactions électrostatiques entre l'ADN et la polylysine gluconoylée est réalisée en fonction de la force ionique et de la nature des contre ions.

Polylysine gluconoylée munie d'un signal de reconnaissance.

La polylysine gluconoylée peut être substituée par un ligand de petite masse moléculaire tel que le lactose ayant une affinité moyenne pour un récepteur membranaire ou la biotine ayant une forte affinité pour un récepteur membranaire.

Polylysine gluconoylée et lactosylée.

Lorsque la polylysine est substituée par 30 résidus de lactose, les cellules HepG2 sont très faiblement transfectées comparativement aux résultats obtenus avec la polylysine substituée par 60 résidus de lactose (Tableau I). La polylysine substituée par 30 lactoses possède encore beaucoup de charges positives, interagit fortement avec l'ADN et ne permet pas une dissociation rapide de l'ADN du complexe. Lorsque la polylysine contenant 30 résidus de lactose est en plus substituée par 30 résidus gluconoyles, l'expression de la luciférase est élevée et comparable à celle obtenue avec la polylysine substituée par 60 résidus de lactose. L'addition de résidus gluconoyle permet de réduire les interactions électrostatiques entre l'ADN et la polylysine, facilitant une dissociation rapide de l'ADN dans la cellule.

De plus, comme le montre la figure 7 bis, la substitution de la polylysine à la fois par des résidus lactose et gluconoyle, augmente la solubilité des complexes par rapport à la polylysine uniquement lactosylée et permet de faire des préparations de complexes concentrés jusqu'à 100 µg/ml utilisables *in vivo*.

Polylysine gluconoylée et biotinylée.

Lorsque la polylysine gluconoylée (contenant 60 résidus gluconoyles) est substituée par un très petit nombre (2.5) de résidus de biotine, les complexes ADN/polylysine biotinylée ont une très forte affinité ($10^{15}$ l/ mole) pour la streptavidine. Si de plus la streptavidine est munie d'un signal de reconnaissance reconnu par un récepteur membranaire spécifique d'un type cellulaire qui induit l'endocytose des complexes, l'ADN acquière alors une spécificité cellulaire. Ceci est montré dans le Tableau II: le plasmide complexé par l'intermédiaire de la polylysine gluconoylée et biotinylée à de la streptavidine munie d'un signal de reconnaissance tel que la sérum albumine lactosylée reconnue et endocytée via la lectine membranaire des cellules HepG2, l'expression de la luciférase est beaucoup plus importante

que lorsque la sérum albumine est dépourvue de lactose ou bien que la streptavidine est dépourvue d'un signal de reconnaissance.

Transfert de gènes par des complexes ADN/cytokine.

Le transfert de gènes dans des lignées myéloïdes par des complexes avec la polylysine liée au facteur de croissance des cellules souches (Stem Cell Factor, SCF) est représenté sur la figure 8. Un plasmide contenant le gène de la luciférase a été complexé par l'intermédiaire de la polylysine gluconoylée et biotinylée au SCF biotinylé en utilisant de la streptavidine. Les complexes ainsi formés sont efficaces pour transfecter les cellules HEL qui expriment de nombreux récepteurs (c-kit) pour le SCF.

Polylysine gluconoylée et transfert de gènes dans divers types cellulaires.

La polylysine substituée par 45 à 70% de gluconoyles permet de transfecter avec une bonne efficacité, des cellules adhérentes telles que des macrophages humains, un hépatocarcinome humain, des cellules endothéliales de rat mais également des cellules non adhérentes telles qu'un lymphome T humain et des cellules leucémiques de la lignée érythroïde (Figures 4 et 5).

Conclusions

Le caractère inventif de la polylysine gluconoylée repose:

- sur l'utilisation d'une polylysine partiellement substituée pour réduire les interactions électrostatiques entre l'ADN et le polycation afin de faciliter un relargage intracellulaire de l'ADN après internalisation dans la cellule par un phénomène d'endocytose non spécifique (la présence de récepteurs membranaires capables de reconnaître spécifiquement les résidus gluconoyles, n'est pas connue);
- les résidus gluconoyles agissent également comme hydrosolubilisant et permettent de préparer des complexes ADN/polylysine très concentrés et donc une meilleure utilisation *in vivo,* ce qui n'est pas le cas avec la polylysine lactosylée ou bien la polylysine substituée par des protéines comme la transferrine ou l'asialooromucoïde;
- la polylysine gluconoylée peut être utilisée pour transfecter divers types cellulaires et en particulier des cellules non adhérentes;
- la polylysine gluconoylée peut être utilisée comme polymère de base pour conférer une spécificité cellulaire à l'ADN par addition de quelques molécules de ligands de faible masse moléculaire reconnues par des récepteurs membranaires spécifiques. L'avantage de la polylysine gluconoylée par rapport à la polylysine non gluconoylée réside dans le fait que celle-ci est déjà optimisée pour permettre la formation de complexes ADN/polymère-ligand facilement dissociables dans la cellule ce qui réduit le nombre de molécules de ligands à fixer sur la polylysine. En effet, on a déjà montré que l'efficacité de transfection dépend du nombre de molécules de d'oses fixées sur la polylysine non gluconoylée (60 résidus lactose ou 80 résidus galactose sont nécessaires pour une transfection optimale dans les cellules possédant un récepteur reconnu pour le lactose ou le galactose).

## Revendications

1. Complexe entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres des susdits motifs et étant tel que:

   - les fonctions $NH_3^+$ libres des susdits motifs sont substituées dans un rapport d'au moins 10%, avantageusement de 45% à 70%, notamment de 60%, ce rapport étant déterminé par méthode colorimétrique (Fields R. (1971) Biochem. J., 124: 581-590), ou avantageusement de 35% à 70%, notamment de 40%, ce rapport étant déterminé par résonance magnétique nucléaire, par des résidus non chargés entraînant une diminution des charges positives par rapport au même conjugué polymérique non substitué, facilitant le relargage de l'acide nucléique par la dissociation du complexe,
   - les susdits résidus possédant en outre les propriétés suivantes:

     → ils comportent au moins un groupe hydroxyle,
     → ils ne correspondent à aucun signal de reconnaissance reconnu par un récepteur membranaire cellulaire,

- les fonctions $NH_3^+$ libres des susdits motifs et/ou les groupes hydroxyles des susdits résidus pouvant être également substituées par au moins une molécule qui constitue un signal de reconnaissance reconnu par un récepteur membranaire cellulaire, sous réserve que le conjugué polymérique contienne au moins 30% de fonctions $NH_3^+$ libres.

**2.** Complexe selon la revendication 1, entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres des susdits motifs et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives par rapport au même conjugué polymérique non substitué, facilitant le relargage de l'acide nucléique par la dissociation du complexe,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les fonctions $NH_3^+$ libres des susdits motifs des susdits résidus pouvant être également substituées par au moins une molécule qui constitue un signal de reconnaissance reconnu par un récepteur membranaire cellulaire, sous réserve que le conjugué polymérique contienne au moins 30% de fonctions $NH_3^+$ libres.

**3.** Complexe selon la revendication 1, entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres des susdits motifs et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives, par rapport au même conjugué polymérique non substitué, facilitant le relargage de l'acide nucléique par la dissociation du complexe,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les groupes hydroxyles des susdits résidus pouvant être substituées par au moins une molécule qui constitue un signal de reconnaissance reconnu par un récepteur membranaire cellulaire, sous réserve que le conjugué polymérique contienne au moins 30% de fonctions $NH_3^+$ libres.

**4.** Complexe selon l'une des revendications 1 ou 2, entre au moins un acide nucléique chargé négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres, notamment des résidus de lysine et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives par rapport au même conjugué polymérique non substitué, facilitant ainsi la dissociation du complexe et le relargage de l'acide nucléique,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les fonctions $NH_3^+$ libres des susdits motifs étant également substituées de 0 à 40% par une molécule qui constitue un signal de reconnaissance reconnu par un récepteur membranaire cellulaire, ce signal de reconnaissance ayant un poids moléculaire inférieur à 5000, sous réserve que le conjugué polymérique contienne

au moins 30% de fonctions $NH_3^+$ libres, et lorsqu'il est présent, ce signal de reconnaissance peut l'être à raison d'une molécule pour environ 10 000 motifs du conjugué polymérique ou d'environ 60 molécules pour environ 10 000 motifs du conjugué polymérique.

**5.** Complexe selon l'une des revendications 1, 2 ou 4, entre au moins un acide nucléique chargé-négativement et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres, notamment des résidus de lysine et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges positives par rapport au même conjugué polymérique non substitué, facilitant ainsi la dissociation du complexe et le relargage de l'acide nucléique,
- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les fonctions $NH_3^+$ libres des susdits motifs pouvant être également substituées par une molécule qui constitue un signal de reconnaissance reconnu par un récepteur membranaire cellulaire, ce signal de reconnaissance ayant un poids moléculaire inférieur à 5000,

et lorsqu'il est présent, ce signal de reconnaissance peut l'être à raison d'une molécule pour environ 10 000 motifs du conjugué polymérique ou d'environ 60 molécules pour environ 10 000 motifs du conjugué polymérique.

**6.** Complexe selon l'une des revendications 1, 2, 4 ou 5, dans lequel le polymère contient un groupement polymérique de formule (I) suivante:

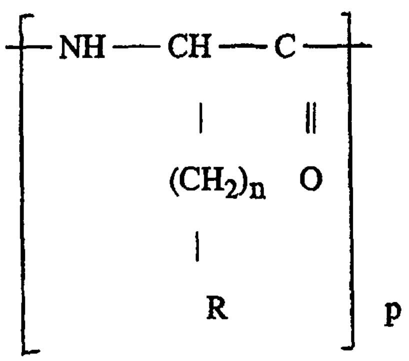

(I)

dans laquelle:

. p est un nombre entier variant de 2 à 500 de préférence de 150 à 200,
. n est un nombre entier variant de 1 à 5 et vaut de préférence 4,
. ce groupement polymérique contenant p radicaux R parmi lesquels:

  * 10% à 70% du nombre des radicaux R représentent un groupe $NH-CO-(CHOH)_m-R_1$, notamment un reste dihydroxypropionoyle, érythrononoyle, thréonoyle, ribonoyle, arabinoyle, xylonoyle, lyxonoyle, gluconoyle, galactonoyle, mannonoyle, glycoheptonoyle, glycooctonoyle,

. m est un nombre entier de 2 à 15, de préférence de 2 à 7,
. $R_1$ représente H ou un radical alcoyle de 1 à 15 atomes de carbone, notamment $CH_3$,

  * le reste des radicaux, c'est-à-dire 30% à 90% du nombre des radicaux R représentent $NH_3^+$,
  * R pouvant en outre être constitué de 0 à 25% par une molécule qui constitue un signal de reconnaissance reconnu par un récepteur membranaire cellulaire, sous réserve que le conjugué polymérique contienne

au moins 30% de fonctions $NH_3^+$ libres, et notamment à raison de 0,5 à 5, avantageusement de 1 molécule pour environ 10 000 motifs,

ou à raison de 10 à 100, avantageusement de 60 molécules pour environ 10 000 motifs.

**7.** Complexe selon la revendication 6, dans lequel le polymère comprend un groupement polymérique de formule (II):

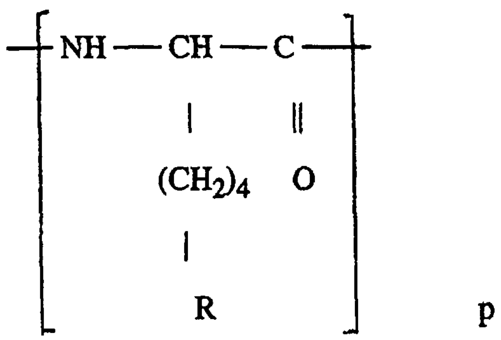

(II)

dans laquelle:

. p a les significations indiquées à la revendication 6,

* 10% à 70% du nombre des résidus R représentent un groupe NH-CO-$(CHOH)_m$-$R_1$, m et $R_1$ ayant les significations indiquées à la revendication 6,
* le reste des radicaux, c'est-à-dire 30% à 90% du nombre des radicaux R représentent $NH_3^+$, et de 0 à 25% des radicaux R pouvant être subtitués par une molécule qui constitue un signal de reconnaissance reconnu par un récepteur membranaire cellulaire, sous réserve que le conjugué polymérique au moins 30% de fonctions $NH_3^+$ libres.

**8.** Complexe selon la revendication 6, dans lequel le polymère comprend un groupement polymérique de formule (II):

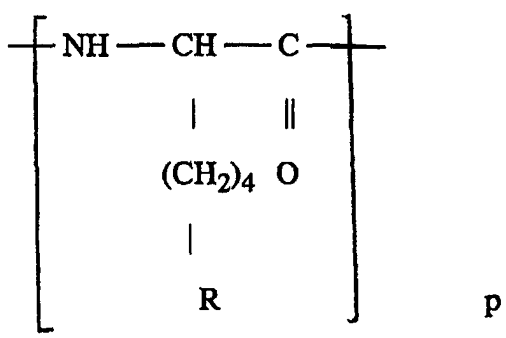

(II)

dans laquelle:

. p a les significations indiquées à la revendication 6,

* 10% à 70% du nombre des résidus R représentent un groupe NH-CO-$(CHOH)_m$-$R_1$, m et $R_1$ ayant les significations indiquées à la revendication 6,
* le reste des radicaux, c'est-à-dire 30% à 90% du nombre des radicaux R représentent $NH_3^+$.

**9.** Complexe selon la revendication 6, dans lequel le polymère comprend un groupement polymérique de formule (II):

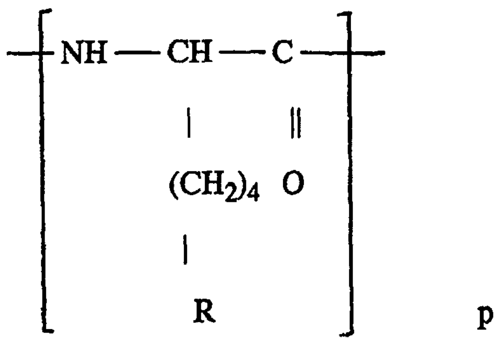

(II)

dans laquelle:

p a la signification indiquée à la revendication 6,

. ce groupement polymérique contenant p radicaux R parmi lesquels:

* 10% à 70% du nombre des radicaux R représentent un groupe NH-CO-$(CHOH)_m$-$R_1$, m et $R_1$ ayant les significations indiquées à la revendication 6,
* le reste des radicaux, c'est-à-dire 30% à 90% des radicaux R représentent d'une part $NH_3^+$ et d'autre part une molécule qui constitue un signal de reconnaissance reconnu par un récepteur membranaire cellulaire à raison de 0,5 à 5, avantageusement de 1 molécule pour 10 000 motifs.

**10.** Complexe selon la revendication 6, dans lequel le polymère comprend un groupement polymérique de formule (II):

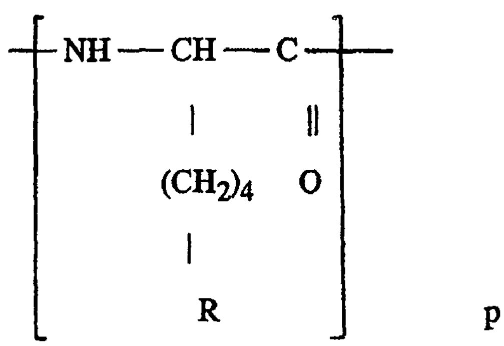

(II)

dans laquelle:

. p a la signification indiquée à la revendication 6,
. ce groupement polymérique contenant p radicaux R parmi lesquels:

* 10% à 70% du nombre des radicaux R représentent un groupe NH-CO-$(CHOH)_m$-$R_1$, m et $R_1$ ayant les significations indiquées à la revendication 6,
* le reste des radicaux, c'est-à-dire 30% à 90% de ces radicaux R représentent d'une part $NH_3^+$ et d'autre part une molécule qui constitue un signal de reconnaissance reconnu par un récepteur membranaire cellulaire à raison de 10 à 100, avantageusement de 60 molécules pour environ 10 000 motifs.

**11.** Complexe selon l'une des revendications 1 à 10, **caractérisé en ce que** le signal de reconnaissance est choisi parmi:

A) - des osides simples ou complexes reconnus par des lectines membranaires, et choisis parmi:

**a. Asialo-oligoside de type triantennaire lactosamine: récepteur d'asialoglycoprotéine**

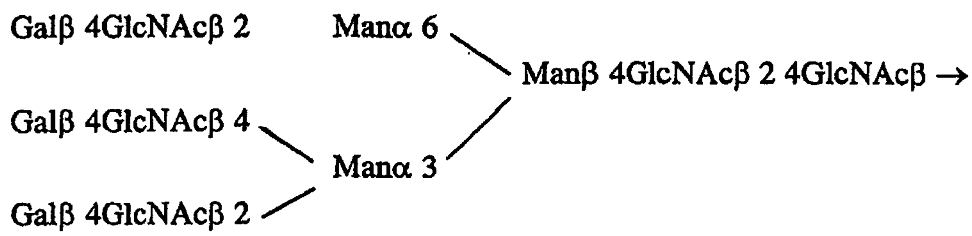

b. Asialo oligoside de type lactosamine tetraantennaire: récepteur d'asialoglycoprotéine

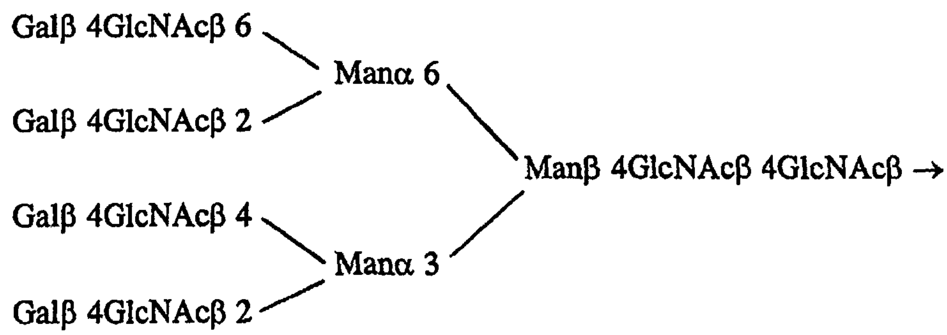

c. Lewis x: LECAM 2/3

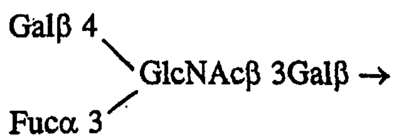

d. Lewis x sialyl: LECAM 3/2

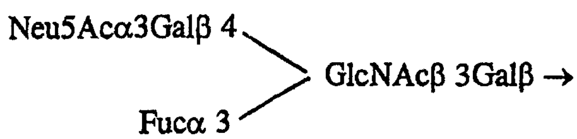

e. Dérivé de Lewis x sulfaté (HNK1): LECAM 1

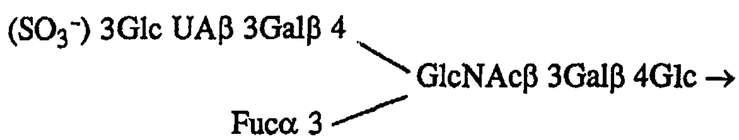

f. Oligomannoside: récepteur du mannose

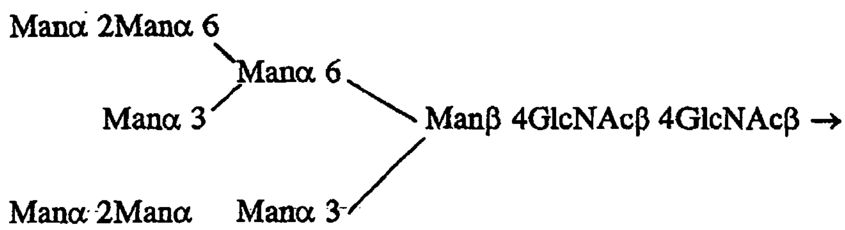

**g. Oligomannoside phosphorylé: récepteur de mannose 6 phosphate**

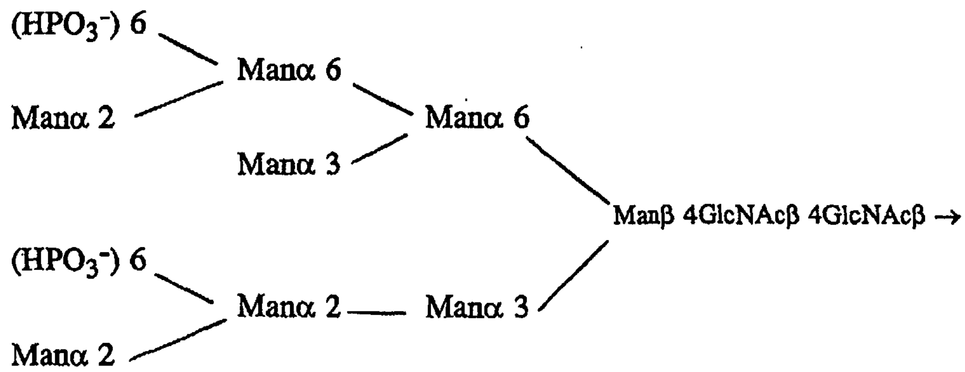

**h. Oligosaccharide de type lactosamine sulfaté: récepteur de GalNAc 4 sulfaté**

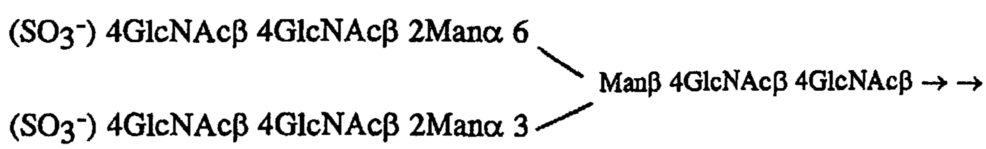

B) des peptides

a) peptides anti-inflammatoires ou certains de leurs fragments reconnus par des récepteurs de la paroi vasculaire, tels que

- polypeptide vasodilatateur intestinal (VIP)
  HSDAVFTDNYTRLRKQMAVKKYLNSILN-$NH_2$
- polypeptide atrial natriurétique (ANP)
  SLRRSSCFGGRMDRIGAQSGLGCNSFRY
- lipocortine
  HDMNKVLDL
- bradykinine
  RPPGFSPFR;

b) peptides ligands des intégrines, tels que les peptides contenant la séquence RGD tel que le récepteur de la fibronectine;
c) facteurs chimiotactiques, tels que les formyl peptides et antagonistes: FMLP, (N-formyl-Met-Leu-Phé);
d) hormones peptidiques tels que

$\alpha$-MSH: Ac-SYSMEHFRWGKPV-$NH_2$

C) Métabolites naturels tels que:

- la biotine,
- le tétrahydrofolate,

- l'acide folique,
- la carnitine.

**12.** Complexe selon l'une des revendications 1 à 11, **caractérisé en ce que** l'acide nucléique peut être choisi parmi:

a) des gènes marqueurs, tels que

- gènes codant pour la luciférase,
- gènes codant pour la β-galactosidase,
- gènes codant pour la chloramphénicol acétyl transférase,
- gènes conférant la résistance à un antibiotique, tel que l'hygromycine et la néomycine etc...;

b) des gènes à visée thérapeutique, tels que des gènes codant pour des

- récepteurs des lipoprotéines de faible densité, déficient dans les cas d'hypercholestérolémie (foie),
- facteurs de coagulation: facteurs VIII et IX,
- phénylalanine-hydroxylase (phénylcétonurie),
- adénosine désaminase (immunodéficience ADA),
- enzymes lysosomiques, tels que la β-glucosidase dans le cas de la maladie de Gaucher,
- dystrophine et minidistriphine (myophatie),
- tyrosine hydroxylase (Parkinson),
- facteurs de croissance des neurones (Alzheimer),
- CFTR cystic-fibrosis transmembrane conductance regulator (mucoviscidose),
- alpha1-antitrypsine,
- cytokines (interleukines, TNF facteur de nécrose des tumeurs),
- thymidine kinase du virus Herpes simplex,
- NO synthase,
- récepteurs de l'angiotensine II,
- gènes suppresseurs de tumeurs, tels que le gène de la protéine p53,
- protéines du MHC, système majeur d'histocompatibilité, en particulier HLA-B7,
- cytosine désaminase,
- gènes codant pour des ARN sens et antisens,
- gènes codant pour des ribozymes,

c) des gènes à visée vaccinale

- gènes codant pour des antigènes viraux (vaccination), par exemple: gène codant pour la nucléoprotéine du virus de la grippe.

**13.** Complexe selon l'une des revendications 1 à 12, dans lequel:

- le polymère, notamment la polylysine présente un degré de polymérisation d'environ 100 à environ 500, de préférence 190,
- les fonctions $NH_3$+ libres des motifs lysine étant substituées dans un rapport de 60% par des groupements gluconoyle et éventuellement par une molécule constituant un signal de reconnaissance pour 10 000 résidus de lysine lorsque ladite molécule signal possède une affinité d'au moins $10^6$ l $mole^{-1}$ vis à vis du récepteur de la cellule que le complexe doit cibler ou éventuellement par 60 molécules de signal de reconnaissance pour 10 000 résidus de lysine lorsque ladite molécule signal possède une affinité inférieure d'au moins $10^4$ l $mole^{-1}$ vis à vis du susdit récepteur,
- l'acide nucléique présente un poids moléculaire d'environ $6.10^5$ à environ $25.10^6$, et
- le nombre moyen de paires de base de l'acide nucléique par molécule de motif de monomère, notamment la lysine est d'environ 0,9 à environ 1,1, de préférence d'environ 0,95 à environ 1,05.

**14.** Conjugué polymérique chargé positivement, contenant des motifs portant des fonctions $NH_3^+$ libres, notamment des résidus de lysine et étant tel que:

- les fonctions $NH_3^+$ libres des susdits motifs sont substituées dans un rapport d'au moins 10%, avantageusement de 45% à 70%, notamment de 60%, par des résidus non chargés entraînant une diminution des charges

positives par rapport au même conjugué polymérique non substitué, facilitant le relargage d'un acide nucléique par la dissociation d'un complexe entre un acide nucléique chargé négativement et ledit conjugué polymérique,

- les susdits résidus possédant en outre les propriétés suivantes:

  → ils comportent au moins un groupe hydroxyle,
  → ils ne correspondent à aucun signal de reconnaissance reconnu par un récepteur membranaire cellulaire,

- les fonctions $NH_3^+$ libres des susdits motifs et/ou les groupes hydroxyles des susdits résidus pouvant être également substituées par au moins une molécule qui constitue un signal de reconnaissance reconnu par un récepteur membranaire-cellulaire, sous réserve que le conjugué polymérique contienne au moins 30% de fonctions $NH_3^+$ libres.

**15.** Conjugué polymérique selon la revendication 14, et tel que défini selon l'une des revendications 2 à 5, ou contenant un groupement polymérique de formule selon l'une des revendications 6 à 10.

**16.** Utilisation d'un complexe selon l'une des revendications 1 à 13, ou d'un conjugué selon l'une des revendications 14 ou 15, pour la transfection *in vitro* ou *ex vivo* de cellules à l'aide d'un gène, notamment ceux définis à la revendication 12.

**17.** Utilisation d'un complexe ou d'un conjugué selon la revendication 16, **caractérisé en ce que** les cellules sont choisies parmi:

- des cellules souches hématopoiétiques;
- cellules du foie;
- cellules des muscles squelettiques;
- cellules de la peau:

  . fibroblastes,
  . kératinocytes,
  . cellules dendritiques,
  . mélanocytes.

- cellules des parois vasculaires;

  . endothéliales;
  . musculaires lisses;

- cellules épithéliales des voies aériennes;
- cellules du système nerveux central;
- cellules cancéreuses;
- cellules du système immunitaire, telles que des lymphocytes, des macrophages, des cellules NK etc...

**18.** Méthode de transfection *in vitro* ou *ex vivo*, **caractérisée en ce que** l'on met en présence un complexe, selon l'une quelconque des revendications 1 à 13, dans un milieu contenant des cellules à transfecter, dans des conditions telles qu'il y a:

- passage du complexe à partir du milieu dans le cytoplasme des cellules,
- relargage de l'acide nucléique impliqué dans le susdit complexe dans le cytosol des cellules,
- transcription et expression de l'acide nucléique dans les cellules transfectées.

**19.** Composition pharmaceutique, **caractérisée en ce qu'**elle comprend à titre de substance active, l'un au moins des complexes selon l'une quelconque des revendications 1 à 13, en association avec un véhicule pharmaceutiquement acceptable.

**20.** Utilisation d'un complexe selon l'une des revendications 1 à 13, ou d'un conjugué selon l'une des revendications 14 ou 15, pour la préparation d'un médicament destiné par exemple au traitement de déficience métabolique congénitale ou acquise, ou au traitement de tumeurs, ou pour la préparation d'un vaccin, par exemple vaccin contre la grippe.

**21.** Trousse ou kit comprenant:

- un conjugué polymérique selon l'une des revendications 14 ou 15, tel que la polylysine, substituée par un résidu entraînant une diminution des charges des groupes $NH_3^+$ libres, ce conjugué polymérique étant apte à comporter éventuellement un signal de reconnaissance reconnu par un récepteur membranaire cellulaire, lequel est préalablement fixé ou non sur le susdit conjugué polymérique, ledit signal de reconnaissance étant fonction de la cellule à cibler,
- un plasmide contenant au moins un gène à transférer, et le système de régulation du susdit gène,
- des réactifs permettant la fixation éventuelle du signal de reconnaissance sur le susdit conjugué polymérique,
- des réactifs permettant la formation d'un complexe selon l'une des revendications 1 à 13, entre le conjugué polymérique et le plasmide,
- des réactifs permettant la transfection de la cellule par le susdit complexe.

## Patentansprüche

**1.** Komplex zwischen wenigstens einer negativ geladenen Nukleinsäure und wenigstens einem positiv geladenen konjugierten Polymer, wobei die Bindung zwischen der Nukleinsäure und dem konjugierten Polymer von elektrostatischer Natur ist, das konjugierte Polymer ein Polymer enthält, das aus Monomereinheiten gebildet ist, die freie $NH_3^+$ Funktionen der oben genannten Einheiten tragen und wobei:

- die freien $NH_3^+$ Funktionen der oben genannten Einheiten in einem Verhältnis von wenigstens 10%, vorzugsweise 45% bis 70%, insbesondere 60%, wobei das Verhältnis durch ein kolorimetrisches Verfahren ermittelt wird (Fields R. (1971) Biochem. J., 124: 581-590), oder vorzugsweise von 35% bis 70%, insbesondere 40%, wobei das Verhältnis durch Kernspinresonanz (NMR) ermittelt wird, wobei eine Verminderung der positiven Ladungen durch nicht geladene Reste im Verhältnis zu dem gleichen, nicht substituierten konjugierten Polymer verursacht wird, was die Wiederentladung der Nukleinsäure durch Dissoziation des Komplexes erleichtert,
- wobei die oben genannten Reste im übrigen die folgenden Eigenschaften besitzen:

  → sie wenigstens eine Hydroxylgruppe tragen,
  → sie keinem Erkennungssignal entsprechen, das durch einen Rezeptor der Zellmembran erkannt wird,

- die freien $NH_3^+$ Funktionen der oben genannten Einheiten und/oder die Hydroxylgruppen der oben genannten Reste auch durch wenigstens ein Molekül substituiert sein können, das ein Erkennungssignal zusammensetzt, das durch einen Rezeptor der Zellmembran erkannt wird, unter der Voraussetzung, dass das konjugierte Polymer wenigstens 30% freie $NH_3^+$ Funktionen enthält.

**2.** Komplex gemäß Anspruch 1, zwischen wenigstens einer negativ geladenen Nukleinsäure und wenigstens einem positiv geladenen konjugierten Polymer, wobei die Bindung zwischen der Nukleinsäure und dem konjugierten Polymer von elektrostatischer Natur ist, wobei das konjugierte Polymer ein Polymer enthält, das aus Monomereinheiten gebildet ist, die freie $NH_3^+$ Funktionen der vorgenannten Einheiten tragen und wobei:

- die freien $NH_3^+$ Funktionen der oben genannten Einheiten in einem Verhältnis von wenigstens 10%, vorzugsweise 45 bis 70%, insbesondere 60% durch nicht geladene Einheiten substituiert sind, wobei eine Verminderung der positiven Ladungen im Verhältnis zu dem gleichen substituierten, nicht konjugierten Polymer verursacht wird, was die Wiederentladung der Nukleinsäure durch die Dissoziation des Komplexes erleichtert,
- die oben genannten Reste im übrigen die folgenden Eigenschaften besitzen:

  → sie wenigstens eine Hydroxylgruppe tragen,
  → sie keinem Erkennungssignal entsprechen, das durch einen Rezeptor der Zellmembran erkannt wird,

- die freien $NH_3^+$ Funktionen der oben genannten Einheiten der oben genannten Reste auch durch wenigstens ein Molekül substituiert sein können, das ein Erkennungssignal zusammensetzt, das durch einen Rezeptor der Zellmembran erkannt wird, unter der Voraussetzung, dass das konjugierte Polymer wenigstens 30% freie $NH_3^+$ Funktionen enthält.

**3.** Komplex gemäß Anspruch 1, zwischen wenigstens einer negativ geladenen Nukleinsäure und wenigstens einem positiv geladenen konjugierten Polymer, wobei die Bindung zwischen der Nukleinsäure und dem konjugierten

Polymer von elektrostatischer Natur ist, wobei das konjugierte Polymer ein Polymer enthält, das aus Monomereinheiten gebildet ist, die freie $NH_3^+$ Funktionen der oben genannten Einheiten tragen und das wie folgt ist:

- die freien $NH_3^+$ Funktionen der oben genannten Einheiten in einem Verhältnis von wenigstens 10%, vorzugsweise 45% bis 70%, insbesondere 60% durch nicht geladene Reste substituiert sind, wobei eine Verminderung der positiven Ladungen im Verhältnis zu dem gleichen, nicht substituierten konjugierten Polymer verursacht wird, was die Wiederentladung der Nukleinsäure durch die Dissoziation des Komplexes erleichtert,
- wobei die oben genannten Reste im übrigen die folgenden Eigenschaften besitzen:

  → sie wenigstens eine Hydroxylgruppe tragen,
  → sie keinem Erkennungssignal entsprechen, das durch einen Rezeptor der Zellmembran erkannt wird,

- die Hydroxylgruppen der oben genannten Reste durch wenigstens ein Molekül substituiert sein können, das ein Erkennungssignal zusammensetzt, das durch einen Rezeptor der Zellmembran erkannt wird, unter der Voraussetzung, dass das konjugierte Polymer wenigstens 30% freie $NH_3^+$ Funktionen enthält.

**4.** Komplex gemäß Ansprüchen 1 oder 2, zwischen wenigstens einer negativ geladenen Nukleinsäure und wenigstens einem positiv geladenem konjugierten Polymer, wobei die Bindung zwischen der Nukleinsäure und dem konjugierten Polymer von elektrostatischer Natur ist, wobei das konjugierte Polymer ein Polymer enthält, das aus Monomereinheiten gebildet ist, die freie $NH_3^+$ Funktionen enthalten, insbesondere Lysin-Reste und wobei:

- die freien $NH_3^+$ Funktionen der oben genannten Einheiten in einem Verhältnis von wenigstens 10%, vorzugsweise 45% bis 70%, insbesondere 60%, durch nicht geladene Reste substituiert sind, wobei eine Verminderung der positiven Ladungen im Verhältnis zu dem gleichen, nicht substituierten konjugierten Polymer verursacht wird, was die Dissoziation des Komplexes und die Wiederentladung der Nukleinsäure erleichtert,
- wobei die oben genannten Reste im übrigen die folgenden Eigenschaften besitzen:

  → sie wenigstens eine Hydroxylgruppe tragen,
  → sie keinem Erkennungssignal entsprechen, das durch einen Rezeptor der Zellmembran erkannt wird,

- die freien $NH_3^+$ Funktionen der oben genannten Einheiten zu 0 bis 40% durch ein Molekül substituiert sein können, das ein Erkennungssignal zusammensetzt, das durch einen Rezeptor der Zellmembran erkannt wird, wobei das Erkennungssignal ein Molekulargewicht von kleiner als 5000 aufweist, unter der Voraussetzung, dass das konjugierte Polymer wenigstens 30% freie $NH_3^+$ Funktionen enthält und wenn dieses vorhanden ist, das Erkennungssignal in einer Menge von ungefähr 1 Molekül auf ungefähr 10000 Einheiten des konjugierten Polymers oder ungefähr 60 Moleküle auf ungefähr 10000 Einheiten des konjugierten Polymers sein kann.

**5.** Komplex gemäß einem der Ansprüche 1, 2- oder 4, zwischen wenigstens einer negativ geladenen Nukleinsäure und wenigstens einem positiv geladenen konjugierten Polymer, wobei die Bindung zwischen der Nukleinsäure und dem konjugierten Polymer von elektrostatischer Natur ist, wobei das konjugierte Polymer ein Polymer enthält, das aus Monomereinheiten gebildet ist, die freie $NH_3^+$ Funktionen enthalten, insbesondere aus Lysin-Einheiten und wobei:

- die freien $NH_3^+$ Funktionen der oben genannten Einheiten auch in einem Verhältnis von wenigstens 10%, vorzugsweise 45% bis 70%, insbesondere 60% durch nicht geladene Reste substituiert sind, wobei eine Verminderung der positiven Ladungen im Verhältnis zu dem gleichen, nicht substituierten konjugierten Polymer verursacht wird, was so die Dissoziation des Komplexes und die Wiederentladung der Nukleinsäure erleichtert,
- wobei die vorgenannten Reste im übrigen die folgenden Eigenschaften besitzen:

  → sie wenigstens eine Hydroxylgruppe tragen,
  → sie keinem Erkennungssignal entsprechen, das durch einen Rezeptor der Zellmembran erkannt wird,

- die freien $NH_3^+$ Funktionen der oben genannten Einheiten durch ein Molekül substituiert sein können, das ein Erkennungssignal zusammensetzt, das durch einen Rezeptor der Zellmembran erkannt wird, wobei das Erkennungssignal ein Molekulargewicht von kleiner als 5000 aufweist,

und, wenn es vorhanden ist, das Erkennungssignal in einer Menge von ungefähr 1 Molekül auf ungefähr 10000 Einheiten des konjugierten Polymers oder ungefähr 60 Molekülen auf ungefähr 10000 Einheiten des konjugierten Polymers sein kann.

6. Komplex gemäß einem der Ansprüche 1, 2, 4 oder 5, in welchem das Polymer eine Polymergruppe der folgenden Formel (I) enthält:

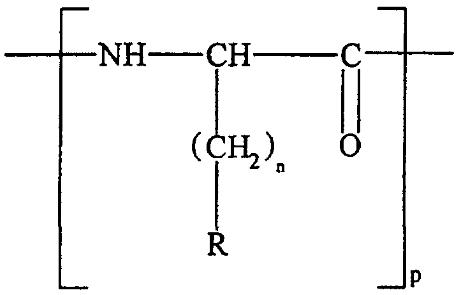

(I)

in welcher:

- p eine ganze Zahl ist, die von 2 bis 500, vorzugsweise von 150 bis 200 variiert,
- n eine ganze Zahl ist, die von 1 bis 5 variiert und vorzugsweise 4 ist,
- wobei die Polymergruppe p Reste R enthält, unter welchen

  * 10% bis 70% der Zahl der Reste R eine Gruppe NH-CO-$(CHOH)_m$-$R_1$ darstellen, insbesondere einen der folgenden Reste: Dihydroxypropionoyl, Erythrononoyl, Threonoyl, Ribonoyl, Arabinoyl, Xylonoyl, Lyxonoyl, Gluconoyl, Galactonoyl, Mannonoyl, Glycoheptonoyl, Glycooctonoyl.

- m eine ganze Zahl von 2 bis 15, vorzugsweise von 2 bis 7 ist,
- $R_1$ = H oder ein Alkylrest mit 1 bis 15 Kohlenstoffatomen, vorzugsweise $CH_3$, darstellt,

  * die restlichen Reste, das heißt 30% bis 90% der Zahl der Reste R, $NH_3^+$ darstellen,
  * R im übrigen von 0 bis 25% durch ein Molekül zusammengesetzt sein kann, das ein Erkennungssignal zusammensetzt, das durch einen Rezeptor der Zellmembran erkannt wird, unter der Voraussetzung, dass das konjugierte Polymer wenigstens 30% freie $NH_3^+$ Funktionen und insbesondere in einer Menge von 0,5 bis 5, vorzugsweise 1 Molekül auf ungefähr 10000 Einheiten, oder in einer Menge von 10 bis 100, vorzugsweise 60 Moleküle auf ungefähr 10000 Einheiten enthält.

7. Komplex gemäß Anspruch 6, in welchem das Polymer eine Polymergruppe der Formel (II) umfasst:

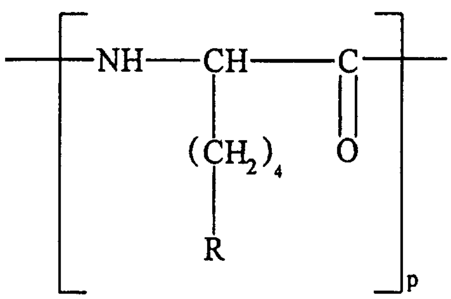

(II)

in welcher:

- p wie in Anspruch 6 definiert ist,

  * 10% bis 70% der Zahl der Reste R eine Gruppe NH-CO-$(CHOH)_m$-$R_1$ darstellen, wobei m und $R_1$ wie in Anspruch 6 definiert sind,
  * die restlichen Reste, das heißt 30% bis 90% der Zahl der Reste R $NH_3^+$ darstellen und 0 bis 25% der

Reste R durch ein Molekül substituiert sein können, das ein Erkennungssignal zusammensetzt, das durch einen Rezeptor der Zellmembran erkannt wird, unter der Voraussetzung, dass das konjugierte Polymer wenigstens 30% freie $NH_3^+$ Funktionen enthält.

**8.** Komplex gemäß Anspruch 6, in welchem das Polymer eine Polymergruppe der Formel (II) umfasst:

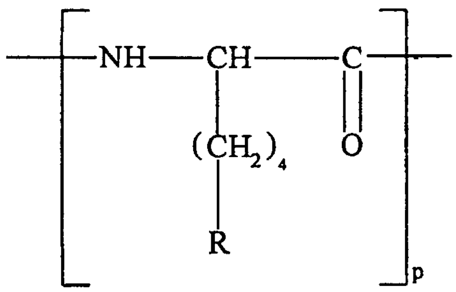

(II)

in welcher:

- p wie in Anspruch 6 definiert ist,
    - * 10% bis 70% der Zahl der Reste R eine Gruppe NH-CO-$(CHOH)_m$-$R_1$ darstellen, wobei m und $R_1$ wie in Anspruch 6 definiert sind,
    - * die restlichen Reste, das heißt 30% bis 90% der Zahl der Reste R $NH_3^+$ darstellen.

**9.** Komplex gemäß Anspruch 6, in welchem das Polymer eine Polymergruppe der Formel (II) umfasst:

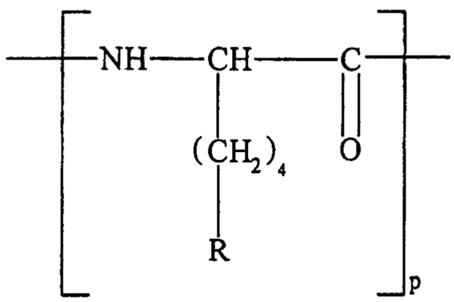

(II)

in welcher:

- p wie in Anspruch 6 definiert ist,
- wobei die Polymergruppe p Reste R enthält, unter welchen:
    - * 10% bis 70% der Zahl der Reste R eine Gruppe NH-CO-$(CHOH)_m$-$R_1$ darstellen, wobei m und $R_1$ wie in Anspruch 6 definiert sind,
    - * der Rest der Reste, das heißt 30% bis 90% der Zahl der Reste R zu einem Teil $NH_3^+$ und zum anderen Teil ein Molekül darstellen, das ein Erkennungssignal zusammensetzt, das durch einen Rezeptor der Zellmembran erkannt wird, in einer Menge von 0,5 bis 5, vorzugsweise von 1 Molekül auf 10000 Einheiten.

**10.** Komplex gemäß Anspruch 6, in welchem das Polymer eine Polymergruppe der Formel (II) umfasst:

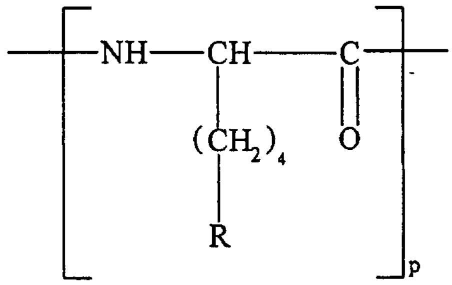

(II)

in welcher:

- p wie in Anspruch 6 definiert ist,
- wobei die Polymergruppe p Reste R enthält, unter welchen:

  * 10% bis 70% der Zahl der Reste R eine Gruppe NH-CO-$(CHOH)_m$-$R_1$ darstellen, wobei m und $R_1$ wie in Anspruch 6 definiert sind,
  * der Rest der Reste, das heißt 30% bis 90% der Reste R zu einem Teil $NH_3^+$ und zum anderen Teil ein Molekül darstellen, das ein Erkennungssignal zusammensetzt, das durch einen Rezeptor der Zellmembran erkannt wird, in einer Menge von 10 bis 100, vorzugsweise von 60 Molekülen auf ungefähr 10000 Einheiten.

**11.** Komplex gemäß einem der Ansprüche 1 bis 10 **dadurch gekennzeichnet, dass** das Erkennungssignal ausgewählt ist aus:

A) - den einfachen oder komplexen Osiden, die durch die Membranlectine erkannt werden, und aus folgenden ausgewählt sind:

a. Asialo-Oligoside vom Triantennärlactosamin-Typ: Rezeptor Asialoglycoprotein

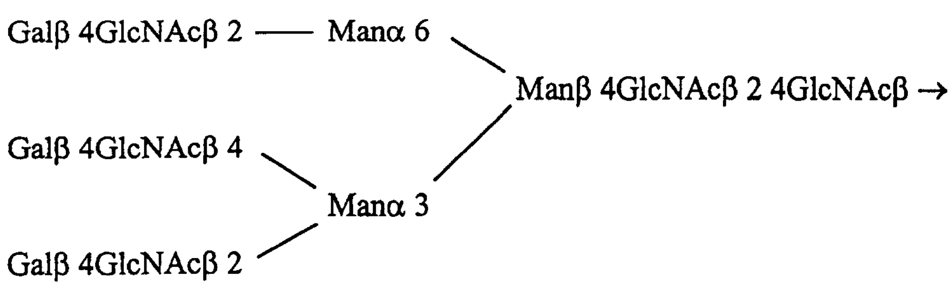

b. Asialo-oligosid vom Lactosamintetraantennär-Typ: Rezeptor Asialoglycoprotein

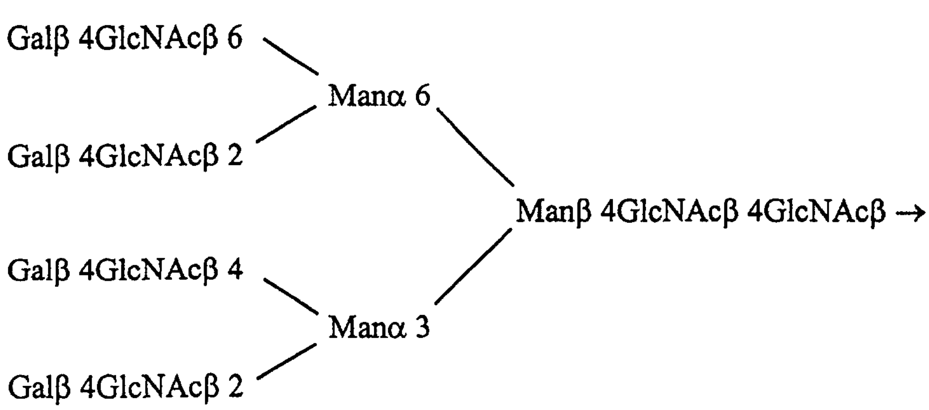

c. Lewis x: LECAM 2/3

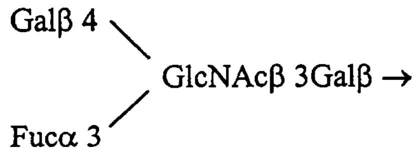

d. Lewis x sialyl: LECAM 3/2

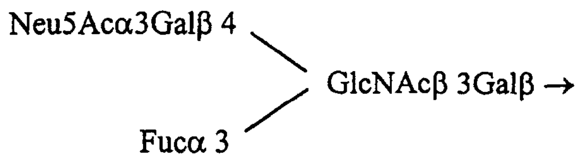

e. Sulfatiertes Derivat von Lewis x (HNK1): LECAM 1

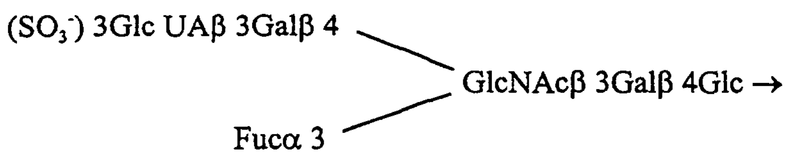

f. Oligomannosid: Rezeptor von Mannose

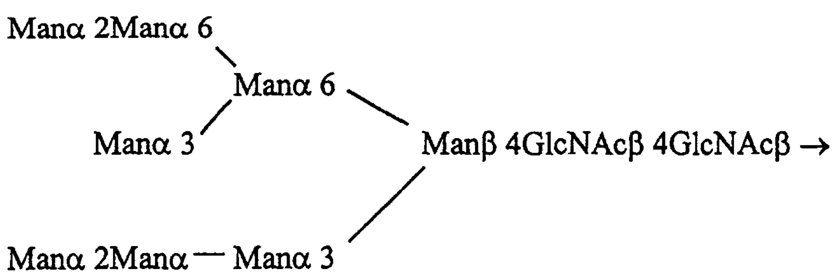

g. Phosphoryliertes Oligomannosid: Mannose Rezeptor 6 Phosphat

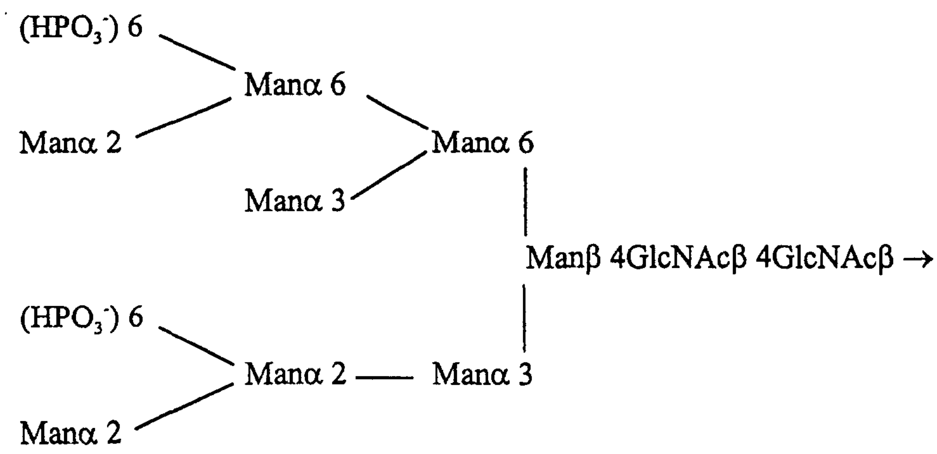

h. Oligosaccharid vom Sulfatierten Lactosamin: Rezeptor von GalNAc 4 Sulfat

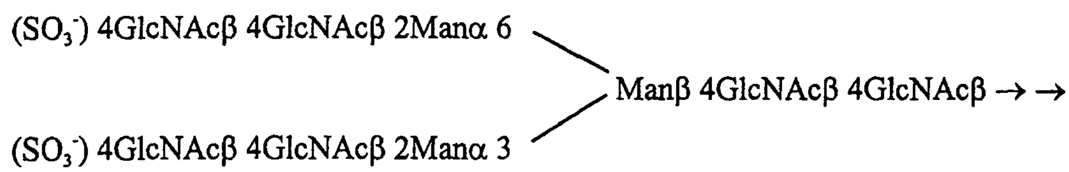

B) Peptide

a) Entzündungshemmende Peptide oder manche von deren Fragmenten, die durch Rezeptoren der Gefäßwand erkannt werden, wie etwa

- vasoaktives intestinales Polypeptid (VIP)
  HSDAVFTDNYTRLRKQMAVKKYLNSILN-$NH_2$
- atriales natriuretisches Polypeptid (ANP)
  SLRRSSCFGGRMDRIGAQSGLGCNSFRY
- Lipocortin
  HDMNKVLDL
- Bradykinin
  RPPGFSPFR;

b) Integrierende Peptidliganden, sowie die Peptide, die die Sequenz RGD enthalten, sowie der Rezeptor des Fibronectin;
c) Chemiotatische Faktoren, sowie die Formylpeptide und Antagonisten: FMLP, (N-Formyl-Met-Leu-Phe);
d) Peptidhormone, sowie

$\alpha$-MSH: Ac-SYSMEHFRWGKPV-$NH_2$,

C) natürliche Metaboliten, sowie:

- das Biotin,
- das Tetrahydrofolat,
- Folsäure,
- das Carnetin.

**12.** Komplex gemäß einem der Ansprüche 1 bis 11, **dadurch gekennzeichnet, dass** die Nukleinsäure ausgewählt sein kann unter:

a) den Markergenen, wie etwa

- Genen, die die Luciferase kodieren,
- Genen, die die β-Galactosidase kodieren,
- Genen, die die Chloramphenicol-acetyl-transferase kodieren,
- Gene, die die Resistenz gegen ein Antibiotikum übertragen, wie etwa das Hygromycin und das Neomycin etc.;

b) Gene mit therapeutischem Ziel, wie etwa Gene, die kodieren:

- Rezeptoren von Lipoproteinen von schwacher Dichte, die im Fall von Hypercholesterolemie (Leber) fehlen,
- Coagulationsfaktoren: Faktoren 8 und 9,
- Phenylalanin-Hydroxylase (Phenylketonurie),
- Adenosin-desaminase (Immunodefizienz ADA),
- Lysosomenzyme, wie etwa die β-Glucosidase im Fall der Gaucher-Krankheit,
- Dystrophin und Minidistriphin (Myopathie),
- Tyrosin Hydroxylase (Parkinson),
- Wachstumsfaktoren für Neuronen (Alzheimer),
- CFTR Transmembran Leitfähigkeitsregulator der cystischen Fibrose(Muskoviszidose),
- Alpha1-Antitrypsin,
- Cytokine (Interleukine, TNF Nekrosefaktor von Tumoren),
- Thymidin Kinase vom Virus Herpes simplex,
- NO Synthase,
- Rezeptoren des Angiotensins II,
- Supressorgene von Tumoren, wie etwa das Gen des Proteins 53,
- Proteine des MHC, Haupthistokompatibilitätssystem, insbesondere HLA-B7,
- Cytosin Desaminase,
- Gene, die für die RNA-sens und -antisens kodieren,
- Gene, die für die Ribozyme kodieren,

c) Gene zum Impfzweck

- Gene, die für virale Antigene kodieren (Impfung), z.B.: Gene, die für das Nukleoprotein des GrippeVirus kodieren.

**13.** Komplex gemäß einem der Ansprüche 1 bis 12, in welchem:

- das Polymer, insbesondere das Polylysin einen Polymerisationsgrad von zwischen 100 und ungefähr 500, vorzugsweise 190, darstellt,
- wobei die freien $NH_3^+$ Funktionen der Lysineinheiten in einem Verhältnis von 60% durch Glukonoyl-Gruppen und gegebenenfalls durch ein Molekül substituiert sind, das ein Erkennungssignal für 10000 Lysinreste zusammensetzt, bis das Molekülsignal eine Affinität von wenigstens $10^6$ l $Mol^{-1}$ gegenüber dem Rezeptor der Zelle besitzt, die der Komplex anzielen muss, oder gegebenenfalls durch 60 Moleküle des Erkennungssignals auf 10000 Lysinreste bis das Molekülsignal eine schwächere Affinität als wenigstens $10^4$ l $Mol^{-1}$ gegenüber dem Rezeptor besitzt,
- die Nukleinsäure ein Molekulargewicht von ungefähr $6.10^5$ bis ungefähr $25.10^6$ darstellt, und
- die durchschnittliche Zahl der Basenpaare der Nukleinsäure pro Molekül der Monomereinheit, insbesondere des Lysins, ungefähr 0,9 bis ungefähr 1,1, vorzugsweise ungefähr 0,95 bis ungefähr 1,05 beträgt.

**14.** Positiv geladenes kondensiertes Polymer, das freie $NH_3^+$ Funktionen tragende Einheiten enthält, insbesondere Lysinreste und wobei:

- die freien $NH_3^+$ Funktionen der vorgenannten Einheiten in einem Verhältnis von wenigstens 10%, vorzugsweise 45% bis 70%, insbesondere 60% durch nicht geladene Reste substituiert sind, wobei für eine Verminderung der positiven Ladungen im Verhältnis zum gleichen nicht substituierten konjugierten Polymer verursacht wird, was die Wiederentladung einer Nukleinsäure durch die Dissoziation eines Komplexes zwischen einer negativ geladenen Nukleinsäure und des konjugierten Polymers erleichtert,
- wobei die vorgenannten Reste im Übrigen die folgenden Eigenschaften besitzen:

→ sie wenigstens eine Hydroxylgruppe tragen,
→ sie keinem Erkennungssignal entsprechen, das durch einen Rezeptor der Zellmembran erkannt wird,

- die freien $NH_3^+$ Funktionen der vorgenannten Einheiten und/oder die Hydroxylgruppen der vorgenannten Reste auch durch wenigstens ein Molekül substituiert sein können, das ein Erkennungssignal zusammensetzt, das durch einen Rezeptor der Zellmembran erkannt wird, unter der Voraussetzung, dass das konjugierte Polymer wenigstens 30% freie $NH_3^+$ Funktionen enthält.

**15.** Konjugiertes Polymer gemäß Anspruch 14, und so wie in einem der Ansprüche 2 bis 5 definiert, oder, dass eine Polymergruppe der in einem der Ansprüche 6 bis 10 definierten Formel enthält.

**16.** Verwendung eines Komplexes gemäß einem der Ansprüche 1 bis 13, oder eines Konjugats gemäß einem der Ansprüche 14 oder 15, für die Transfektion *in vitro* oder *ex vivo* von Zellen mit Hilfe eines Gens, insbesondere denen, die in Anspruch 12 definiert sind.

**17.** Verwendung eines Komplexes oder eines Konjugats gemäß Anspruch 16, **dadurch gekennzeichnet, dass** die Zellen ausgewählt sind unter:

- Blutkeimzellen;
- Leberzellen;
- Skelettmuskelzellen;
- Hautzellen:

  . Fibroblasten,
  . Keratinocyten,
  . Dentritenzellen,
  . Melanocyten.

- Zellen von Gefäßwänden;

  . Endothelzellen;
  . glatte Muskeln;

- Epithelzellen der Luftwege;
- Zellen des zentralen Nervensystems;
- Krebszellen;
- Zellen des Immunsystems, wie etwa Lymphozyten, Makrophagen, NK-Zellen etc.

**18.** Transfektionsverfahren *in vitro* oder *ex vivo,* **dadurch gekennzeichnet, dass** man in Anwesenheit eines Komplexes gemäß einem der Ansprüche 1 bis 13 in einem Milieu, das Zellen zum Transfizieren enthält ausführt, unter den Bedingungen, dass es Folgendes gibt:

- einen Weg für den Komplex vom Milieu in das Cytoplasma der Zellen,
- Wiederentladung der Nukleinsäure, die in dem vorgenannten Komplex enthalten ist, in das Cytosol der Zellen,
- Transfektion und Expression der Nukleinsäure in die transfizierten Stellen.

**19.** Pharmazeutische Zusammensetzung, **dadurch gekennzeichnet, dass** sie als aktive Substanz wenigstens eines der Komplexe gemäß einem der Ansprüche 1 bis 13 umfasst, zusammen mit einem pharmazeutisch akzeptablen Träger.

**20.** Verwendung eines Komplexes gemäß einem der Ansprüche 1 bis 13, oder eines Konjugats gemäß einem der Ansprüche 14 oder 15 für die Herstellung eines Medikaments, das z.B. zur Behandlung von angeborener oder erworbener metabolischer Defizienz, oder zur Behandlung von Tumoren, oder zur Herstellung eines Impfstoffs, z.B. Impfstoff gegen Grippe, bestimmt ist.

**21.** Paket oder Kit, das Folgendes umfasst:

- ein konjugiertes Polymer gemäß einem der Ansprüche 14 oder 15, wie etwa das Polylysin, das durch einen

Rest substituiert ist, der zur Verminderung der Ladungen der freien $NH_3^+$ Gruppen führt, wobei das konjugierte Polymer gegebenenfalls zum Tragen eines Erkennungssignals geeignet ist, das durch einen Rezeptor der Zellmembran erkannt wird, welches im Vorhinein auf das vorstehende konjugierte Polymer fixiert ist oder nicht, wobei das Erkennungssignal eine Zellfunktion zum Zielen besitzt,

- ein Plasmid, das wenigstens ein Gen zum Übertragen, und das Regulationssystem des vorstehenden Gens enthält,
- Reagenzien, die die eventuelle Fixierung des Erkennungssignals auf dem vorstehenden konjugierten Polymer ermöglichen,
- Reagenzien, die die Bildung eines Komplexes gemäß einem der Ansprüche 1 bis 13 zwischen dem konjugierten Polymer und dem Plasmid ermöglichen,
- Reagenzien, die die Transfektion der Zelle durch den vorgenannten Komplex ermöglichen.

**Claims**

**1.** Complex between at least one negatively charged nucleic acid and at least one positively charged polymeric conjugate, the association between the nucleic acid and the polymeric conjugate being electrostatic in nature, the polymeric conjugate containing a polymer formed from monomeric components having free $NH_3^+$ functions of the aforementioned components and being as follows:

- the free $NH_3^+$ functions of the above mentioned components are substituted in a ratio of at least 10%, advantageously from 45% to 70%, particularly 60%, this ratio being determined by colorimetric method (Fields R. (1971) Biochem. J., 124: 581-590), or advantageously from 35% to 70%, particularly of 40%, this ratio being determined by nuclear magnetic resonance, by non-charged residues leading to a reduction of positive charges in comparison to the same non-substituted polymeric conjugate, facilitating the release of the nucleic acid by the dissociation of the complex,
- the above mentioned residues possess in addition the following properties:

  → they contain at least one hydroxyl group,
  → they do not correspond to a recognition signal recognized by a cellular membrane receptor,

- the free $NH_3^+$ functions from the aforementioned components and/or the hydroxyl groups from the aforementioned residues being also able to be substituted by at least one molecule which constitutes a recognition signal recognized by a cellular membrane receptor, under the condition that the polymeric conjugate contains at least 30% free $NH_3^+$ functions.

**2.** Complex according to claim 1, between at least one negatively charged nucleic acid and at least one positively charged polymeric conjugate, the association between the nucleic acid and the polymeric conjugate being electrostatic in nature, the polymeric conjugate containing a polymer formed from monomeric components having free $NH_3^+$ functions of the above mentioned components and being as follows:

- the free $NH_3^+$ functions of the above mentioned components are substituted in a ratio of at least 10%, advantageously from 45% to 70%, particularly 60%, by non-charged residues leading to a reduction of positive charges in comparison to the same non-substituted polymeric conjugate, facilitating the release of the nucleic acid by the dissociation of the complex,
- the above mentioned residues possess in addition the following properties:

  → they contain at least one hydroxyl group,
  → they do not correspond to a recognition signal recognized by a cellular membrane receptor,

- the free $NH_3^+$ functions from the aforementioned components of the above mentioned residues being also able to be substituted with at least one molecule which constitutes a recognition signal recognized by a cellular membrane receptor, under the condition that the polymeric conjugate contains at least 30% free $NH_3^+$ functions.

**3.** Complex according to claim 1, between at least one negatively charged nucleic acid and at least one positively charged polymeric conjugate, the association between the nucleic acid and the polymeric conjugate being electrostatic in nature, the polymeric conjugate containing a polymer formed from monomer components having free

$NH_3^+$ functions of the above mentioned components and being as follows:

- the free $NH_3^+$ functions of the above mentioned components are substituted in a ratio of at least 10%, advantageously from 45% to 70%, particularly 60%, by non-charged residues leading to a reduction of positive charges in comparison to the same non-substituted polymeric conjugate, facilitating the release of the nucleic acid by the dissociation of the complex,
- the above mentioned residues possess in addition the following properties:

  → they contain at least one hydroxyl group,
  → they do not correspond to a recognition signal recognized by a cellular membrane receptor,

- the hydroxyl groups of the above mentioned residues being able to be substituted by at least one molecule which constitutes a recognition signal recognized by a cellular membrane receptor, under the condition that the polymeric conjugate contains at least 30% free $NH_3^+$ functions.

**4.** Complex according to anyone of claims 1 or 2, between at least one negatively charged nucleic acid and at least one positively charged polymeric conjugate, the association between the nucleic acid and the polymeric conjugate being electrostatic in nature, the polymeric conjugate containing a polymer formed from monomer components having free $NH_3^+$ functions, particularly lysine residues, and being as follows:

- the free $NH_3^+$ functions of the above mentioned components are substituted in a ratio of at least 10%, advantageously from 45% to 70%, particularly 60%, by non-charged residues leading to a reduction of positive charges in comparison to the same non-substituted polymeric conjugate, thus facilitating the dissociation of the complex and the release of the nucleic acid,
- the above mentioned residues possess in addition the following properties:

  → they contain at least one hydroxyl group,
  → they do not correspond to a recognition signal recognized by a cellular membrane receptor,

- the free $NH_3^+$ functions of the above mentioned components being equally substituted from 0 to 40% by a molecule which constitutes a recognition signal recognized by a cellular membrane receptor, this recognition signal having a molecular weight less than 5,000, under the condition that the polymeric conjugate contains at least 30% free $NH_3^+$ functions,

and when it is present, this recognition signal can exist at the ratio of one molecule for approximately 10,000 components of the polymeric conjugate or approximately 60 molecules for approximately 10,000 components of the polymeric conjugate.

**5.** Complex according to anyone of claims 1, 2 or 4, between at least one negatively charged nucleic acid and at least one positively charged polymeric conjugate, the association between the nucleic acid and the polymeric conjugate being electrostatic in nature, the polymeric conjugate containing a polymer formed from monomer components having free $NH_3^+$ functions, particularly lysine residues, and being as follows:

- the free $NH_3^+$ functions of the above mentioned components are substituted in a ratio of at least 10%, advantageously from 45% to 70%, particularly 60%, by non-charged residues leading to a reduction of positive charges in comparison to the same non-substituted polymeric conjugate, thus facilitating the dissociation of the complex and the release of the nucleic acid,
- the above mentioned residues possess in addition the following properties:

  → they contain at least one hydroxyl group,
  → they do not correspond to a recognition signal recognized by a cellular membrane receptor,

- the free $NH_3^+$ functions of the above mentioned components being also able to be substituted by a molecule which constitutes a cellular membrane recognition signal, this recognition signal having a molecular weight less than 5,000,

and when it is present, this recognition signal can exist at the ratio of one molecule for approximately 10,000 components of the polymeric conjugate or approximately 60 molecules for approximately 10,000 components of

the polymeric conjugate.

6. Complex according to anyone of the claims 1, 2, 4 or 5, in which the polymer contains a polymeric group of the following formula (I):

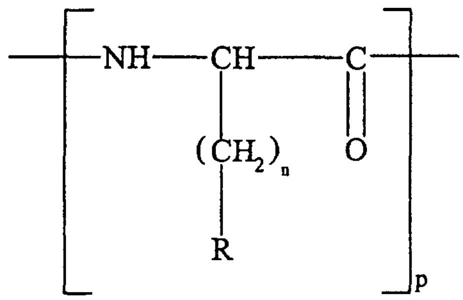

(I)

in which:

- p is an integer varying from 2 to 500, preferably from 150 to 200,
- n is an integer varying from 1 to 5 and being preferably 4,
- this polymeric group containing a number p of R residues among which:

  * 10% to 70% of the number of R residues represent a NH-CO-$(CHOH)_m$-$R_1$ group, particularly a dihydroxypropionoyle, erythrononoyle, threonoyle, ribonoyle, arabinoyle, xylonoyle, lyxonoyle, gluconoyle, galactonoyle, mannonoyle, glycoheptonoyle, glycooctonoyle residue,

- m is an integer from 2 to 15, preferably from 2 to 7,
- $R_1$ represents H or an alkyl residue from 1 to 15 carbon atoms, particularly $CH_3$,

  * the remaining residues, which are 30% to 90% of the number of R residues, represent $NH_3^+$,
  * R, moreover, having the ability to be constituted in 0 to 25% of the cases by a molecule which constitutes a recognition signal recognized by a cellular membrane receptor, under the condition that the polymeric conjugate contains at least 30% free $NH_3^+$ functions, and particularly at the ratio of 0.5 to 5, advantageously at 1 molecule for approximately 10,000 components,

or at the ratio of 10 to 100, advantageously at 60 molecules for approximately 10,000 components.

7. Complex according to claim 6, in which the polymer includes a polymer of formula (II):

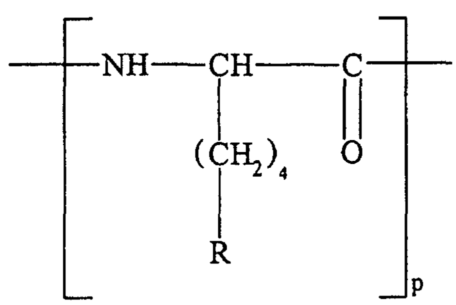

(II)

in which:

- p has the meanings indicated in claim 6,

  * 10% to 70% of the number of R residues represent a NH-CO-$(CHOH)_m$-$R_1$ group, m and $R_1$ having the meanings indicated in claim 6,

* the remaining residues, which are the 30% to 90% of the number of R residues, represent $NH_3^+$, and from 0 to 25% of the R residues being able to be substituted by a molecule which constitutes a recognition signal recognized by a cellular membrane receptor, under the condition that the polymeric conjugate contains at least 30% free $NH_3^+$ functions.

**8.** Complex according to claim 6, in which the polymer includes a polymeric group of formula (II):

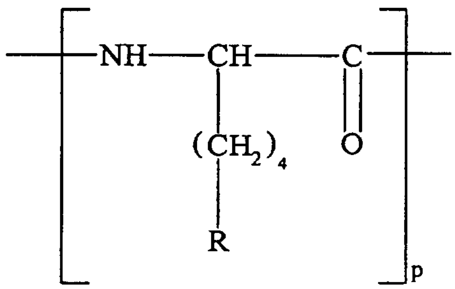

(II)

in which:

- p has the meanings indicated in claim 6,

  * 10% to 70% of the number of R residues represent a $NH-CO-(CHOH)_m-R_1$ group, m and $R_1$ having the meanings indicated in claim 6,
  * the remaining residues, which are the 30% to 90% of the number of R residues, represent $NH_3^+$.

**9.** Complex according to claim 6, in which the polymer includes a polymeric group of formula (II):

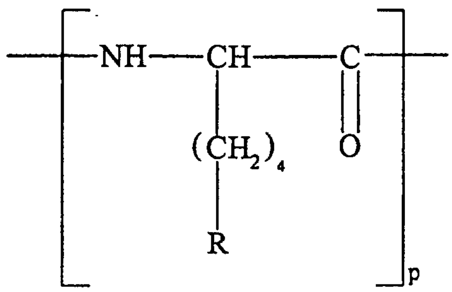

(II)

in which:

- p has the meanings indicated in claim 6,
- this polymeric group containing a number p of R residues among which:

  * 10% to 70% of the number of R residues represent a $NH-CO-(CHOH)_m-R_1$ group, m and $R_1$ having the meanings indicated in claim 6,
  * the remaining residues, which are the 30% to 90% of R residues, represent in one part $NH_3^+$ and, in another part, represent a molecule which constitutes a recognition signal recognized by a cellular membrane receptor at the ratio of 0.5 to 5, advantageously at 1 molecule for 10,000 components.

**10.** Complex according to claim 6, in which the polymer includes a polymeric group of formula (II):

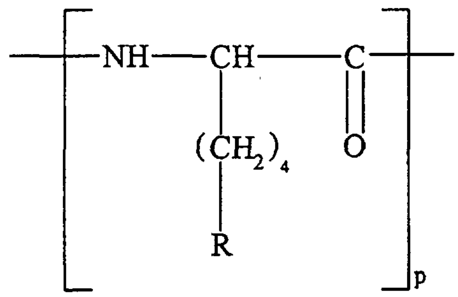

(II)

in which:

- p has the meanings indicated in claim 6,
- this polymeric group containing a number p of R residues among which:

  * 10% to 70% of the number of R residues represent a NH-CO-$(CHOH)_m$-$R_1$ group, m and $R_1$ having the meanings indicated in claim 6,
  * the remaining residues, which are the 30% to 90% of the number of R residues, represent in one part $NH_3^+$ and, in another part, represent a molecule which constitutes a recognition signal recognized by a cellular membrane receptor at the ratio of 10 to 100, advantageously at 60 molecules for approximately 10,000 components.

**11.** Complex according to anyone of claims 1 to 10, **characterized by** the fact that the recognition signal is chosen from the following:

A) from simple or complex osides recognized by membrane lectins, and chosen among the following items:

**a. Asialo-oligoside of triantennary lactosamin type: asialoglycoprotein receptor**

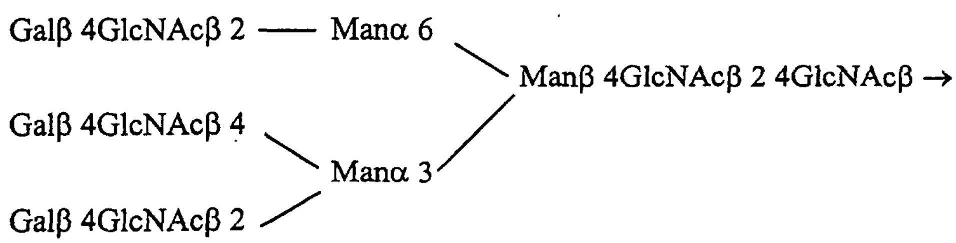

**b. Asialo oligoside of tetraantennary lactosamin type: asialoglycoprotein receptor**

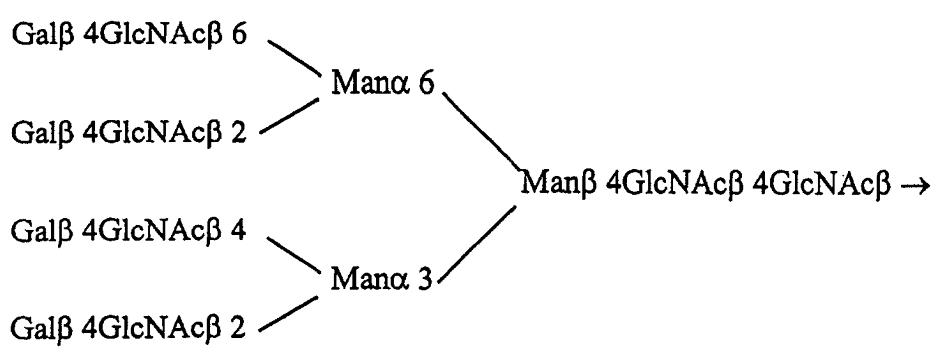

**c. Lewis x: LECAM 2/3**

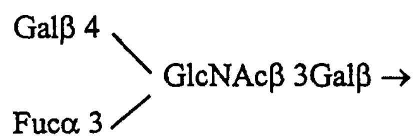

d. Sialyl Lewis x: LECAM 3/2

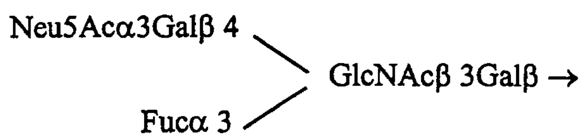

e. Sulfated Lewis x (HNK1): LECAM 1

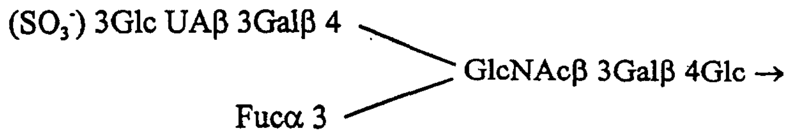

f. Oligomannoside: mannose receptor

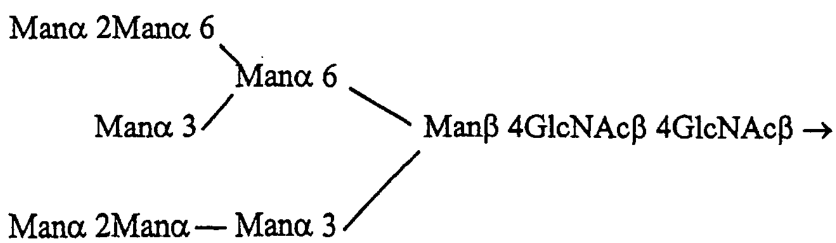

g. Phosphorylated oligomannoside: mannose 6 phosphate receptor

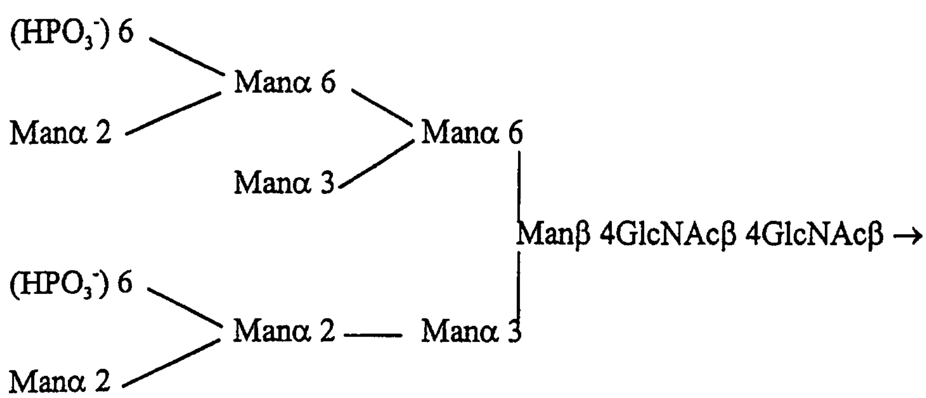

h. Sulfated oligosaccharide of lactosamin type: sulfated GalNAc 4 receptor

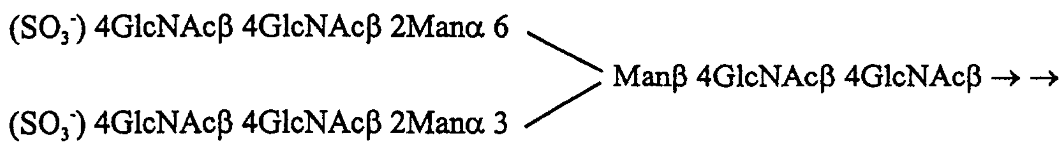

B) from peptides

a) anti-inflammatory peptides or certain of their fragments recognized by the vascular wall receptors, such as:

- intestinal vasodilator polypeptide (IPV)
  HSDAVFTDNYTRLRKQMAVKKYLNSILN-$NH_2$
- natriuretic atrial polypeptide (NAP)
  SLRRSSCFGGRMDRIGAQSGLGCNSFRY
- lipocortine
  HDMNKVLDL
- bradykinine
  RPPGFSPFR;

b) peptide ligands of integrines, such as peptides containing the sequence RGD such as the receptor of the fibronectine;
c) chemotactic factors, such as formyl peptides and antagonists: FMLP, (N-formyl-Met-Leu-Phe);
d) peptide hormones such as

α-MSH: Ac-SYSMEHFRWGKPV-$NH_2$,

C) Natural metabolites such as

- biotine,
- tetrahydrofolate,
- folic acid,
- carnitine.

**12.** Complex according to anyone of claims 1 to 11, **characterized by** the fact that the nucleic acid may be chosen from the following:

a) gene markers, such as

- genes coding for luciferase,
- genes coding for β-galactosidase,
- genes coding for chloramphenicol acetyl transferase,
- genes bestowing the resistance to an antibiotic, such as hygromycine and neomycine, etc.

b) genes for therapeutic purposes, such as gene encoding

- low density lipoprotein receptors, deficient in the cases of hypercholesterolomia (liver),
- coagulation factors: factors VIII and IX,
- phenylalanine-hydroxylase (phenylcetonuria)
- adenosine desaminase (ADA immunodeficiency)
- lysosomic enzymes, such as β-glucosidase in the case of Gaucher's disease,
- dystrophine and minidistriphine (myopathy)
- tyrosine hydroxylase (Parkinson),
- neuron growth factors (Alzheimer),
- CFTR cystic-fibrosis transmembrane conductance regulator (mucoviscidose),
- alpha1-antitrypsine,
- nuclear factors: NF-KB, CII TA, ...

- cytokines (interleukines, TNF: tumor necrosis factor),
- thymidine kinase of the Herpes simplex virus,
- NO synthase,
- angiotensin II receptors,
- tumor suppression genes, such as protein p53 gene,
- MHC proteins, major histocompatibility system, in particular HLA-B7,
- cytosine desaminase,
- genes coding for sense and anti-sense RNA,
- genes coding for ribozymes,

c) genes with vaccine purposes:

- genes coding for viral antigens (vaccination), for example, gene coding for the nucleoprotein of the influenza virus.

**13.** Complex according to anyone of claims 1 to 12, in which:

- the polymer, particularly polylysine, presents a degree of polymerization of approximately 100 to approximately 500, preferably 190,
- the free $NH_3^+$ functions of the lysine components are substituted at 60% by gluconoyle groups and possibly by a molecule constituting a recognition signal for 10,000 lysine residues when the said signal molecule possesses an affinity of at least $10^6$ l $mole^{-1}$ in relation to the receptor of the cell which the complex should target, or possibly by 60 recognition signal molecules for 10,000 lysine residues when the said signal molecule possesses an affinity inferior to at least $10^4$ l $mole^{-1}$ in relation to the aforementioned receptor,
- the nucleic acid has a molecular mass of approximately $6.10^5$ to approximately $25.10^6$, and
- the ratio between the mean number of base pairs of nucleic acid to the molecules of monomeric components, particularly the lysine, is approximately 0.9 to approximately 1.1, preferably approximately 0.95 to approximately 1.05.

**14.** Positively charged polymeric conjugate, containing components having free $NH_3^+$ functions, particularly lysine residues, and being as follows:

- the free $NH_3^+$ functions of the above mentioned components are substituted in a ratio of at least 10%, advantageously from 45% to 70%, particularly 60%, by non-charged residues leading to a reduction of positive charges in comparison to the same non-substituted polymeric conjugate, facilitating the release of a nucleic acid by the dissociation of a complex between a negatively charged nucleic acid and said polymeric conjugate,
- the above mentioned residues possess in addition the following properties:

  → they contain at least one hydroxyl group,
  → they do not correspond to a recognition signal recognized by a cellular membrane receptor,

- the free $NH_3^+$ functions of the above mentioned components and/or the hydroxyl groups of the above mentioned residues being also able to be substituted by at least one molecule which constitutes a recognition signal recognized by a cellular membrane receptor, under the condition that the polymeric conjugate contains at least 30% free $NH_3^+$ functions.

**15.** Polymeric conjugate according to claim 14, and such as defined according to anyone of claims 2 to 5, or containing a polymeric group of formula according to anyone of claims 6 to 10.

**16.** Use of a complex according to one of claims 1 to 13, or of a conjugate according to anyone of claims 14 or 15, for the transfection *in vitro* or *ex vivo* of cells with the aid of a gene, particularly those defined in claim 12.

**17.** Use of a complex or a conjugate according to claim 16, **characterized by** the fact that the cells are chosen from amongst the following:

- hematopoietic stem cells;
- liver cells;
- cells of skeletal muscles;

- skin cells:

  . fibroblasts,
  . keratinocytes,
  . dendritic cells,
  . melanocytes,

- cells of the vascular walls:

  . endothelial cells,
  . smooth muscle cells,

- epithelial cells of the respiratory tract;
- cells of the central nervous system;
- cancer cells;
- cells of the immune system, such as lymphocytes, macrophages, NK cells, etc.

**18.** Method of transfection *in vitro* or *ex vivo*, **characterized by** the fact that it has been put in the presence of a complex, according to anyone of claims 1 to 13, in a medium containing cells to be transfected, under conditions where there is:

- passage of the complex from the medium into the cytoplasm of the cells,
- release of the nucleic acid involved in said complex into the cytosol of the cells,
- transcription and expression of the nucleic acid into the transfected cells.

**19.** Pharmaceutical composition, **characterized by** the fact that it includes, as an active substance, at least one of the complexes according to anyone of claims 1 to 13, in association with an acceptable pharmaceutical vehicle.

**20.** Use of a complex according to one of claims 1 to 13, or of a conjugate according to one of claims 14 or 15, for the preparation of a medicament to be used, for example, for the treatment of congenital or acquired metabolic deficiency, or for the treatment of tumors, or for the preparation of a vaccine, for example for an influenza vaccine.

**21.** Case or kit comprising:

- a polymeric conjugate according to one of claims 14 or 15, such as the polylysine, substituted by a residue leading to a reduction of charges of $NH_3^+$ free groups, this polymeric conjugate being able to comprise possibly a recognition signal recognized by a cellular membrane receptor, which beforehand is fixed or not fixed on the above mentioned polymeric conjugate, said recognition signal being a function of the cell to be targeted,
- a plasmid containing at least one gene to be transfected, and the regulation system of said gene,
- reagents permitting the eventual fixation of the recognition signal on said polymeric conjugate,
- reagents permitting the formation of a complex according to anyone of claims 1 to 13, between the polymeric conjugate and the plasmid,
- reagents permitting the transfection of the cell by said complex.

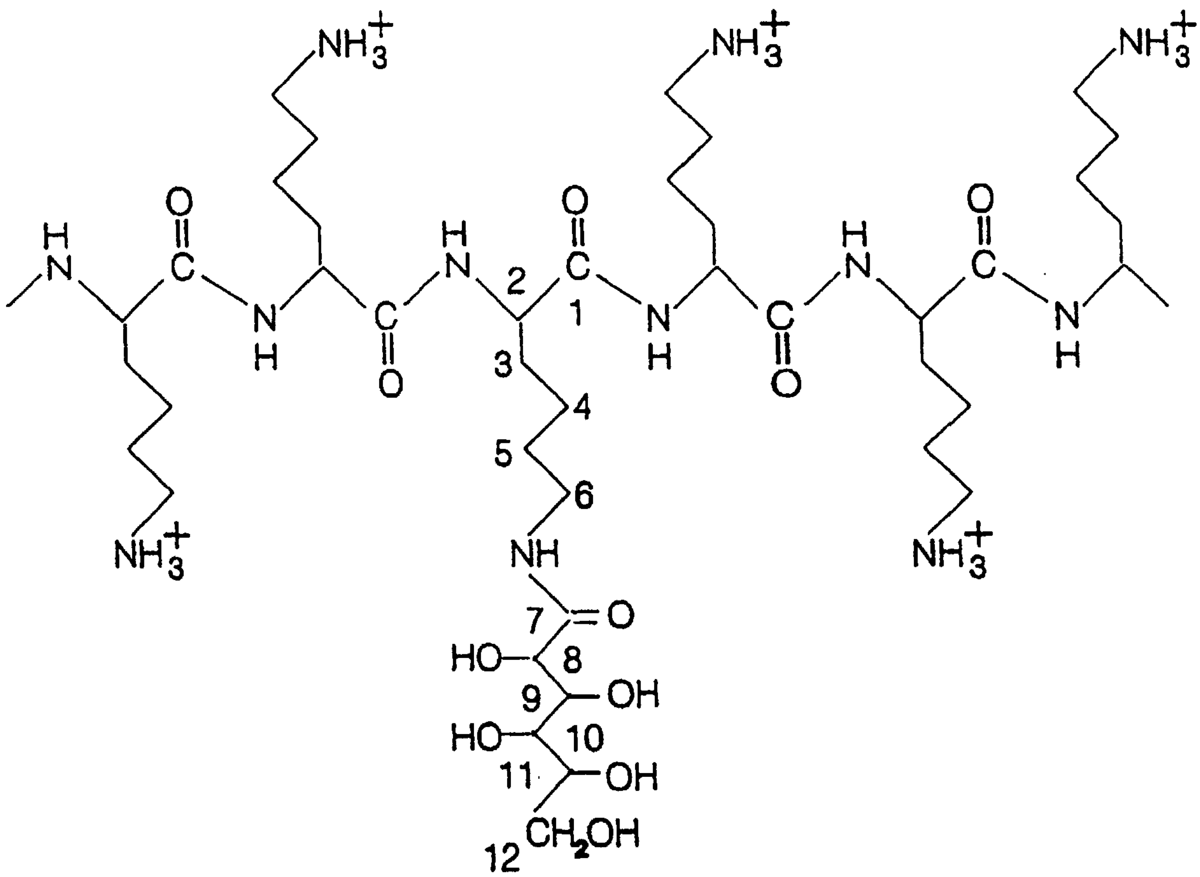

Figure 1a

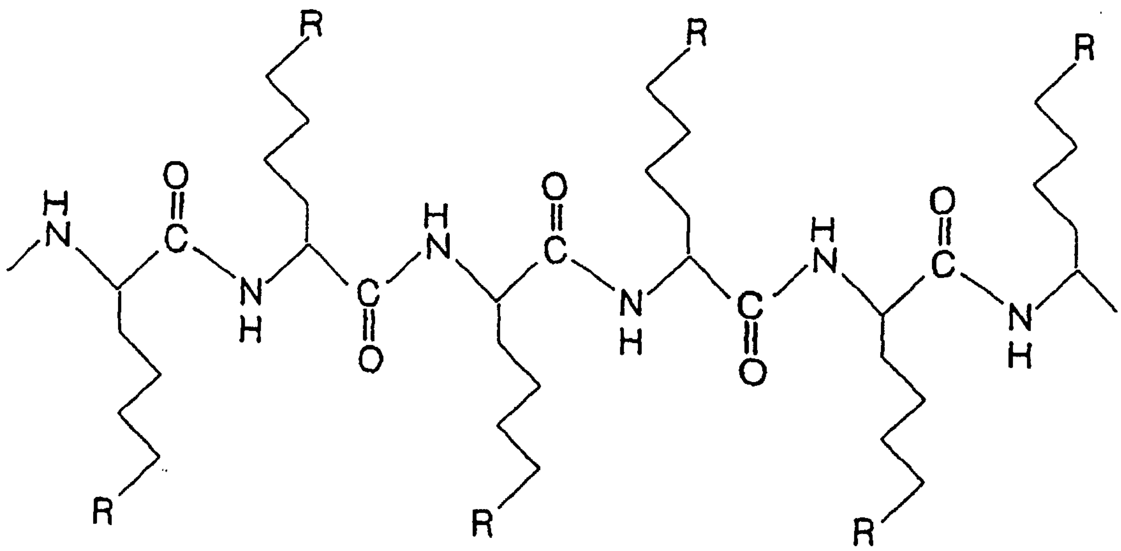

$R = NH_3^+$ ou $R = NH - CO - (CHOH)_m - R_1$

**Figure 1b**

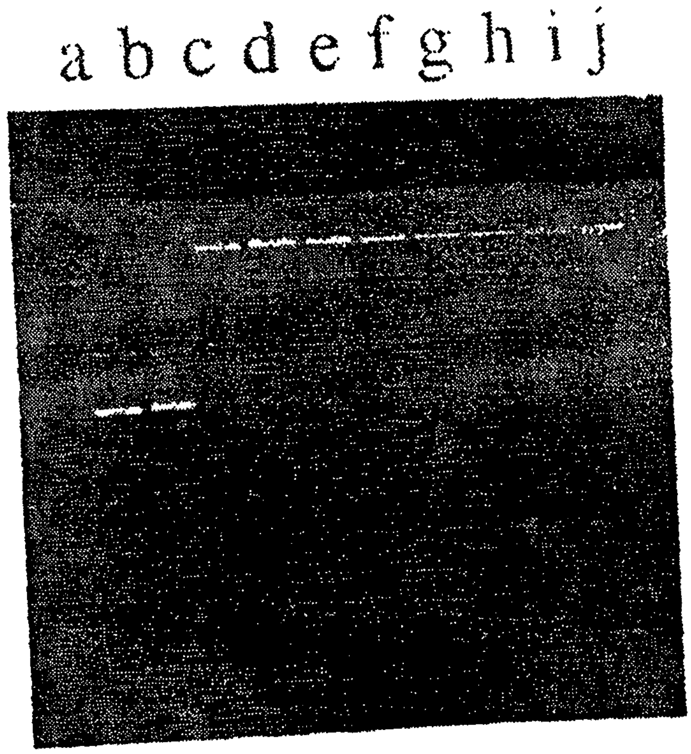


**Figure 1c**

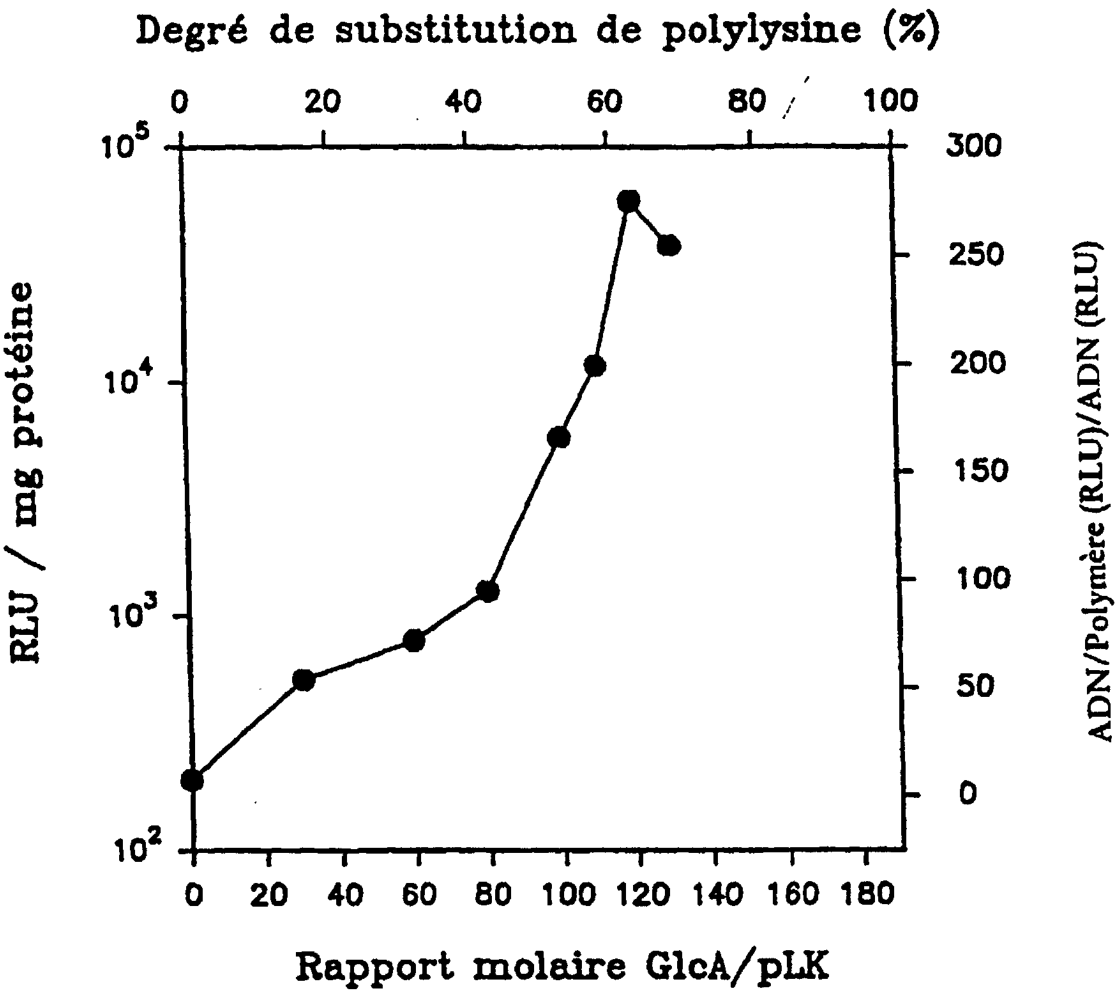
Degré de substitution de polylysine (%)
0
20
40
60
80
100
10^5
10^4
10^3
10^2
300
250
200
150
100
50
0
RLU / mg protéine
ADN/Polymère (RLU)/ADN (RLU)
0 20 40 60 80 100 120 140 160 180
Rapport molaire GlcA/pLK

Figure 2a

HepG2

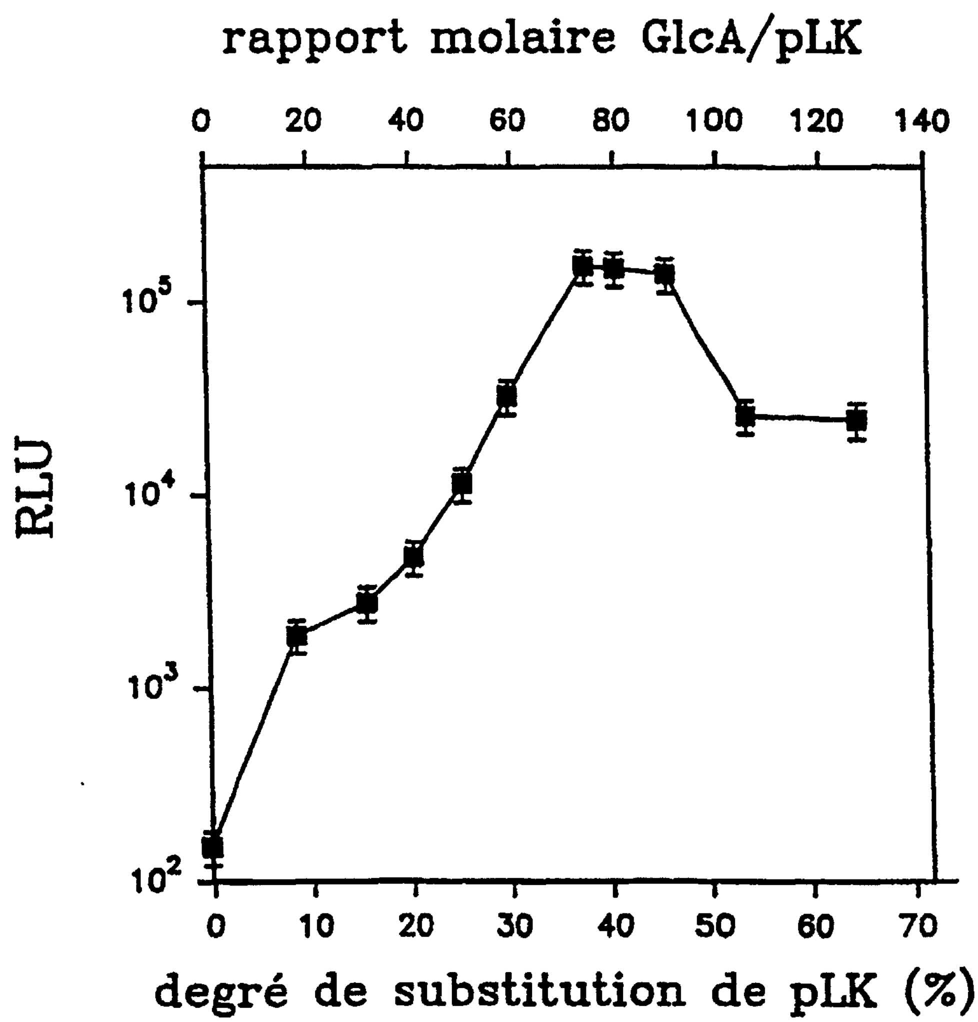


Figure 2b

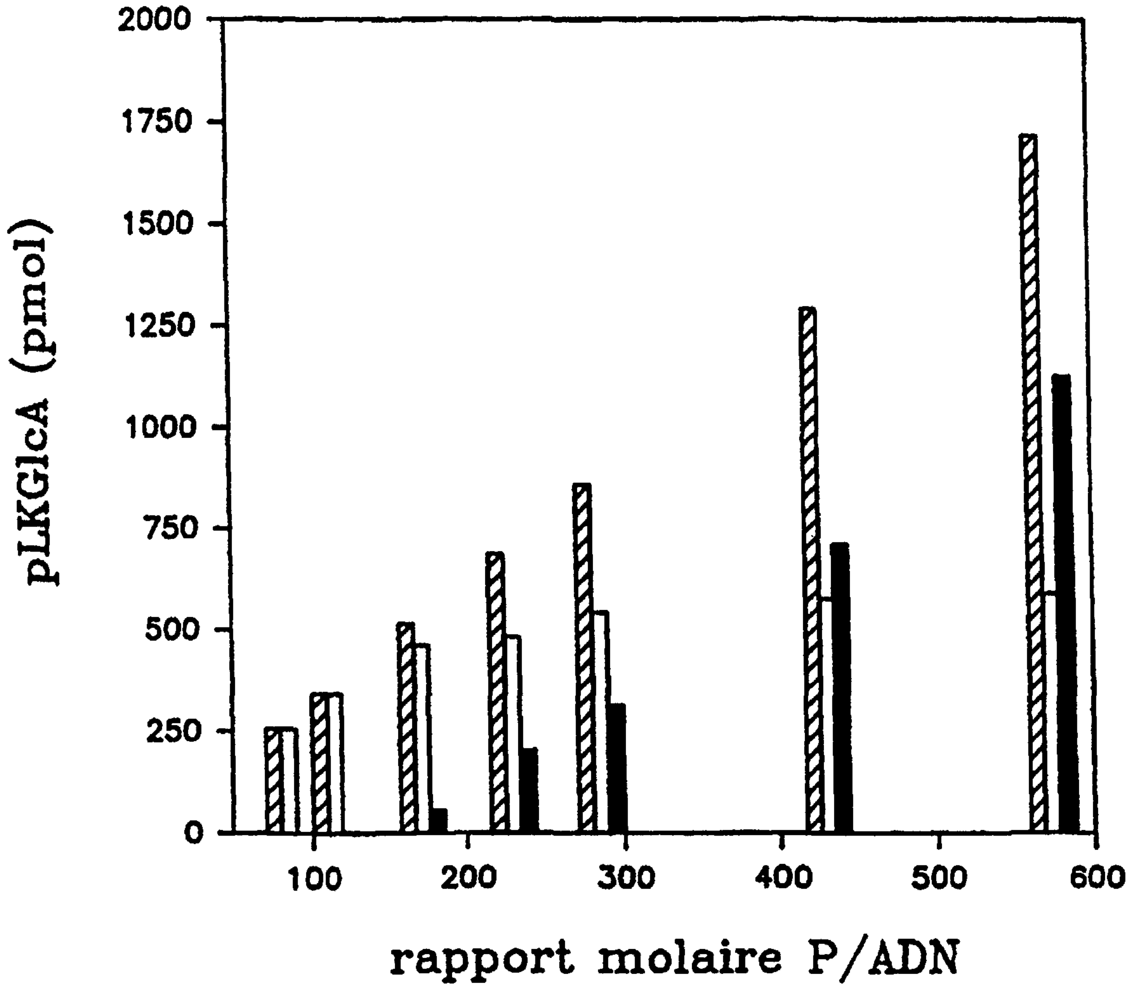
2000
1750
1500
1250
1000
750
500
250
0
pLKGlcA (pmol)
100
200
300
400
500
600
rapport molaire P/ADN

Figure 2c

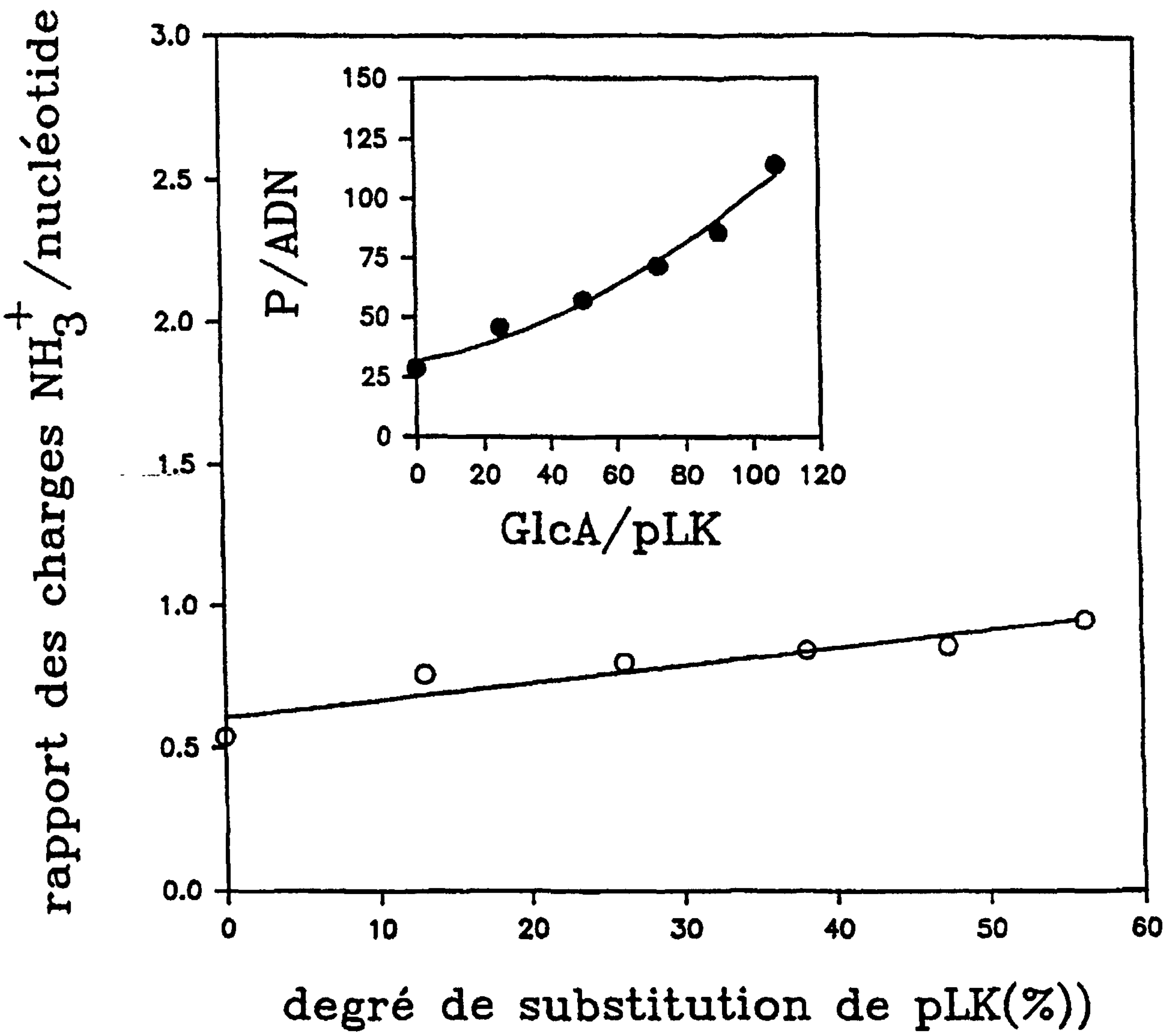
rapport des charges $NH_3^+$ /nucléotide
3.0
2.5
2.0
1.5
1.0
0.5
0.0
0
10
20
30
40
50
60
degré de substitution de pLK(%))
P/ADN
150
125
100
75
50
25
0
0
20
40
60
80
100
120
GlcA/pLK

Figure 2d

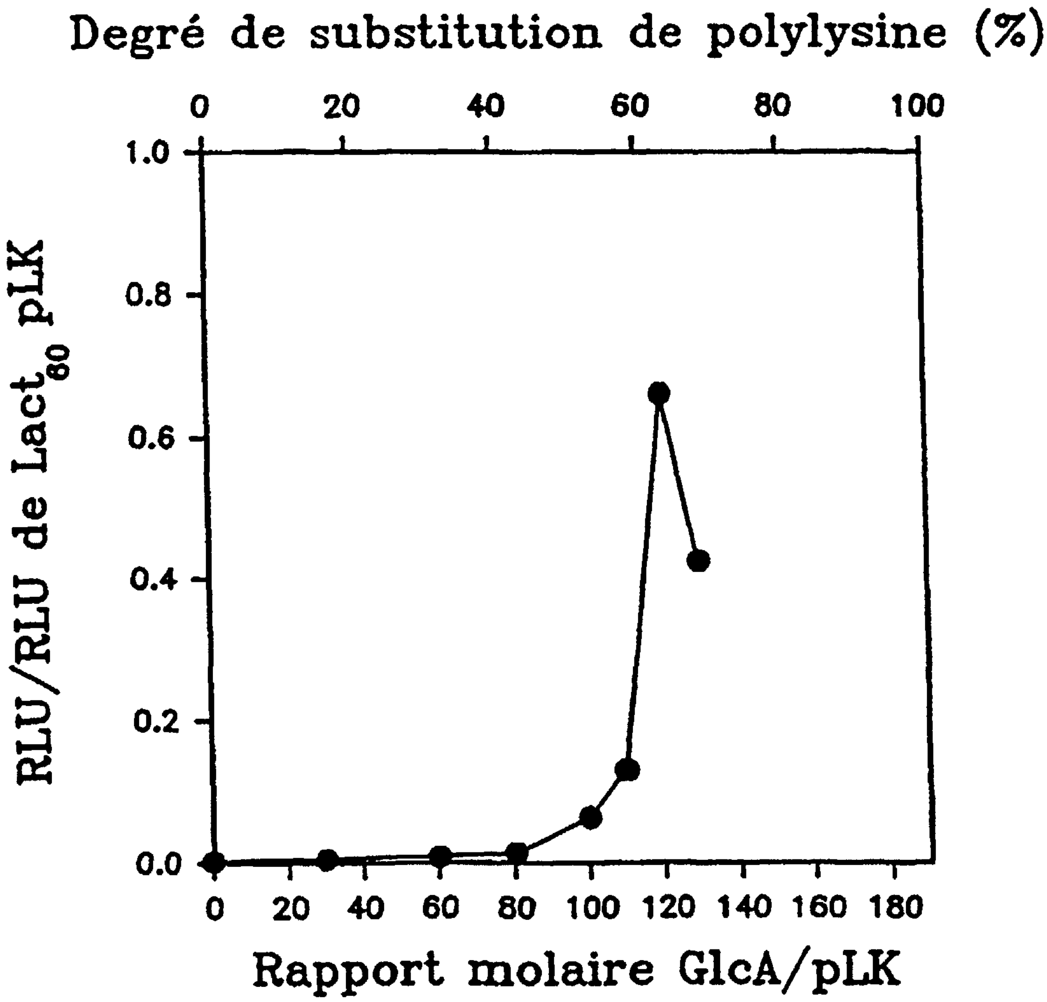
Degré de substitution de polylysine (%)
0 20 40 60 80 100
1.0 0.8 0.6 0.4 0.2 0.0
RLU/RLU de Lact80 pLK
0 20 40 60 80 100 120 140 160 180
Rapport molaire GlcA/pLK

Figure 3a

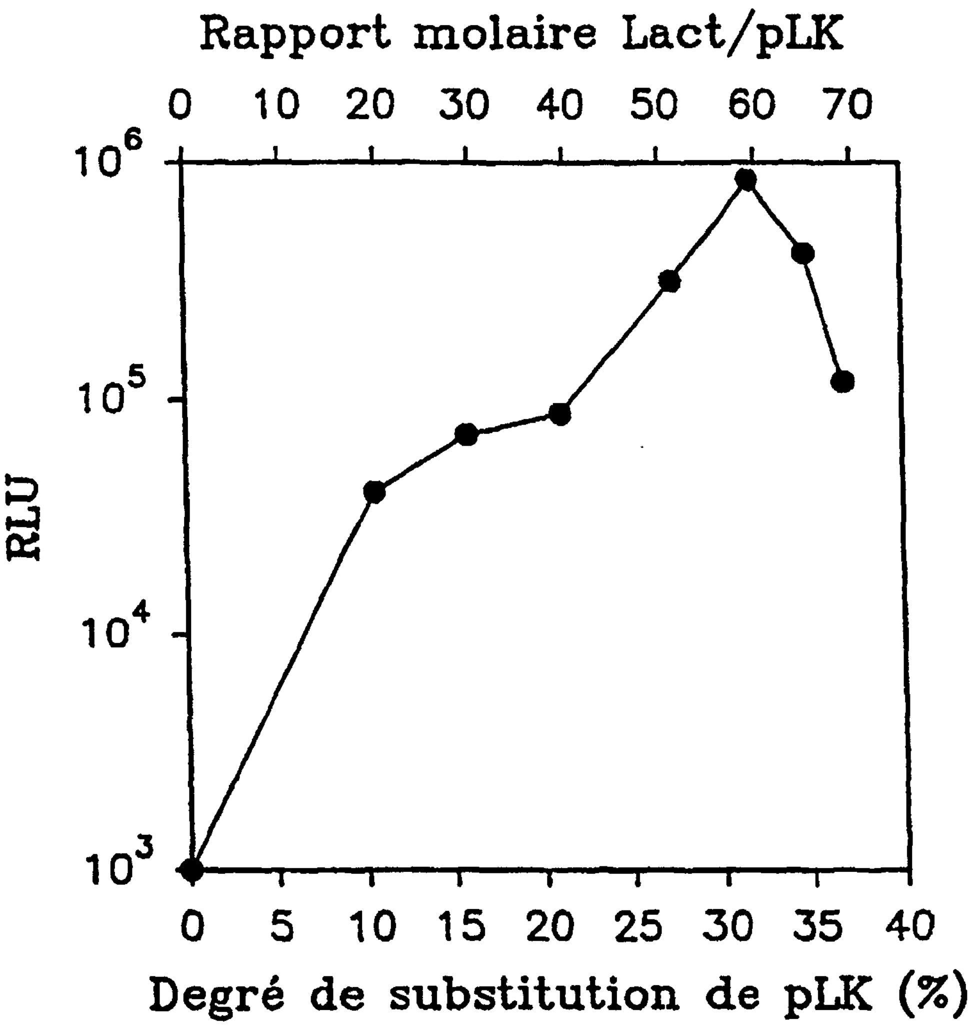
Rapport molaire Lact/pLK
0 10 20 30 40 50 60 70
10^6
10^5
10^4
10^3
RLU
0 5 10 15 20 25 30 35 40
Degré de substitution de pLK (%)

Figure 3b

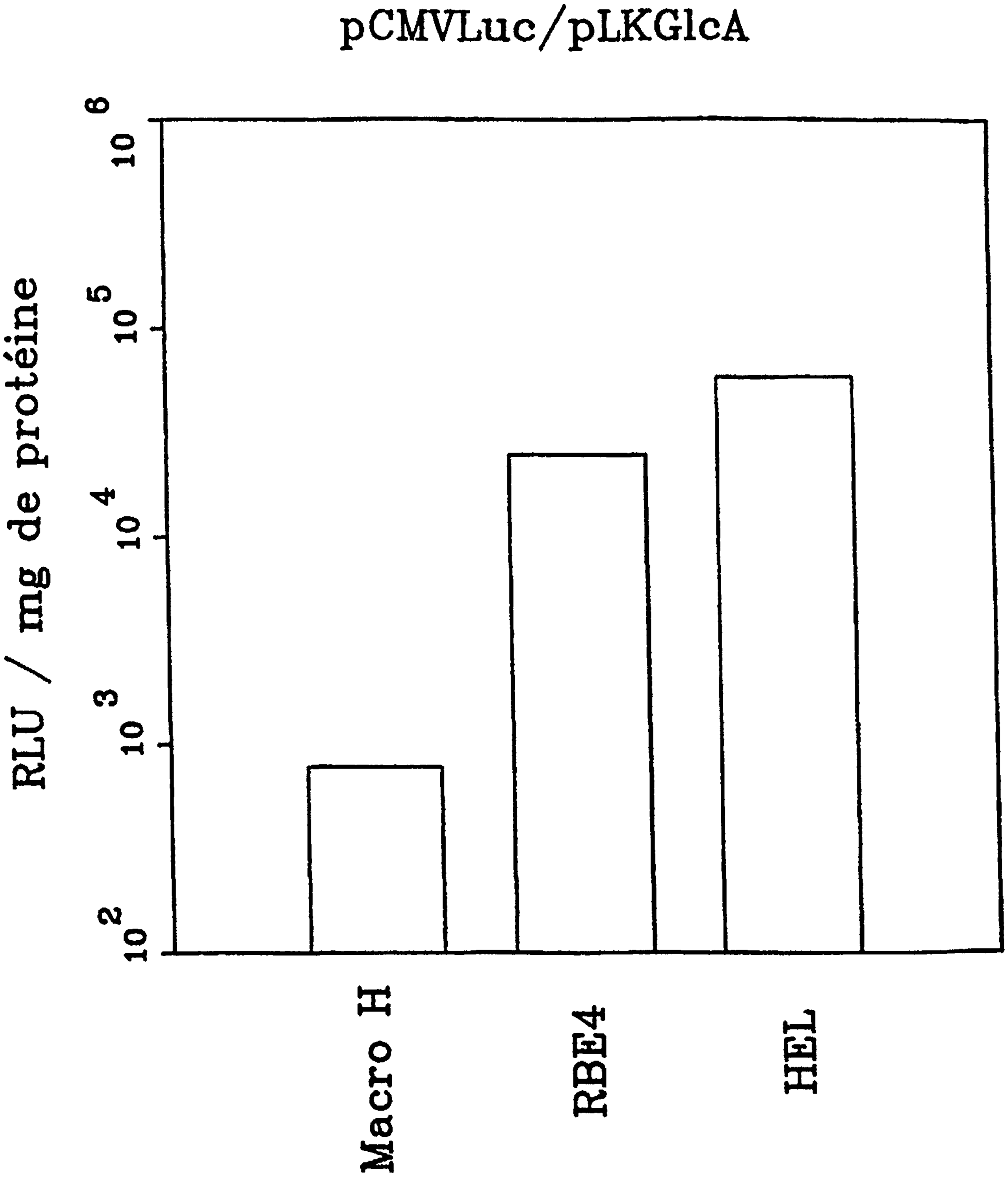
pCMVLuc/pLKGlcA
RLU / mg de protéine
10^6
10^5
10^4
10^3
10^2
Macro H
RBE4
HEL

Figure 4

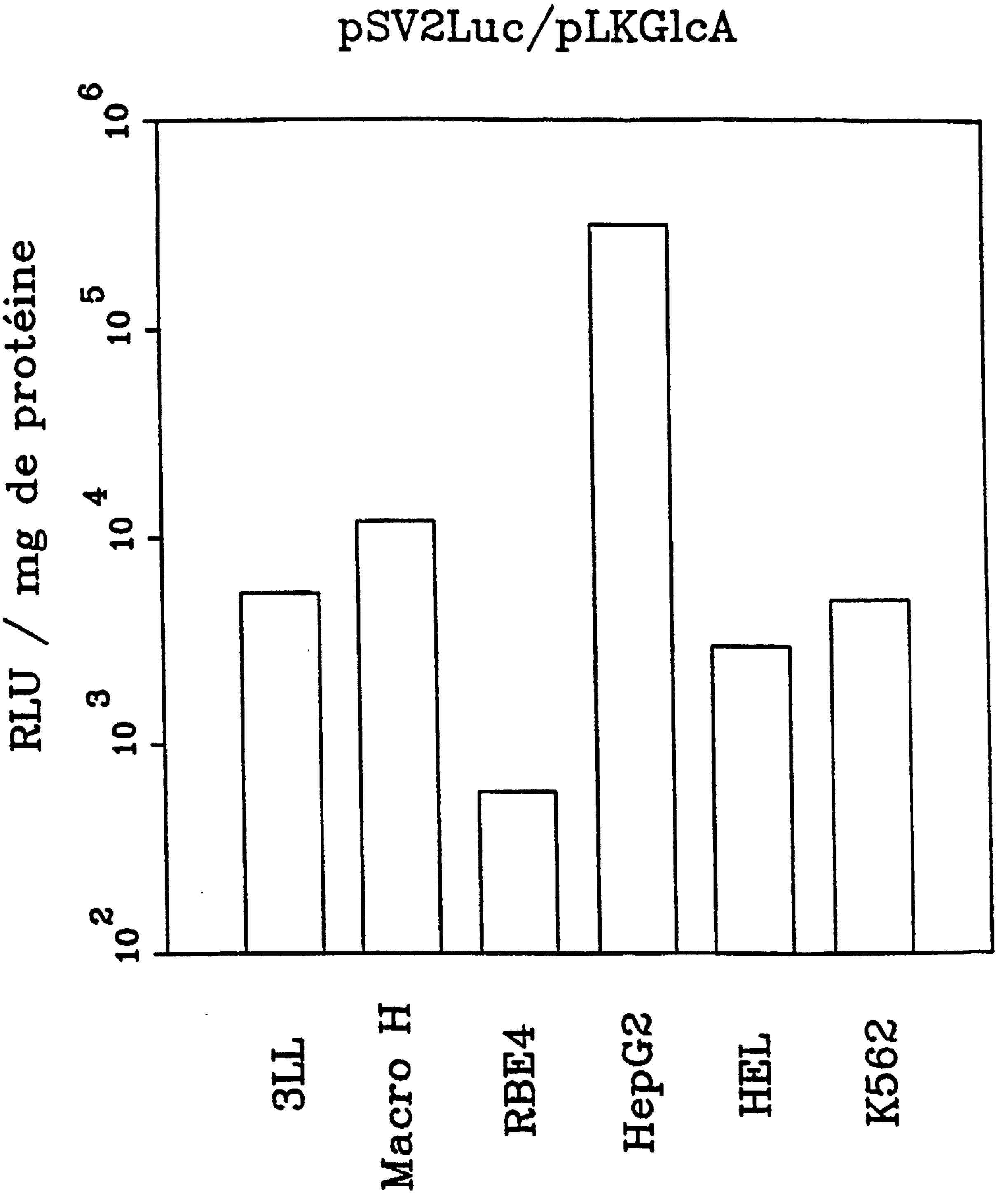
pSV2Luc/pLKGlcA
RLU / mg de protéine
10^6
10^5
10^4
10^3
10^2
3LL
Macro H
RBE4
HepG2
HEL
K562

**Figure 5**

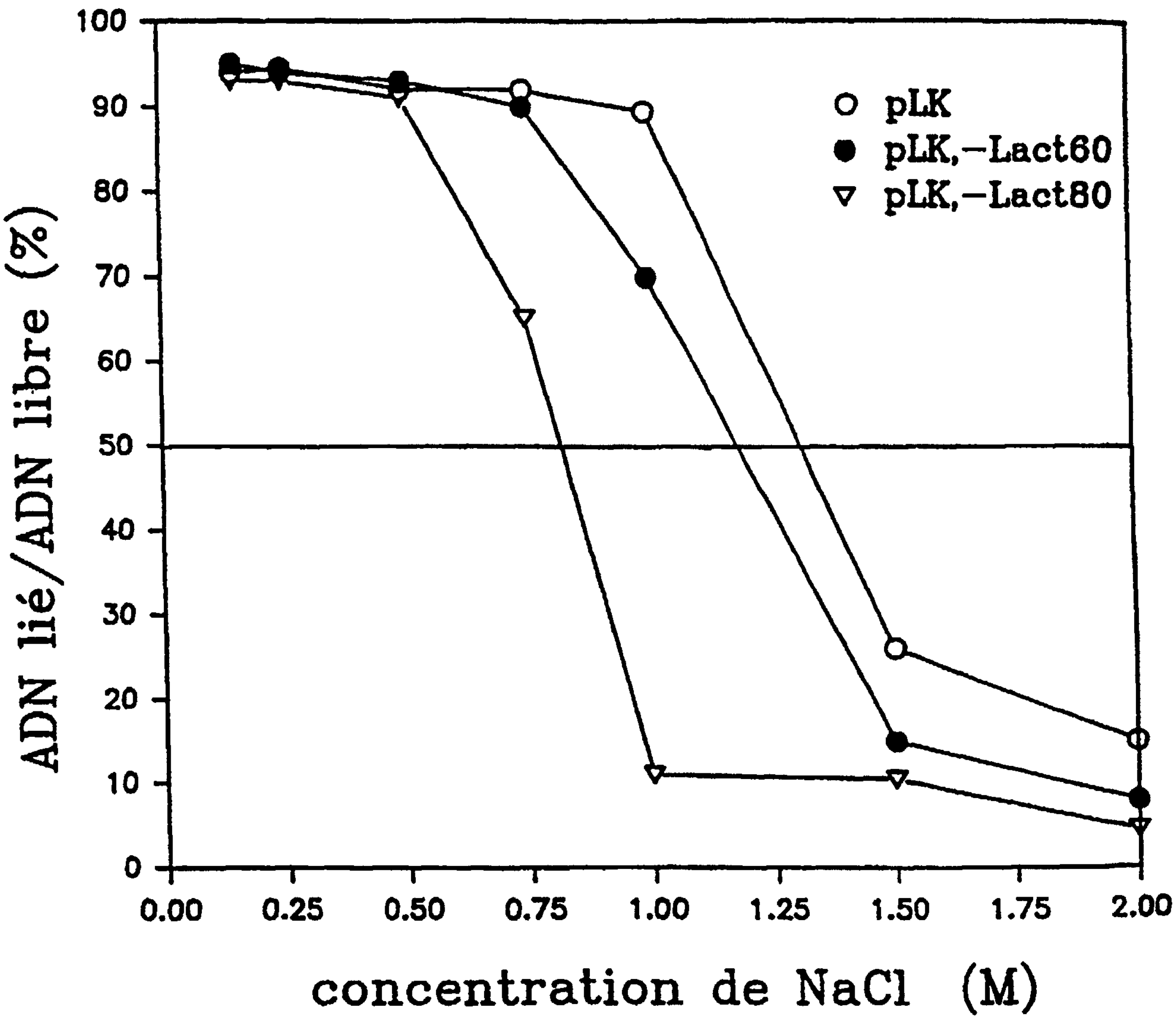
100
90
80
70
60
50
40
30
20
10
0
ADN lié/ADN libre (%)
0.00
0.25
0.50
0.75
1.00
1.25
1.50
1.75
2.00
concentration de NaCl (M)
pLK
pLK,-Lact60
pLK,-Lact80

Figure 6

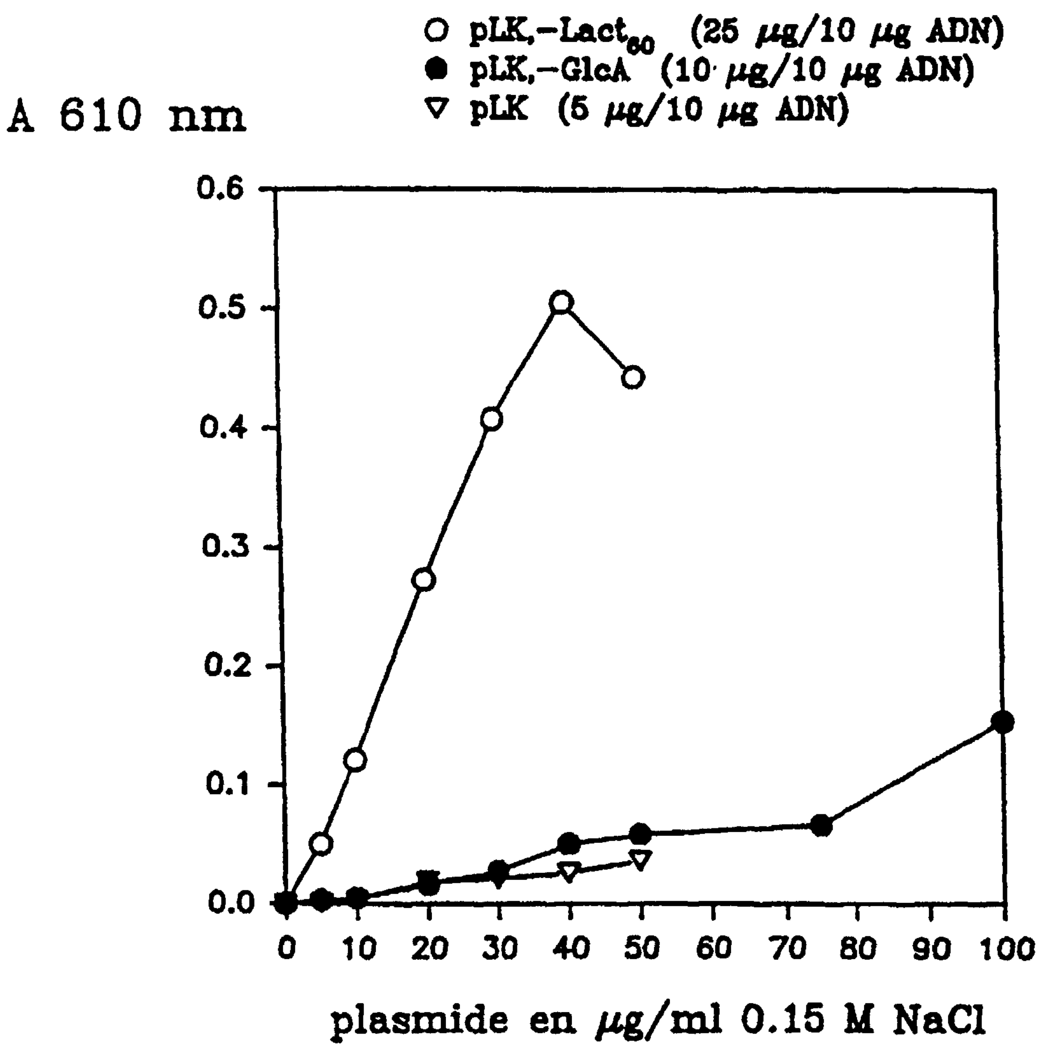

○ pLK,-Lact60 (25 μg/10 μg ADN)
● pLK,-GlcA (10 μg/10 μg ADN)
▽ pLK (5 μg/10 μg ADN)
A 610 nm
0.6
0.5
0.4
0.3
0.2
0.1
0.0
0 10 20 30 40 50 60 70 80 90 100
plasmide en μg/ml 0.15 M NaCl


Figure 7a

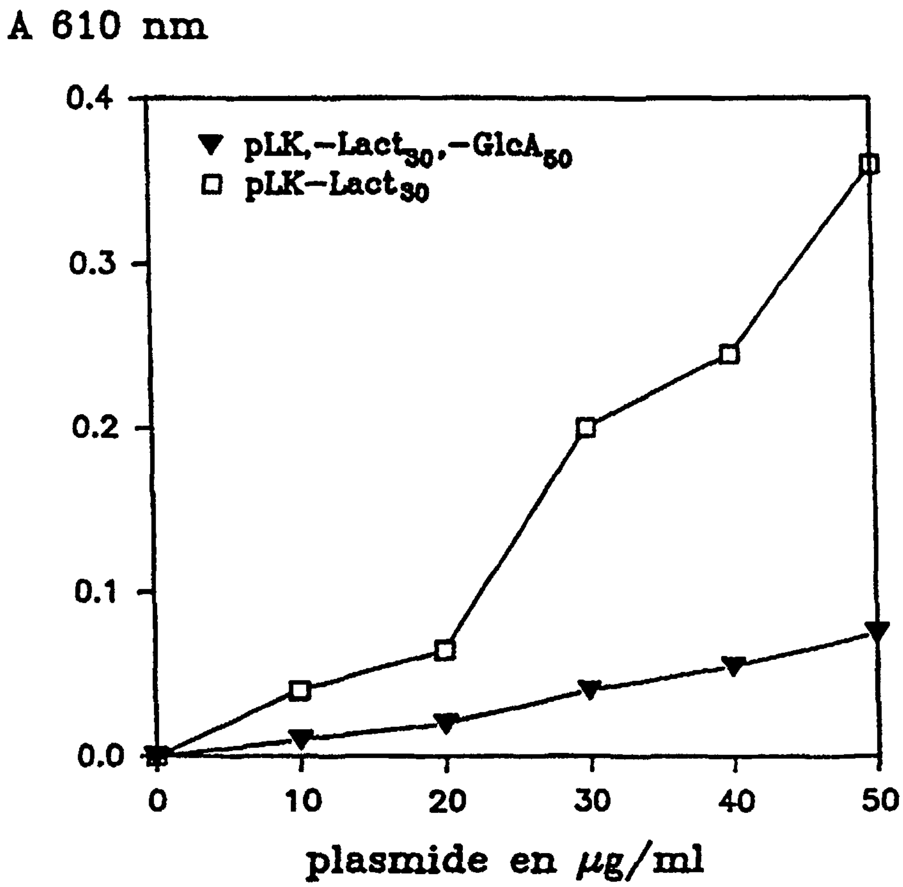
A 610 nm
0.4
0.3
0.2
0.1
0.0
pLK,-Lact30,-GlcA50
pLK-Lact30
0
10
20
30
40
50
plasmide en µg/ml

Figure 7b

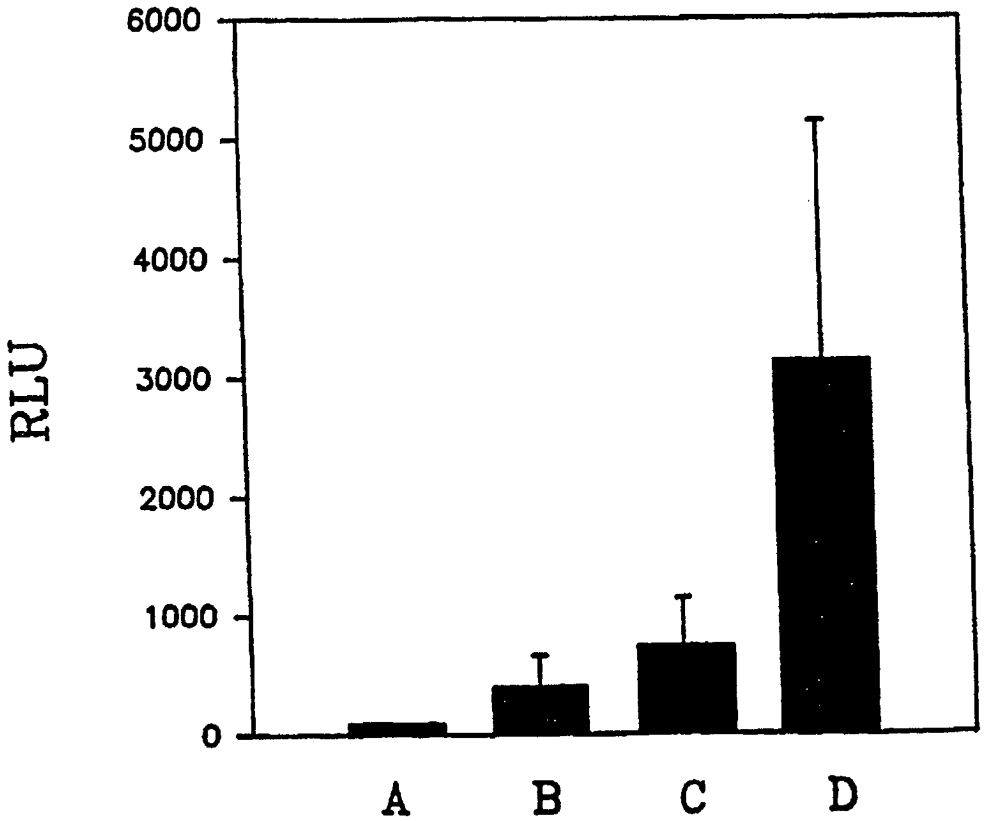


Figure 8

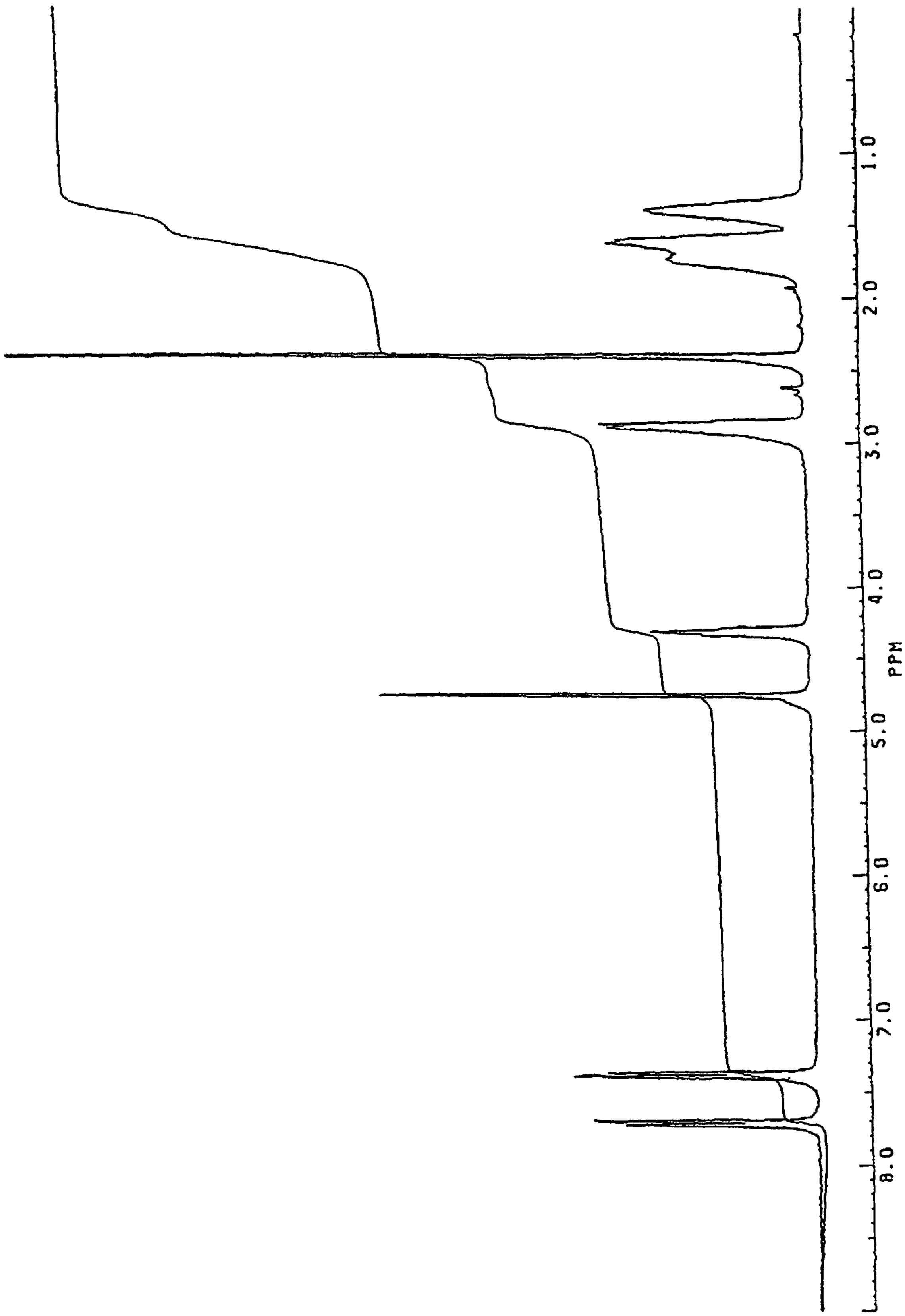


Figure 9

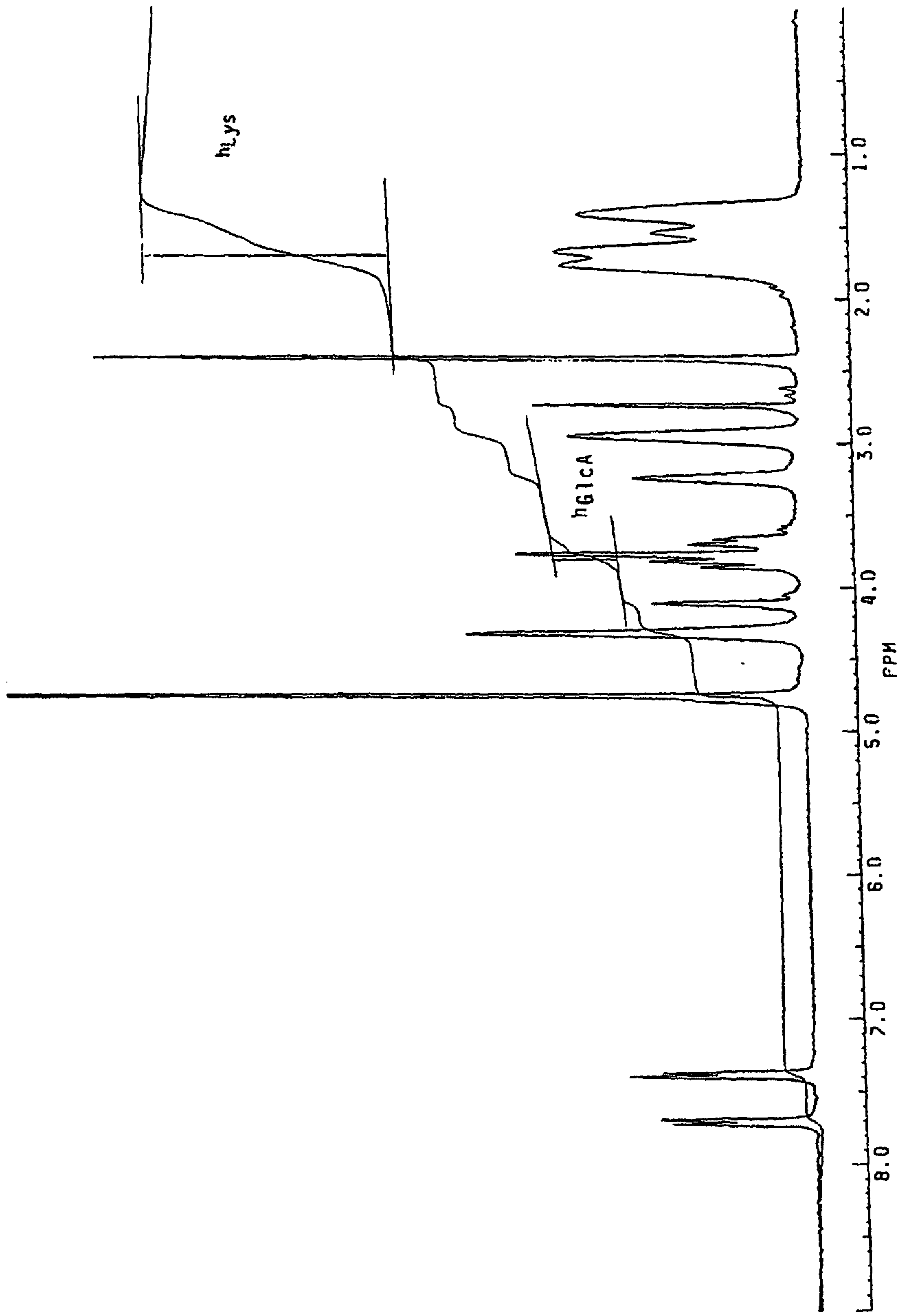


Figure 10